# EUROPÄISCHE PATENTSCHRIFT

(11) **EP 2 069 289 B1**
(45) Veröffentlichungstag und Bekanntmachung des Hinweises auf die Patenterteilung: **02.10.2013**
(21) Anmeldenummer: 07802031.0
(22) Anmeldetag: 31.08.2007
(51) Int. Cl.: C07C 237/22, C07D 207/34, C07D 209/42, C07D 213/56, C07D 215/48, C07D 217/22, C07D 235/30, C07D 239/28, C07D 239/95, C07D 241/44, C07D 277/82, C07D 307/54, C07D 307/85, C07D 333/38

(54) **ISOSERINDERIVATE ALS INHIBITOREN DES KOAGULATIONSFAKTORS IXa**
ISOSERINE DERIVATIVES FOR USE AS COAGULATION FACTOR IXa INHIBITORS
DÉRIVÉS D'ISOSÉRINE UTILISÉS COMME INHIBITEURS DU FACTEUR DE COAGULATION IXa

(30) Priorität: 13.09.2006 DE 102006042926
(43) Veröffentlichungstag der Anmeldung: 17.06.2009
(73) Patentinhaber: SANOFI, 75008 Paris (FR)
(72) Erfinder: STEINHAGEN, Henning, 65926 Frankfurt am Main (DE); FOLLMANN, Markus, 42489 Wülfrath (DE); GOERLITZER, Jochen, 65926 Frankfurt am Main (DE); SCHREUDER, Herman, 65926 Frankfurt am Main (DE)
(74) Vertreter: Then, Johann
(86) Internationale Anmeldenummer: PCT/EP2007/007613
(87) Internationale Veröffentlichungsnummer: WO 2008/031509

(56) Entgegenhaltungen:
- WO-A-94/22885
- US-A1- 2001 006 977
- US-A1- 2005 137 168
- US-B1- 6 297 269
- BATT D G ET AL: "5-Amidinoindoles as dual inhibitors of coagulation factors IXa and Xa" BIOORGANIC & MEDICINAL CHEMISTRY LETTERS, OXFORD, GB, Bd. 14, Nr. 21, 1. November 2004 (2004-11-01), Seiten 5269-5273, XP004580512 ISSN: 0960-894X

## Beschreibung

Die Erfindung betrifft neue Verbindungen der Formel I mit antithrombotischer Aktivität, die insbesondere den Blutgerinnungsfaktor IXa inhibieren, Verfahren zu ihrer Herstellung und Verwendung derselben als Arzneimittel.

Die Blutgerinnung ist ein für das Überleben von Säugetieren wesentlicher Vorgang der Kontrolle des Blutstroms. Der Vorgang der Gerinnung und der nachfolgenden Auflösung des Gerinnsels nach erfolgter Wundheilung setzt nach einer Gefäßschädigung ein und lässt sich in vier Phasen einteilen:
1. Die Phase der Vasokonstriktion oder Gefäßkontraktion: Hierdurch wird der Blutverlust in das geschädigte Areal vermindert:
2. Die nächste Phase ist die der Plättchenaktivierung durch Thrombin. Die Plättchen heften sich an der Stelle des Gefäßwandschadens an und bilden ein PlättchenAggregat. Das Protein Fibrinogen ist hierbei für die Quervernetzung der Plättchen über entsprechende Oberflächenrezeptoren verantwortlich. Plättchen binden auch an freigelegtes Kollagen der extrazellulären Matrix der geschädigten Gefäßwand und werden hierdurch aktiviert. Nach Aktivierung der Plättchen wird eine Reihe von Botenstoffen ausgeschüttet, die die Aktivierung von weiteren Plättchen induzieren. Gleichzeit wird ein Membranlipid, Phosphatidylserin, von der Membraninnenseite der Plättchen auf die Außenseite transportiert, an das sich Komplexe aus Gerinnungsfaktoren anlagern können. Die Plättchen beschleunigen über diesen Mechanismus die Blutgerinnung.
3. Die Bildung dieser Gerinnungskomplexe führt zur massiven Bildung von Thrombin welches das lösliche Fibrinogen durch Abspaltung zweier kleiner Peptide in Fibrin überführt. Fibrinmonomere bilden spontan fadenförmig Stränge aus denen nach Quervernetzung durch den Gerinnungsfaktor XIII ein stabiles Proteinnetz bildet. Das anfänglich noch lockere Plättchenaggregat wird durch dieses Fibrinnetz stabilisiert, Plättchenaggregate und Fibrinnetz sind die beiden essentiellen Bestandteile eines Thrombus.
4. Nach Wundheilung wird der Thrombus durch die Einwirkung des Schlüsselenzyms des körpereigenen Fibrinolysesystems Plasmin aufgelöst.

Zwei alternative Wege können zur Bildung eines Fibringerinnsels führen, der intrinsische und der extrinsische Weg. Diese Wege werden durch unterschiedliche Mechanismen eingeleitet, in späterer Phase konvergieren sie jedoch zu einer gemeinsamen Endstrecke der Gerinnungskaskade. In dieser Endstrecke der Gerinnung wird der Gerinnungsfaktor X aktiviert. Der aktivierte Faktor X ist für die Bildung von Thrombin aus der im Blut zirkulierenden inaktiven Vorstufe Prothrombin verantwortlich. Die Bildung eines Thrombus auf dem Boden einer Gefäßwandabnormität ohne Wunde ist das Resultat des intrinsischen Weges. Die Fibringerinnselbildung als Antwort auf einen Gewebsschaden oder eine Verletzung ist das Resultat des extrinsischen Weges. Beide Wege beinhalten eine größere Anzahl von Proteinen, die als Gerinnungsfaktoren bekannt sind.

Der intrinsische Weg erfordert die Gerinnungsfaktoren V, VIII, IX, X, XI und XII sowie Präkallikrein, hochmolekulares Kininogen, Calciumionen und Phospholipide aus Plättchen.

Der intrinsische Weg wird eingeleitet, wenn Präkallikrein, hochmolekulares Kininogen Faktor XI und XII an eine negativ geladene Oberfläche binden. Dieser Zeitpunkt wird als Kontaktphase bezeichnet. Die Exposition gegenüber einem Gefäßwandkollagen ist der primäre Stimulus der Kontaktphase. Resultat der Vorgänge der Kontaktphase ist die Umwandlung von Präkallikrein in Kallikrein, das wiederum den Faktor XII aktiviert. Faktor XIIa hydrolysiert weiteres Präkallikrein zu Kallikrein, so dass eine Aktivierung die Folge ist. Mit zunehmender Aktivierung von Faktor XII kommt es zur Aktivierung des Faktors XI, der zu einer Freisetzung von Bradykinin, einem Vasodilatator führt. Dadurch kommt es zur Beendigung der initialen Phase der Vasokonstriktion. Bradykinin entsteht aus dem hochmolekularen Kininogen. In Anwesenheit von Ca²⁺-Ionen aktiviert der Faktor XIa den Faktor IX. Faktor IX ist ein Proenzym, das Vitamin-K abhängige, γ-Carboxyglutaminsäure (GLA)-Reste enthält. Die Serinproteaseaktivität kommt nach Bindung von Ca²⁺ an diese GLA-Reste zum Tragen. Mehrere der Serinproteasen der Blutgerinnungskaskade (Faktoren II, VII, IX und X) enthalten derartige Vitamin-K-abhängige GLA-Reste. Faktor IXa spaltet den Faktor X und führt zur Aktivierung zum Faktor Xa. Voraussetzung für die Bildung von Faktor IXa ist die Bildung eines Tenasekomplexes aus Ca²⁺ und den Faktoren VIIIa, IXa und X an der Oberfläche aktivierter Plättchen. Eine der Reaktionen aktivierter Plättchen ist die Präsentation von Phosphatidylserin und Phosphatidylinositol entlang der Oberflächen. Die Exposition dieser Phospholipide macht erst die Bildung des Tenasekomplexes möglich. Faktor VIII hat in diesem Vorgang die Funktion eines Rezeptors für die Faktoren IXa und X. Faktor VIII stellt daher einen Cofaktor in der Gerinnungskaskade dar. Die Aktivierung des Faktors VIII mit Bildung des Faktors VIIIa, dem eigentlichen Rezeptor, bedarf nur einer minimalen Menge von Thrombin. Mit Zunahme der Konzentration von Thrombin wird der Faktor VIIIa schließlich durch Thrombin weiter gespalten und inaktiviert. Diese duale Aktivität des Thrombins in Bezug zum Faktor VIII führt zu einer Selbstbegrenzung der Tenasekomplexbildung und damit zu einer Eingrenzung der Blutgerinnung.

Der extrinsische Weg erfordert einen Gewebefaktor TF (Tissue Faktor) und die Gerinnungsfaktoren V, VII, VIII, IX und X. Bei einer Gefäßverletzung kommt der Gewebefaktor TF (Tissue Faktor) mit dem Gerinnungsfaktor VII zusammen und dieser wird aktiviert. Der Komplex aus TF und dem Gerinnungsfaktor VII hat zwei Substrate, die Gerinnungsfaktoren X und IX.

Der Gerinnungsfaktor IX kann über den intrinsischen Weg und den extrinsischen Weg aktiviert werden. Die Aktivierung von Faktor IXa stellt somit eine zentrale Schnittstelle zwischen beiden Wegen der Gerinnungsaktivierung dar.

Der Faktor IXa hat eine wichtige Rolle in der Blutgerinnung. Defekte am Faktor IXa führen zu Hämophilie B, während erhöhte Konzentrationen von Faktor IXa im Blut zu einem signifikant erhöhten Risiko von Thrombosebildung führen (Weltermann A, et al., J Thromb Haemost. 2003; 1: 28-32). Die Regulation der Faktor IXa Aktivität kann die Thrombusbildung in Tiermodellen reduzieren (Feuerstein GZ, et al., Thromb Haemost. 1999; 82: 1443-1445).

In der Internationalen Anmeldung WO2005/058868 A1 werden durch Heterocyclen modifizierte Mandelsäure-Derivate beschrieben, die sich als Inhibitoren der Gerinnungsfaktoren Xa, IXA und Thrombin eignen. In der Internationalen Anmeldung WO94/22885 werden durch sulfatierte Disaccharide beschrieben, die sich als Inhibitor des Gerinnungsfaktors IXA eignen. Douglas G. Batt et al. (Bioorganic & Medicinal Chemistry Letters, Bd. 14, Nr. 21 (2004), Seiten 5269 - 5273) beschreiben 5-Amidino-lndolderivate die sich als duale Inhibitoren der Gerinnungsfaktoren IXa und Xa eignen.

Die erfindungsgemäßen Verbindungen der Formel I eignen sich für die prophylaktische als auch für die therapeutische Anwendung am Menschen, die an Erkrankungen leiden, die mit Thrombosen, Embolien, Hyperkoagulabilität oder fibrotischen Veränderungen einhergehen. Sie können zur Sekundär-Prävention eingesetzt werden und eignen sich sowohl für eine akute als auch für eine Langzeittherapie.

Die Erfindung betrifft daher eine Verbindung der Formel 1 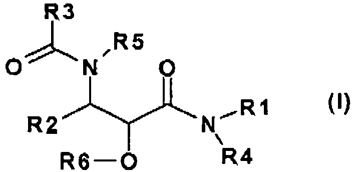 und/oder alle stereoisomeren Formen der Verbindung der Formel und/oder Gemische dieser Formen in jedem Verhältnis, und/oder ein physiologisch verträgliches Salz der Verbindung der Formel I, wobei
- R1 für: 1) -(C₆-C₁₄)-Aryl-Z, wobei Aryl ausgewählt ist aus der Gruppe Phenyl und Naphthyl und worin Aryl unsubstituiert oder ein-, zwei- oder dreifach durch T substituiert ist und Z Amino, Amidino, Aminomethylen, Aminopyridinyl, Azetidinyl, Guanidino, Piperidinyl, Pyridinyl oder Pyrrolidinyl bedeutet, oder
2) ein vier- bis fünfzehngliedrigen Het-Z, wobei Het ausgewählt ist aus der Gruppe Acridinyl, Azepinyl, Azetidinyl, Benzimidazolinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, beta-Carbolinyl, Chinazolinyl, Chinolinyl, Chinolizinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, Dioxolenyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Phenanthridinyl, Phenanthrenyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinofinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazinyl, Thiazolyl, Thienyl, Thienoimidazolyl, Thienooxazolyl, Thienopyridinyl, Thienopyrrolyl, Thienothiazolyl, Thienothiophenyl, Thiomorpholinyl, Thiopyranyl, Triazinyl, 1,2,3-Triazolyl, 1,2,4-Triazolyl, 1,2,5-Triazolyl, 1,3,4-Triazolyl oder Xanthenyl und worin Het unsubstituiert oder ein-, zwei- oder dreifach durch T substituiert ist und worin Z wie oben definiert ist, steht,
- R2 für: -(C₀-C₄)-Alkylen-(C₆-C₁₄)-Phenyl, worin Phenyl unsubstituiert oder ein-, zwei- oder dreifach durch T substituiert ist, steht,

- R3 für: 1) -(C₀-C₄)-Alkylen-(C₆-C₁₄)-Aryl, worin Aryl wie oben definiert ist und unsubstituiert oder ein-, zwei- oder dreifach durch T substituiert ist,
2) -(C₀-C₄)-Alkylen-Het, worin Het wie oben definiert ist und unsubstituiert oder ein-, zwei- oder dreifach durch T substituiert ist,
3) -(C₀-C₄)-Alkylen-(C₆-C₁₄)-Aryl-Q-(C₆-C₁₄)-Aryl, worin die beiden Aryle jeweils unabhängig voneinander wie oben definiert sind und jeweils unabhängig voneinander unsubstituiert oder ein-, zwei- oder dreifach durch T substituiert sind,
4) -(C₀-C₄)-Alkylen-(C₆-C₁₄)-Aryl-Q-(C₃-C₁₂)-Cycloalkyl, worin Aryl wie oben definiert ist und Cycloalkyl jeweils unabhängig voneinander unsubstituiert oder ein-, zwei- oder dreifach durch T substituiert sind,
5) -(C₀-C₄)-Alkylen-(C₆-C₁₄)-Aryl-Q-Het, worin Aryl und Het wie oben definiert sind und jeweils unabhängig voneinander unsubstituiert oder ein-, zwei- oder dreifach durch T substituiert sind,
6) -(C₀-C₄)-Alkylen-Het-Q-(C₆-C₁₄)-Aryl, worin Aryl und Het wie oben definiert sind und jeweils unabhängig voneinander unsubstituiert oder ein-, zwei- oder dreifach durch T substituiert sind, oder
7) -(C₀-C₄)-Alkylen-Het-Q-Het, worin die beiden Het-Reste wie oben definiert sind und jeweils unabhängig voneinander unsubstituiert oder ein-, zwei- oder dreifach durch T substituiert sind, steht,
- Q für: kovalente Bindung, -(C₁-C₄)-Alkylen, -NH-, -N((C₁-C₄)-Alkyl)- oder -O-steht,
- T für: 1) Halogen,
2) -(C₁-C₆)-Alkyl, worin Alkyl unsubstituiert oder unabhängig voneinander ein, zwei- oder dreifach durch -(C₁-C₃)-Fluoralkyl, -N-C(O)-OH oder -N-C(O)-(C₁-C₄)-Alkyl substituiert sind,
3) -(C₁-C₃)-Fluoralkyl,
4) -(C₃-C₆)-Cycloalkyl,
5) -OH,
6) -O-(C₁-C₄)-Alkyl,
7) -O-(C₁-C₃)-Fluoralkyl,
8) -NO₂,
9) -CN,
10) -N(R10)(R11), worin R10 und R11 unabhängig voneinander Wasserstoffatom, -(C₃-C₆)-Cycloalkyl, Halogen oder -(C₁-C₆)-Alkyl bedeuten,
11) -C(O)-NH-R10,
12) -NH-C(O)-R10,
13) -NH-SO₂-R10,
14) -SO₂-(C₁-C₄)-Alkyl,
15) -SO₂-NH-R10,
16) -SO₂-(C₁-C₃)-Fluoralkyl,
17) -S-(C₁-C₄)-Alkyl oder
18) -S-(C₁-C₃)-Fluoralkyl, steht,
- R4 und R5: gleich oder verschieden sind und unabhängig voneinander Wasserstoffatom oder -(C₁-C₄)-Alkyl bedeuten, und
- R6 für: Wasserstoffatom, -C(O)-R12, -C(O)-O-R12, -C(O)-NH-R12 oder -(C₁-C₄)-Alkyl steht, wobei
- R12: -(C₁-C₆)-Alkyl, -(C₃-C₆)-Cycloalkyl, -(C₆-C₁₄)-Aryl oder Het bedeutet.

Die Erfindung betrifft ferner eine Verbindung der Formel I
und/oder alle stereoisomeren Formen der Verbindung der Formel I und/oder Gemische dieser Formen in jedem Verhältnis, und/oder ein physiologisch verträgliches Salz der Verbindung der Formel I, wobei
- R1 für: 4-Benzamidin, Aminomethylphenyl oder Het-Z steht, wobei Het ausgewählt ist aus der Gruppe Benzimidazolyl, Benzthiazolyl und Isochinolinyl, und worin Z für Amino steht,
- R2 für: Phenyl, worin Phenyl unsubstituiert oder ein- oder zweifach durch T substituiert ist, steht,

- R3 für: 1) Phenyl, worin Phenyl unsubstituiert oder ein- oder zweifach durch T substituiert ist,
2) Het-2, worin Het-2 ausgewählt ist aus der Gruppe Benzimidazolyl, Benzofuranyl, Benzothiophenyl, Chinolinyl, Chinoxalinyl, Furanyl, Indolyl, Isochinolinyl, Isoxazolyl, Morpholinyl, Piperidinyl, Pyridyl, Pyrimidinyl, Pyrrolidinyl, Pyrrolyl, Thienyl, Thienopyrrolyl oder Thienothiophenyl und worin Het-2 unsubstituiert oder ein- oder zweifach durch T substituiert ist,
3) -Phenyl-Q-Phenyl, worin die beiden Phenylreste jeweils unabhängig voneinander unsubstituiert oder ein- oder zweifach durch T substituiert sind,
4) Phenyl-Q-(C₃-C₆)-Cycloalkyl, worin Phenyl und Cycloalkyl jeweils unabhängig voneinander unsubstituiert oder ein- oder zweifach durch T substituiert sind,
5) Phenyl-Q-Het-2, worin Het-2 wie oben definiert ist und Phenyl und Het-2 jeweils unabhängig voneinander unsubstituiert oder ein- oder zweifach durch T substituiert sind,
6) Het-2-Q-Phenyl, worin Het-2 wie oben definiert ist und Phenyl und Het-2 jeweils unabhängig voneinander unsubstituiert oder ein- oder zweifach durch T substituiert sind, oder
7) Het-2-Q-Het-2, worin die beiden Het-2-Reste wie oben definiert sind und jeweils unabhängig voneinander unsubstituiert oder ein- oder zweifach durch T substituiert sind, steht,
- Q für: kovalente Bindung, -CH₂-, -N(CH₃)- oder -O- steht,
- T für: 1) F, Cl oder Br,
2) -(C₁-C₄)-Alkyl, worin Alkyl unsubstituiert oder unabhängig voneinander ein- oder zweifach durch -CF₃ oder -N-C(O)-CH₃ substituiert sind,
3) -CF₃,
4) -O-(C₁-C₄)-Alkyl,
5) -O-CF₃,
6) -NO₂,
7) -N(R10)(R11), worin R10 und R11 unabhängig voneinander Wasserstoffatom oder -(C₁-C₄)-Alkyl bedeuten,
8) -SO₂-CH₃,
9) -S-CF₃ oder
10) -S-(C₁-C₂)-Alkyl, steht,
- R4, R5 und R6: jeweils Wasserstoffatom bedeuten.

Unter dem Begriff "(C₁-C₄)-Alkyl" oder "(C₁-C₆)-Alkyl" werden Kohlenwasserstoff-Reste verstanden, deren Kohlenstoffkette geradkettig oder verzweigt ist und 1 bis 4 Kohlenstoffatome oder 1 bis 6 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutan oder Neohexyl.

Unter dem Begriff "-(C₀-C₄)-Alkylen" werden Kohlenwasserstoffreste verstanden, deren Kohlenstoffkette geradkettig oder verzweigt ist und 0 bis 4 Kohlenstoffatome enthält, beispielsweise Methylen, Ethylen, Propylen, Iso-Propylen, Iso-Butylen, Butylen oder tertiär-Butylen. "-C₀-Alkylen" ist eine kovalente Bindung. Unter dem Begriff "-(C₁-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen (-CH₂-), Ethylen (-CH₂-CH₂-), Propylen (-CH₂-CH₂-CH₂-), [Iso-Propylen, Iso-Butylen, Butylen oder tertiär-Butylen.

Unter dem Begriff "-(C₃-C₁₂)-Cycloalkyl" werden Ringe aus 3 bis 12 Kohlenstoff-Atomen verstanden wie Verbindungen, die sich von Monocyclen mit 3 bis 8 Kohlenstoffatomen im Ring wie Cyclopropan, Cyclobutan, Cyclopentan, Cyclohexan, Cycloheptan oder Cyclooctan herleiten, die sich von den Bicyclen Bicyclo[4.2.0]octan, Octahydro-inden, Decahydro-naphthalen, Decahydro-azulen, Decahydro-benzocyclohepten oder Dodecahydro-heptalen herleiten oder von den überbrücken Cyclen wie Spiro[2.5]octan, Spiro[3.4]octan, Spiro[3.5]nonan, Bicyclo[3.1.1]heptan, Bicyclo[2.2.1]heptan oder Bicyclo[2.2.2]octan herleiten.

Unter dem Begriff "-(C₃-C₈)-Cycloalkyl" bzw. "-(C₃-C₆)-Cycloalkyl" werden Reste verstanden, die sich von Monocyclen mit 3 bis 8 bzw. 3 bis 6 Kohlenstoffatomen im Ring ableiten wie Cyclopropan, Cyclobutan, Cyclopentan, Cyclohexan, Cycloheptan oder Cyclooctan.

Unter dem Begriff "-(C₆-C₁₄)-Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Anthryl oder Fluorenyl. Naphthylreste und insbesondere Phenylreste sind bevorzugte Arylreste.

Unter dem Begriff "vier- bis fünfzehngliedrigen Het"oder "Het" werden Ringsysteme verstanden mit 4 bis 15 Kohlenstoffatomen, die in ein, zwei oder drei miteinander verbundenen Ringsystemen vorliegen und in denen ein, zwei, drei oder vier gleiche oder verschiedene Heteroatome aus der Reihe Sauerstoff, Stickstoff oder Schwefel die jeweiligen Kohlenstoffatome ersetzen können. Beispiele für diese Ringsysteme sind die Reste Acridinyl, Azepinyl, Azetidinyl, Benzimidazolinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, beta-Carbolinyl, Chinazolinyl, Chinolinyl, Chinolizinyl, 4H-Chinolizinyl, Chinoxalinyl, Chinuclidinyl, Chromanyl, Chromenyl, Cinnolinyl, Decahydrochinolinyl, Dibenzofuranyl, Dibenzothiophenyl, Dihydrofuran[2,3-b]-tetrahydrofuranyl, Dihydrofuranyl, Dioxolyl, Dioxanyl, Dioxolenyl, 2H, 6H-1,5,2-Dithiazinyl, Furanyl, Furazanyl, Imidazolidinyl, Imidazolinyl, Imidazolyl, 1 H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Phenanthridinyl, -Phenanthrenyl, Phenanthrolinyl, -Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pyridooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazinyl, Thiazolyl, Thienyl, Thienoimidazolyl, Thienooxazolyl, Thienopyridinyl, Thienopyrrolyl, Thienothiazolyl, Thienothiophenyl, Thiomorpholinyl, Thiopyranyl, Triazinyl, 1,2,3-Triazolyl, 1,2,4-Triazolyl, 1,2,5-Triazolyl, 1,3,4-Triazolyl oder Xanthenyl.

Unter dem Begriff "-(C₁-C₃)-Fluoralkyl" wird ein partiell oder vollständig fluorierter Alkylrest verstanden, der sich beispielsweise von folgenden Resten ableitet -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, -CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F, -CF₂-CH₂-CF₃, -CF₂-CH₂-CHF₂, -CF₂-CH₂-CH₂F, -CF₂-CF₂-CF₃, -CF₂-CF₂-CHF₂ oder -CF₂-CF₂-CH₂F.

Unter dem Begriff "Halogen" wird Fluor, Chlor, Brom oder Jod verstanden, bevorzugt sind Fluor, Chlor oder Brom, insbesondere Chlor oder Brom.

Unter dem Begriff "eine basische stickstoffhaltige Gruppe" werden Reste verstanden, wobei die konjugierte Säure dieser Gruppe einen pKa von etwa 5 bis 15 hat. Beispiele für diese basische stickstoffhaltige Gruppe sind Amino, Aminomethylen, Amidino (Carbamimidoyl), Guanidino, Azetidinyl, Pyrrolidinyl, Piperidinyl, Pyridinyl oder Aminopyridinyl.

Funktionelle Gruppen der verwendeten Intermediate, beispielsweise Amino- oder Carboxylgruppen, können dabei durch geeignete Schutzgruppen maskiert werden. Geeignete Schutzgruppen für Aminofunktionen sind beispielweise die t-Butoxycarbonyl-, die Benzyloxycarbonyl- oder die Phtalolylgruppe sowie die Trityl- oder Tosylschutzgruppe. Geeignete Schutzgruppen für die Carboxylfunktion sind beispielweise Alkyl-, Aryl- oder Arylalkylester. Schutzgruppen können durch wohlbekannte oder hier beschriebene Techniken eingeführt und entfernt werden (siehe Green, T.W., Wutz, P.G.M., Protective Groups in Organic Synthesis (1991), S. 1-16, 2nd Ed., Wiley-Interscience, oder Kocienski, P., Protecting Groups (1994), Thieme).

Der Begriff Schutzgruppe kann auch entsprechende polymergebundene Schutzgruppen umfassen.

Die erfindungsgemäßen Verbindungen können nach wohlbekannten Verfahren oder nach hier beschriebenen Verfahren hergestellt werden.

Die Erfindung betrifft ferner ein Verfahren zur Herstellung der Verbindung der Formel **I** und/oder einer stereoisomeren Form der Verbindung der Formel I und/oder eines physiologisch verträglichen Salzes der Verbindung der Formel I, das dadurch gekennzeichnet ist, dass man die Verbindung der Formel I nach Schema 1 herstellt. 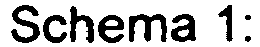 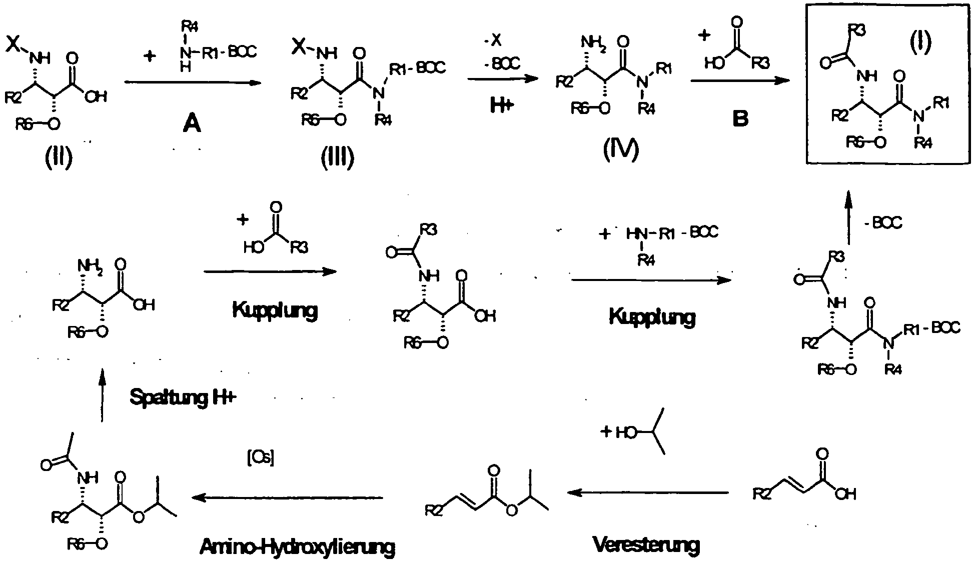 Die in Schema 1 verwendeten Reste R1, R2, R3 und R4 haben dieselbe Bedeutung wie die in der Verbindung der Formel I, X steht für eine Aminoschutzgruppe, BOC steht für die Schutzgruppe Butoxycarbonyl und Os steht für Osmium.

In einem Verfahrensschritt A wird die Verbindung der Formel II, beispielsweise (2R,3S)-3-(Boc-Amino)-2-hydroxy-3-phenyl-propansäure in einem Lösungsmittel wie Dimethylformamid (DMF), Dichloromethan (CH₂Cl₂), Tetrahydrofuran (THF), N-Methylpyrrolidinon (NMP) oder Dioxan gelöst und mit einem geeigneten Amin der Formel NH(R1-BOC)-R4, beispielsweise 6- bzw.7-Amino-iso-chinolin-1-yl)-N-di-carboxyamino tert.-butylester, 2-Amino-6-nitrobenzothiazol oder 2,5-Diamino-benzimidazol-1-carbonsäure-tertiär-butylester zum entsprechenden Amid (III) gekuppelt. Hierzu werden wie oben beschrieben ein übliches Kupplungsreagenz wie TOTU, PyBrop, PyBop, HATU oder EDC und eine geeignete Base wie Aminbasen wie Diisopropylethylamin (Hünigs Base), Triethylamin (NEt₃) oder 4-Dimethylaminopyridin (4-DMAP) verwendet.

Anschließend erhält man durch Abspaltung der Schutzgruppen wie t-Butyloxy-carbonyl (BOC) nach Standardmethoden (wie mit TFA-CH₂Cl₂) Verbindung IV. (Alternative Methoden zur Abspaltung von Schutzgruppen siehe z.B. Kocienski, P.J., Protecting groups, Thieme Verlag 1994).

Durch erneute Amidkupplung mit geeigneten Carbonsäuren des Typs R3-COOH unter analogen Bedingungen wie im Verfahren A beschrieben erhält man schließlich die gewünschten Verbindungen des Typs I.

Bei nicht erhältlichen Verbindungen des Typs II, beispielsweise R2 = Het, können Verbindungen des Typs I auch über eine Route (für R6 = H) ausgehend von den entsprechenden Zimtsäuren hergestellt werden. Hierbei erfolgt im ersten Schritt eine Umsetzung der Zimtsäuren zu den entsprechenden Zimtsäureisopropylestern gefolgt von einer Aminohydroxylierung nach publizierten Standardverfahren. Nach anschließender sauren Acetylabspaltung, zweifacher Amidkupplung (1. mit R3-COOH und 2. mit NH(R1)-R2) und BOC-Abspaltung analog den oben beschriebenen Bedingungen erhält man ebenfalls die gewünschten Verbindungen des Typs I. Für beide beschriebenen Verfahren lassen sich auch die Verbindungen (I) mit R = H prinzipiell direkt in die Derivate mit R6 ungleich H mittels Literaturbekannten Methoden überführen (beispielsweise Esterbildungen oder Carbamoylierungen)

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man
a) eine Verbindung der Formel 11 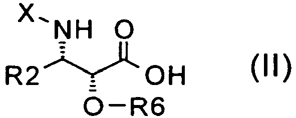 wobei X für eine Aminoschutzgruppe steht und die Reste R2 und R6 wie in Formel I definiert sind mit einer Verbindung NH(R1)(R4) zu einer Verbindung der Formel III umsetzt, 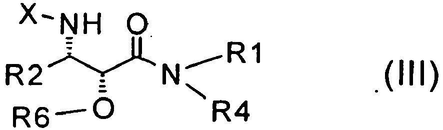 wobei X für eine Aminoschutzgruppe steht und die Reste R1, R2, R4 und R6 wie in Formel I definiert sind und die Verbindung der Formel III durch Abspaltung der Schutzgruppe in eine Verbindung der Formel IV umwandelt, 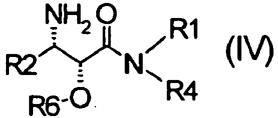 wobei die Reste R1, R2, R4 und R6 wie in Formel I definiert sind und mit der Verbindung der Formel V 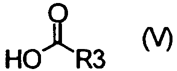 zur Verbindung der Formel umsetzt, oder
b) die nach Verfahren a) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
c) eine nach Verfahren a) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

Eine nach Schema 1 hergestellte Verbindung der Formel I, oder eine geeignete Vorstufe der Formel I, die aufgrund ihrer chemischen Struktur in enantiomeren Formen auftritt, kann durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren aufgetrennt werden (Verfahren c), oder die nach Schema 1 hergestellte Verbindung der Formel I kann entweder in freier Form isoliert oder im Falle des Vorliegens von sauren oder basischen Gruppen in physiologisch verträgliche Salze umwandelt werden (Verfahren b).

Im Verfahrensschritt c) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man,eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literaturbekannten Verfahren enantiomeren- oder diastereomerenrein hergestellt. Das kann insbesondere bedeuten, dass in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze wie Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel I, einschließlich deren stereoisomerer Formen, gemäß Verfahrensschritt b) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze.

Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Zitronen-, Fumar-, Malein-, Glucon-, Glucuron- Palmitin-, oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen beispielsweise zur Prophylaxe, Sekundär-Prävention und Therapie all solcher Erkrankungen, die durch eine Hemmung von Blutgerinnungsfaktor IXa behandelbar sind. So eignen sich die erfindungsgemäßen Verbindungen als Inhibitoren sowohl für eine prophylaktische als auch für eine therapeutische Anwendung am Menschen. Sie eignen sich sowohl für eine akute Behandlung als auch für eine Langzeittherapie. Die Verbindungen der Formel I können eingesetzt werden in Patienten, die an Störungen des Wohlbefindens oder Krankheiten leiden, die mit Thrombosen, Embolien, Hyperkoagulabilität oder fibrotischen Veränderungen einhergehen.

Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können die Verbindungen der Formel I eingesetzt werden bei allen Eingriffen, die zu einem Kontakt des Blutes mit Fremdoberflächen führen wie bei Dialysepatienten und Patienten mit Verweilkathetern. Verbindungen der Formel I können eingesetzt werden, um die Thrombosegefahr nach chirurgischen Eingriffen wie bei Knie- und Hüftgelenksoperationen zu reduzieren.

Verbindungen der Formel I eignen für die Behandlung von Patienten mit disseminierter intravaskulärer Koagulation, Sepsis und anderen intravaskulären Ereignissen, die mit einer Entzündung einhergehen. Weiterhin eignen sich Verbindungen der Formel I für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose. Verbindungen der Formel I eignen sich zur Verlangsamung oder Verhinderung solcher Prozesse.

Weitere Indikationen für den Einsatz der Verbindungen der Formel I sind fibrotische Veränderungen der Lunge wie die chronische obstruktive Lungenerkrankung, das adult respiratory distress syndrome (ARDS) und des Auges wie Fibrinablagerungen nach Augenoperationen. Verbindungen der Formel I eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung von Stents mit Verbindungen der Formel I und anderen Oberflächen, die im Körper mit Blut in Kontakt kommen, ist möglich.

Die Erfindung betrifft auch ein Verfahren zur Herstellung eines Arzneimittels, das dadurch gekennzeichnet, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines Erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Verbindungen der Formel I können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden.

### Beispiele

Endprodukte werden in der Regel durch massenspektroskopische Methoden (FAB-, ESI-MS) und ¹H-NMR bestimmt, angegeben sind jeweils der Hauptpeak oder die beiden Hauptpeaks. Temperaturangaben in Grad Celsius, Ausb. bedeutet Ausbeute. Verwendete Abkürzungen sind entweder erläutert oder entsprechen den üblichen Konventionen. Soweit nicht anders angegeben, wurden chromatographische Trennungen an Kieselgel mit Ethylacetat/Heptan-Gemischen als Laufmittel durchgeführt. Präparative Trennungen an Reversed Phase-(RP)-Kieselgel (HPLC) wurden, soweit nicht anders angegeben, unter folgenden Bedingungen durchgeführt: Säule Merck Hibar RT 250-25 LiChrospher 100 RP-18e 5µm, Merck KGaA, Deutschland, Life Science & Analytics, 64293 Darmstadt; mobile Phase A: H₂O + 0,1% TFA, Phase B: 80% Acetonitril + 0,1% TFA, Fluss 25 ml/ min., 0-7 min. 100% A , 7-22 min. auf 100% B, 22-30 min. 100% B, 30-33 min. auf 100% A, 33-35 min. 100% A. Das Abdampfen von Lösungsmitteln geschah in der Regel unter vermindertem Druck bei 35 °C bis 45 °C am Rotationsverdampfer.

Wenn nicht anders aufgeführt, wurden die LC/MS-Analysen unter folgenden Bedingungen durchgeführt:

| Methode A: | |
|---|---|
| Säule: | YMC J'shere H80 33x2,1 mm; Waters GmbH, Helfmann-Park 10, 65760 Eschbom, Deutschland; Packungsmaterial 4 µm, |
| Lösungsmittel: | ACN+0,05% TFA:H₂O + 0,05% TFA (Fluss 1,3 mL/min) |
| Gradient: | 5:95 (0 min) nach 95:5 (2,5 min) nach 95:5 (3,0 min) |
| Ionisierung: | ESI⁺ |
| | |

| Methode B: | |
|---|---|
| Säule: | YMC J'shere H80 33x2,1 mm; Packungsmaterial 4 µm, |
| Lösungsmittel: | ACN + 0,05% TFA:H₂O + 0,05% TFA (Fluss 1 mL/min) |
| Gradient: | 5:95 (0 min) nach 95:5 (3,4min) nach 95:5 (4,4 min) |
| Ionisierung: | ESI⁺ |
| | |

| Methode C: | |
|---|---|
| Säule: | YMC J'shere H80 33x2.1mm 4µm |
| Lösungsmittel: | ACN + 0,08% TFA:H₂O + 0,1% TFA (Fluss 1,3 mL/min) |
| Gradient: | von 5:95 (0 min) nach 95:5 (2,5 min) nach 95:5 (3 min) |
| Ionisierung: | ESI⁺ |
| | |

| Methode D: | |
|---|---|
| Säule: | YMC J'shere ODS H80 20x2,1 mm Packungsmaterial 4 µm, |
| Lösungsmittel: | ACN:H₂O + 0,05% TFA (Fluss 1 mL/min) |
| Gradient: | 4:96 (0 min) nach 95:5 (2 min) nach 95:5 (2,4 min) nach 96:4 (2,45 min) |
| Ionisierung: | ESI⁺ |

Präparative HPLC wurde mit der folgenden Methode durchgeführt:

| | |
|---|---|
| Säule: | Waters Atlantis dC18 OBD 30x100 mm 5µm; Waters GmbH, Helfmann-Park 10, 65760 Eschborn, Deutschland |
| Lösungsmittel: | ACN:H₂O + 0,1%TFA (Fluss 60 mL/min) |
| Gradient: | 10:90 (0 min) bis 90:10 (10 min) |

### Verwendete Abkürzungen:

| | |
|---|---|
| ACN | Acetonitril |
| Boc | Butoxycarbonyl |
| DCM | Dichlormethan |
| (DHQ)₂PHAL | 1-[(R)-((4S,5R)-5-Ethyl-1-aza-bicyclo[2.2.2]oct-2-yl)-(6-methoxy-chinolin-4-yl)-methoxy]-4-[(R)-((4R,5S)-5-ethyl-1-aza-bicyclo [2.2.2]oct-2-yl)-(6-methoxy-chinolin-4-yl)-methoxy]-phthalazin |
| DIPEA | N,N-Diisopropylethylamin (Hünigs Base) |
| DMF | Dimethylformamid |
| DMSO | Dimethylsulfoxid |
| EDC | N'-(3-Dimethylaminopropyl)-N-ethylcarbodiimid |
| HATU | O-(7-Azabenzotriazol-1-yl)-N,N,N',N'-tetramethyluronium hexafluor-phosphat |
| HOAt | 1-Hydroxy-7-azabenzotriazol |
| K₂[OsO₂(OH)₄) | Kaliumosmat-dihydrat |
| LC/MS | Flüssigkeitschromatographie Massenspektroskopie |
| MeOH | Methanol |
| NMM | N-Methylmorpholin |
| PyBop | 1-Benzotriazolyloxy-tripyrrolidinophosphoniumhexafluor-phosphat |
| PyBrop | Brom-tris-pyrrolidin-phosphonium hexafluorphosphat |
| Rₜ | Retentionszeit |
| TFA | Trifluoressigsäure |
| TOTU | O-((Ethoxycarbonyl)cyanomethylenemino)-N,N,N',N'-tetramethyluronium tetrafluorborat |
| RT | Raumtemperatur (21 °C bis 24 °C) |

### Beispiel 1

### N-[(1S,2R)-2-(1-Amino-isochinolin-7-ylcarbamoyl)-2-hydroxy-1-phenyl-ethyl]-4-tert.-butyl-benzamid; Verbindung mit Trifluoressigsäure

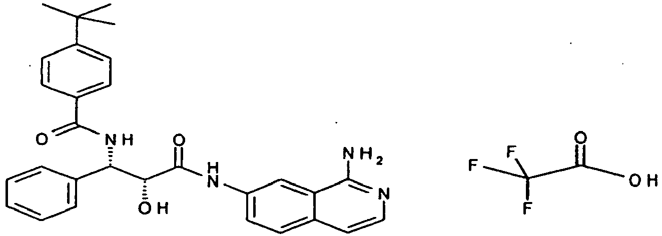

### Verfahrensschritt 1:

### [7-((2R,3S)-3-tert-Butoxycarbonylamino-2-hydroxy-3-phenyl-propionylamino)-isochinolin-1-yl]-N-di-carboxyamino tert-butylester

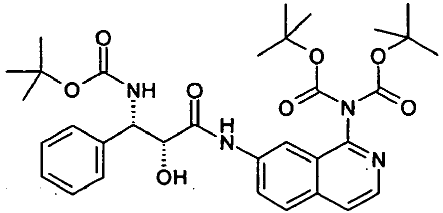

Zu einer Lösung von 0,75 g (2,08 mmol) 7-Amino-isochinolin-1-yl-N-di-carboxyamino tert.-butylester, (2R,3S)-3-(Boc-Amino)-2-hydroxy-3-phenyl-propansäure (0,587 g, 2,08 mmol) und HOAt (0,284 g, 2.08 mmol) in 10 mL DMF wurden 0,69 mL (6,26 mmol) NMM unter Rühren für 10 Minuten (min) zugefügt. Nach der Zugabe von 0,973 g PyBrop (2,08 mmol) wurde die Mischung bei RT für 18 Stunden (h) gerührt. Anschließend wurde zur Reaktionsmischung Wasser hinzugefügt, mit Ethylacetat extrahiert, mit Natriumsulfat getrocknet, filtriert und unter verminderten Druck eingeengt und mit Kieselgel-Chromatographie (Ethylacetat : n-Heptan 1:2) gereinigt.

Es wurden 0,56 g (Ausbeute: 43%) der gereinigten Titelverbindung erhalten.
LC/MS (Methode D) (M+H-BOC)⁺ 523
7-Amino-isochinolin-1-yl-N- di-carboxyamino tert-butylester wurde wie in dem in WO 00/71507, Seite 92, beschriebenen Verfahren erhalten.

### Verfahrensschritt 2:

### (2R,3S)-3-Amino-N-(1-amino-isochinolin-7-yl)-2-hydroxy-3-phenyl-propionsäureamid; Verbindung mit Trifluoressigsäure

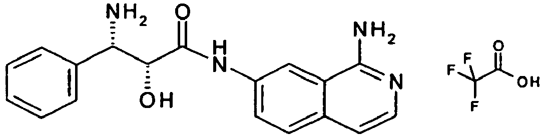

Zu 0,56 g (0,89 mmol) der Verbindung aus Verfahrensschritt 1 in DCM (9 mL) wurden 3 mL TFA zugefügt. Anschließend wurde die Reaktionsmischung 3 h bei RT gerührt. Die Lösungsmittel wurden unter verminderten Druck abdestilliert und der Feststoff wurde in MeOH und Wasser aufgenommen und abschließend lyophilisiert. Es wurden 0, 485 g (Ausbeute: 99%) der gereinigten Titelverbindung als weißer Feststoff erhalten. LC/MS (Methode D) (M+H)⁺ 323

### Verfahrensschritt 3:

### N-[(1S,2R)-2-(1-Amino-isochinolin-7-ylcarbamoyl)-2-hydroxy-1-phenyl-ethyl]-4-tert-butyl-benzamid; Verbindung mit Trifluoressigsäure

Zu einer Lösung von 40 mg (0,073 mmol) der Verbindung aus Verfahrensschritt 2, 4-tertiär-Butylbenzoesäure (12,9 mg, 0,073 mmol), HOAt (9,89 mg, 0,073 mmol) and HATU (27,6 mg, 0,073 mmol) in DMF (1,5 mL) wurde 0,038 mL (0,22 mmol) DIPEA zugefügt. Anschließend wurde die Reaktionsmischung 42 h bei RT gerührt. Die Reaktionsmischung wurde filtriert und über eine präparative Hochdruckflüssigchromatographie (HPLC) gereinigt. Die gereinigten Fraktionen des Produktes wurden lyophilisiert und es wurde ein weißer Feststoff erhalten. Ausbeute 53 %
LCMS (Methode A) 482,23 (Rₜ= 1,43 min, 100%)
¹H NMR (500 MHz, DMSO-d₆) δ (ppm): 1,28 (s, 9H), 4,54 (t, 1 H), 5,56 (dd, 1 H), 6,31 (d, 1 H), 7,18 (d, 1 H), 7,25 (t, 1 H), 7,33 (t, 2H), 7,47 (d, 4H), 7,58 (d, 1 H), 7,78 (d, 2H), 7,91 (d, 1 H), 8,01 (dd, 1 H), 8,45 (d, 1 H), 8,74 (s, 1 H), 8,91 (s, 2H), 10,36 (s, 1 H), 12,90 (s, 1 H)

### Beispiel 2:

### N-[(1S,2R)-2-(1-Amino-isochinolin-6-ylcarbamoyl)-2-hydroxy-1-phenyl-ethyl]-4-diethylamino-benzamide; Verbindung mit HCl

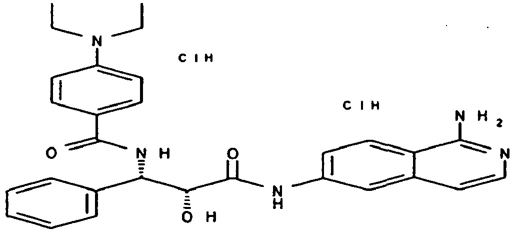

### Verfahrensschritt 1:

### [6-((2R,3S)-3-tert.-Butoxycarbonylamino-2-hydroxy-3-phenyl-propionylamino)-isochinolin-1-yl]-N-di-carboxyamino tert-butylester

In analoger Weise zur Herstellung von Beispiel 1, Verfahrensschritt 1, wurde 6-Amino-iso-chinolin-1-yl)-N-di-carboxyamino tert-butylester (1,5 g, 4,17 mmol) umgesetzt. Es wurden 0,82 g der Titelverbindung erhalten; Ausbeute: 32% von einem Feststoff.
LC/MS (Methode D) (M+H)⁺ 623

6-Amino-iso-chinolin-1-yl)-N- di-carboxyamino tert-butylester wurde wie in WO2004/072101, Seite 108, beschriebenen Verfahren erhalten.

### Verfahrensschritt 2:

### (2R,3S)-3-Amino-N-(1-amino-isochinolin-6-yl)-2-hydroxy-3-phenyl-propionamid; Verbindung mit Trifluoressigsäure

In analoger Weise zur Herstellung von Beispiel 1, Verfahrensschritt 2, wurde die Verbindung aus Verfahrensschritt 1 (0,82 g, 1,32 mmol) umgesetzt. Es wurden 0,71 g der Titelverbindung (Ausbeute: 98%) als Feststoff erhalten.
LC/MS (Methode D) (M+H)⁺ 323

### Verfahrensschritt 3:

### N-[(1S,2R)-2-(1-Amino-isochinolin-6-ylcarbamoyl)-2-hydroxy-1-phenyl-ethyl)-4-diethylamino-benzamid; Verbindung mit Salzsäure

Das erhaltene Produkt aus Verfahrensschritt 2 (0,6 g, 1,09 mmol), 4-Diethylaminobenzoesäure (0,21 g, 1,09 mmol), HOAt (148 mg, 1,09 mmol) und HATU (415 mg, 1,09 mmol) wurden in 10 mL DMF gelöst und 0,56 mL DIPEA (3,27 mmol) wurden zugefügt. Anschließend wurde die Reaktionsmischung 18 h bei RT gerührt. Die Reaktionsmischung wurde filtriert und über eine präparative HPLC gereinigt. Die gereinigten Fraktionen des Produktes wurden lyophilisiert,
in 2 mL MeOH gelöst und anschließend mit 5 mL einer 5 M HCl behandelt und erneut lyophilisiert. Es wurden 278 mg eines weißen Feststoffs erhalten.
Ausbeute: 45 %
LC/MS (Methode B) 497,24 (Rₜ= 1,15 min, 100%)
¹H NMR (500 MHz, DMSO-d₆) δ (ppm): 1,08 (t, 6H), 3,38 (d, 4H), 4,56 (d, 1 H), 5,54 (dd, 1 H), 6,65 (s, 2H), 7,14 (d, 1 H), 7,24 (t, 1 H), 7,32 (t, 2H), 7,45 (d, 2H), 7,59 (t, 1 H), 7,71 (s, 2H), 7,91 (dd, 1 H), 8,15 (s, 1 H), 8,29 (d, 1 H), 8,49 (d, 1 H), 8,90 (s, 2H), 10,64 (s, 1 H), 12,90 (s, 1 H)

### Beispiel 3:

### N-[(1S,2R)-2-(2-Amino-benzothiazol-6-ylcarbamoyl)-2-hydroxy-1-phenyl-ethyl]-4-isopropyl-benzamid; Verbindung mit Trifluoressigsäure

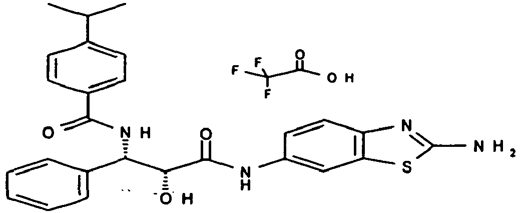

### Verfahrensschritt 1:

### Benzothiazol-2,6-diamin

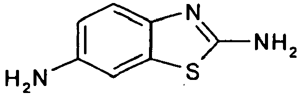

2-Amino-6-nitrobenzothiazol (1,0 g, 5,12 mmol) wurde in 100 mL MeOH gelöst und Palladium auf aktiviertem Kohlenstoff (10%, 545 mg, 0,51 mmol) wurde zugefügt. Die Reaktionsmischung wurde mit Wasserstoff (3 bar H₂) bei RT für 2,5 h hydriert. Die Mischung wurde filtriert, das Lösungsmittel wurde unter verminderten Druck entfernt und der Rückstand wurde mit Kieselgel-Chromatographie (Ethylacetat : n-Heptan 1:2) gereinigt. Es wurden 0,81 g (Ausbeute: 96%) der gereinigten Titelverbindung erhalten.
LC/MS (Methode D) (M+H)⁺ 166

### Verfahrensschritt 2:

### [(1S, 2R)-2-(2-Amino-benzothiazol-6-ylcarbamoyl)-2-hydroxy-1-phenyl-ethyl]-carboxyamino tert.-butylester

In analoger Weise zur Herstellung von Beispiel 1, Verfahrensschritt 1, wurde die erhaltene Verbindung aus Verfahrensschritt 1 (71 mg, 0,43 mmol) umgesetzt. Es wurden 180 mg der Titelverbindung erhalten; Ausbeute: 98 % von einem Feststoff.
LC/MS (Methode D) (M+H)⁺ 429

### Verfahrensschritt 3:

### (2R, 3S)-3-Amino-N-(2-amino-benzothiazol-6-yl)-2-hydroxy-3-phenyl-propionamid; Verbindung mit Trifluoressigsäure

In analoger Weise zur Herstellung von Beispiel 1, Verfahrensschritt 2, wurde die erhaltene Verbindung aus Verfahrensschritt 2 (180 mg, 0,42 mmol) umgesetzt. Es wurden 177 mg der Titelverbindung erhalten; Ausbeute: 76 % von einem Feststoff.
LC/MS (Methode D) (M+H)⁺ 329

### Verfahrensschritt 4:

### N-[(1S, 2R)-2-(2-Amino-benzothiazol-6-ylcarbamoyl)-2-hydroxy-1-phenyl-ethyl]-4-isopropyl-benzamid; Verbindung mit Trifluoressigsäure

Das erhaltene Produkt aus Verfahrensschritt 3 (78 mg, 0,14 mmol), 4-Isopropylbenzoesäure (23 mg, 0,14 mmol), HOAt (19 mg, 0,14 mmol) und HATU (53 mg, 0,14 mmol) wurden in 2 mL DMF gelöst und 0,046 mL NMM (0,42 mmol) wurden zugefügt. Anschließend wurde die Reaktionsmischung 18 h bei RT gerührt. Die Reaktionsmischung wurde filtriert und über eine präparative HPLC gereinigt. Die gereinigten Fraktionen des Produktes wurden lyophilisiert. Es wurden 33 mg eines weißen Feststoffs erhalten. Ausbeute: 40 %
LC/MS (Methode A) 474,17 (Rₜ= 1,63 min, 100%)
¹H NMR (500 MHz, DMSO-d₆) δ (ppm): 1,20 (d, 6H), 2,94 (h, 1 H), 4,44 (d, 1 H), 5,49 (dd, 1 H), 7,24 (t, 1 H), 7,28-7,34 (m, 5H), 7,38 (dd, 1 H), 7,44 (d, 2H), 7,77 (d, 2H), 8,05 (d, 1 H), 8,15 (s, 1 H), 8,43 (d, 1 H), 9,95 (s, 1 H)

### Nicht erfindungsgemäßes Beispiel 4:

N-[(1 R, 2R)-2-(1-Amino-isochinolin-6-ylcarbamoyl)-1-furan-2-yl-2-hydroxy-ethyl]-4-diethylamino-benzamid; Verbindung mit Trifluoressigsäure

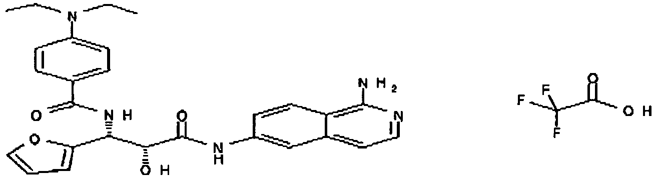

### Verfahrensschritt 1:

### (E)-3-Furan-2-yl-acrylsäure-Isopropylester

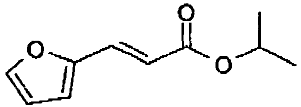

Es wurden zu einer Lösung von 1,38 g 3-(2-Furyl)-acrylsäure (10 mmol) in 10 mL Chloroform und 0,1 mL DMF 1,46 mL Thionylchlorid (20 mmol) zugefügt. Die Lösung wurde auf 70 °C erwärmt und 1 h bei dieser Temperatur gehalten, danach wurden die Lösungsmittel unter verminderten Druck entfernt und der Rückstand wurde in 6,6 mL DCM und 3,3 mL Pyridin gelöst und anschließend auf 0 °C gekühlt. Es wurden 0,96 mL 2-Propanol (12,5 mmol) zugefügt, die Temperatur wurde auf RT erhöht und das Reaktionsgemisch wurde für 2,5 h gerührt. Es wurde mit 30 mL einer 1 M Salzsäure angesäuert und mit 150 mL Ethylacetat extrahiert. Die organische Phase wurde mit wässriger Natriumhydrogencarbonat Lösung gewaschen, über Natriumsulfat getrocknet, filtriert und unter verminderten Druck wurden die Lösungsmittel entfernt. Es wurden 1,72 g (Ausbeute: 96 %) der Titelverbindung erhalten. LC/MS (Methode D) (M+H-isopropyl)⁺ 139

### Verfahrensschritt 2:

### (2R, 3R)-3-Acetylamino-3-furan-2-yl-2-hydroxy-propionsäureisopropylester

Die Umsetzung erfolgte wie bei Sharpless et. al. Angew. Int. Ed. 1997, 36, 1483, beschrieben.

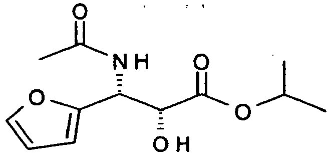

Zu einer Lösung enthaltend 128 mg Lithiumhydroxid-Monohydrat (3,06 mmol) und 44,5 mg K₂[OsO₂ (OH)₄] (0,12 mmol) in 7 mL Wasser wurden 14 mL tertiär-Butanol und 117 mg (DHQ)₂PHAL (0,15 mmol) zugefügt und die Lösung wurde für 10 min bei RT gerührt. Nach der Zugabe von 14 mL Wasser wurde die Mischung auf 4 °C gekühlt und 0,54 g (3 mmol) der Verbindung gemäß Verfahrensschritt 1 und 455 mg N-Bromacetamid (3,3 mmol) wurden zugefügt. Die Mischung wurde bei 4 °C für 3,5 h gerührt. Danach wurden 1,2 g Natriumsulfit zugegeben und 30 min bei RT gerührt. Wasser wurde zugefügt und die wässrige Phase wurde mit Ethylacetat extrahiert. Die organische Phase wurde mit Natriumsulfat getrocknet, filtriert, unter verminderten Druck wurde das Lösungsmittel entfernt und es wurde mit Kieselgel-Chromatographie (Ethylacetat: n-Heptan 2:3) gereinigt. Es wurden 112 mg (Ausbeute: 15 %) der gereinigten Titelverbindung erhalten.
LC/MS (Methode D) (M+H)⁺ 256

### Verfahrensschritt 3:

(2R, 3R)-3-Amino-3-furan-2-yl-2-hydroxy-propionsäure; Verbindung mit Salzsäure

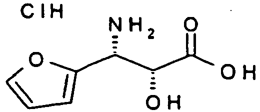

112 mg der erhaltenen Verbindung aus Verfahrensschritt 2 (0,44 mmol) wurden in 8 mL Salzsäure (10 %) gelöst und auf 110 °C für 3 h erhitzt. Es wurde Wasser zur Reaktionsmischung zugefügt und nach Lyophilisierung wurden 91 mg (Ausbeute 100 %) der reinen Titelverbindung erhalten.
LC/MS (Methode D) (M+H-NH₂)⁺ 155

### Verfahrensschritt 4:

### (2R, 3R)-3-(4-Diethylamino-benzoylamino)-3-furan-2-yl-2-hydroxy-propionsäure

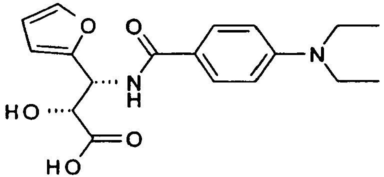

89 mg 4-Diethylaminobenzoesäure (0,46 mmol) und 152 mg TOTU (0,46 mmol) wurden in 2 mL DMF gelöst und 255 µL NMM (2,31 mmol) wurden zugefügt. Die Reaktionsmischung wurde bei RT für 30 min gerührt und 96 mg des Produkts aus Verfahrensschritt 3 (0,46 mmol) wurden zugefügt. Anschließend wurde die Reaktionsmischung 18 h bei RT gerührt. Die Mischung wurde filtriert und über eine präparative HPLC gereinigt. Die gereinigten Fraktionen des Produktes wurden lyophilisiert. Es wurden 142 mg eines weißen Feststoffs erhalten. Ausbeute: 89 % LC/MS (Methode D) (M+H)⁺ 347

### Verfahrensschritt 5:

### {6-[(2R,3R)-3-(4-Diethylamino-benzoylamino)-3-furan-2-yl-2-hydroxy-propionylamino]-isochinolin-1-yl}-N-di-carboxyamino tert-butylester

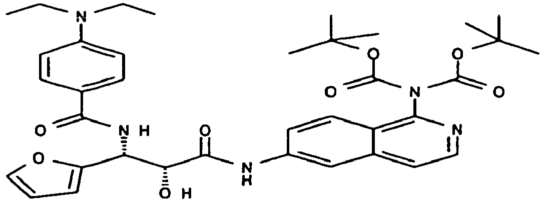

48,2 mg des in Verfahrensschritt 4 erhaltenen Produkts (0,14 mmol), 50 mg 7-Amino-isochinolin-1-yl)-N- di-carboxyamino tert-butylester (0,14 mmol), 19 mg HOAt (0,14 mmol) und 64,8 mg PyBrop (0,14 mmol) wurden in 1,5 mL DMF gelöst und anschließend wurden 61,3 µL NMM (0,56 mmol) zugefügt und die Reaktionsmischung wurde danach bei RT für 42 h gerührt. Die Mischung wurde filtriert und über eine präparative HPLC gereinigt. Die gereinigten Fraktionen des Produktes wurden lyophilisiert. Danach wurde der lyophilisierte Feststoff gelöst und mit Kieselgel-Chromatographie (DCM : MeOH 30:1) gereinigt. Es wurden 13 mg (Ausbeute: 14%) der gereinigten Titelverbindung erhalten.
LC/MS (Methode D) (M+H)⁺ 688

### Verfahrensschritt 6:

### N-[(1 R,2R)-2-(1-Amino-isochinolin-6-ylcarbamoyl)-1-furan-2-yl-2-hydroxy-ethyl]-4-diethylamino-benzamid; Verbindung mit Trifluoressigsäure

Das erhaltene Produkt aus Verfahrensschritt 5 (13 mg, 18,9 µmol) wurde in 3 mL DCM gelöst und 1 mL TFA wurde zugefügt. Anschließend wurde die Reaktionsmischung 1 h bei RT gerührt. Die Reaktionsmischung wurde filtriert und bei vermindertem Druck wurden die Lösungsmittel entfernt. Der Rückstand wurde in MeOH und Wasser gelöst, lyophilisiert und es wurde 11 mg eines weißen Feststoffs erhalten. Ausbeute: 97 %.
LC/MS (Methode A) 487,22 (Rₜ= 1,00 min, 100%)
¹H NMR (500 MHz, DMSO-d₆) δ (ppm): 1,08 (t, 6H), 3,37 (q, 4H), 4,64 (d, 1 H), 5,65 (dd, 1 H), 6,32 (d, 1 H), 6,40 (d, 1 H), 6,63 (d, 2H), 7,15 (d, 1 H), 7,59 (m, 2H), 7,67 (d, 2H), 7,91 (dd, 1 H), 7,96 (d, 1 H); 8,31 (d, 1 H), 8,45 (d, 1 H), 8,81 (s, 2H), 10,56 (s, 1H), 12,73 (s, 1 H)

### Beispiel 5:

### N-[(1S,2R)-2-(2-Amino-benzothiazol-6-ylcarbamoyl)-1-(2-chloro-phenyl)-2-hydroxyethyl]-4-isopropyl-benzamid; Verbindung mit Trifluoressigsäure

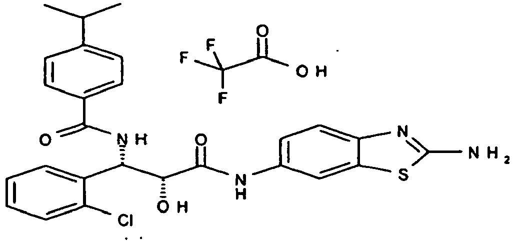

### Verfahrensschritt 1:

### (E)-3-(2-Chlor-phenyl)-acrylsäureisopropylester

In analoger Weise zur Herstellung von Beispiel 4, Verfahrensschritt 1, wurden 1,83 g 2-Chlor-zimtsäure (10 mmol) umgesetzt. Es wurden 1,92 g der Titelverbindung erhalten; Ausbeute: 86 %. LC/MS (Methode D) (M+H)⁺ 225

### Verfahrensschritt 2:

(2R, 3S)-3-Acetylamino-3-(2-chlor-phenyl)-2-hydroxy-propionsäureisopropylester In analoger Weise zur Herstellung von Beispiel 4, Verfahrensschritt 2, wurden 1,9 g der aus Verfahrensschritt 1 erhaltenen Verbindung (8,46 mmol) umgesetzt. Es wurden 1,93 g der Titelverbindung erhalten; Ausbeute: 76 %.
LC/MS (Methode D) (M+H)⁺ 300

### Verfahrensschritt 3:

### (2R, 3S)-3-Amino-3-(2-chlor-phenyl)-2-hydroxy-propionsäure; Verbindung mit Salzsäure

In analoger Weise zur Herstellung von Beispiel 4, Verfahrensschritt 3, wurden 1,93 g der aus Verfahrensschritt 2 erhaltenen Verbindung (6,44 mmol) umgesetzt. Es wurden 1,6 g der Titelverbindung erhalten; Ausbeute: 99 %.
LC/MS (Methode D) (M+H)⁺ 216

### Verfahrensschritt 4:

(2R, 3S)-3-(2-Chlor-phenyl)-2-hydroxy-3-(4-isopropyl-benzoylamino)-propionsäure 525 mg 4-Isopropylbenzoesäure (3,2 mmol) und 525 mg des Verfahrensprodukts aus Schritt 3 (3,2 mmol) wurden in analoger Weise wie in Beispiel 4, Verfahrensschritt 4 umgesetzt. Es wurden 630 mg der Titelverbindung als weißer Feststoff erhalten;
Ausbeute: 54 %. LC/MS (Methode D) (M+H)⁺ 362

### Verfahrensschritt 5:

### N-[(1S,2R)-2-(2-Amino-benzothiazol-6-ylcarbamoyl)-1-(2-chlor-phenyl)-2-hydroxy-ethyl]-4-isopropyl-benzamid; Verbindung mit Trifluoressigsäure

In analoger Weise zur Herstellung von Beispiel 4, Verfahrensschritt 5, wurden 77 mg der aus Verfahrensschritt 4 erhaltenen Verbindung (0,21 mmol) mit 35 mg der aus Beispiel 3, Verfahrensschritt 1, erhaltenen Verbindung (0,21 mmol) umgesetzt. Es wurden 87 mg der Titelverbindung erhalten; Ausbeute: 66 %.
LC/MS (Methode A) 508,13 (Rₜ= 1,40 min, 100%)
¹H NMR (400 MHz, DMSO-d₆) δ (ppm): 1,19 (d, 6H), 2,92 (h, 1H), 4,41 (d, 1H), 5,89 (dd, 1 H), 6,42 (s, 1 H), 7,27-7,34 (m, 5H), 7,45 (m, 2H), 7,56 (dd, 1 H), 7,76 (d, 2H), 8,07 (s, 1 H), 8,10 (d, 1 H), 8,39 (d, 1 H), 9,85 (s, 1 H)

### Beispiel 6:

### N-[(1S,2R)-2-(2-Amino-3H-benzimidazol-5-ylcarbamoyl)-1-(2-fluor-phenyl)-2-hydroxy-ethyl]-4-isopropyl-benzamid; Verbindung mit Trifluoressigsäure

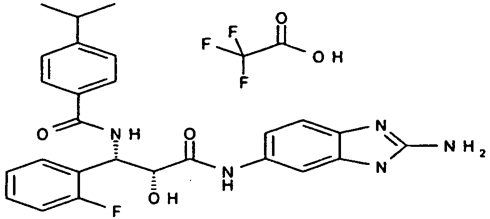

Ethoxycarbonylmethyl-triphenyl-phosphoniumbromid wurde gemäß der Deutschen Patentanmeldung 4238260 und 2,5-Diamino-benzimidazol-1-carbonsäure-tertiär-butylester wurde gemäß der Internationalen Anmeldung WO2002/042273 hergestellt. Verfahrensschritt 1:

### E-3-(2-Fluor-phenyl)-acrylsäureisopropylester

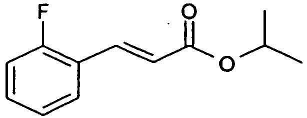

Zu einer gerührten Lösung von 4,4 g Ethoxycarbonylmethyl-triphenyl-phosphonium-bromid (10 mmol) in 20 mL DMF wurde 400 mg Natriumhydrid (60 % in Öl, 10 mmol) zugegeben und bei RT für 10 min weiter gerührt. 1,24 g 2-Fluorbenzaldehyd (10 mmol) wurde zugefügt und für 5 h bei RT gerührt. Das Reaktionsgemisch wurde in 100 mL Ethylacetat aufgenommen und mit einer Kochsalzlösung gewaschen, über Natriumsulfat getrocknet, gefiltert und unter verminderten Druck eingeengt. Die Reinigung erfolgte mit Kieselgelchromatographie (Ethylacetat/Heptan 1:1). Es wurden 1,25 g der Titelverbindung erhalten. Ausbeute: 60%.
LC/MS (Methode D) (M+H)⁺ 209.

### Verfahrensschritt 2:

(1S, $2R)-3-Acetylamino-3-(2-fluor-phenyl)-2-hydroxy-propionsäureisopropylester In analoger Weise zur Herstellung von Beispiel 4, Verfahrensschritt 2, wurden 1,25 g der aus Verfahrensschritt 1 erhaltenen Verbindung (6 mmol) umgesetzt. Es wurden 1,18 g der Titelverbindung erhalten; Ausbeute: 69 %.
LC/MS. (Methode D) (M+H)⁺ 284

### Verfahrensschritt 3:

### (1S, 2R)-3-Amino-3-(2-fluor-phenyl)-2-hydroxy-propionsäure; Verbindung mit Salzsäure

In analoger Weise zur Herstellung von Beispiel 4, Verfahrensschritt 3, wurden 1,18 g der aus Verfahrensschritt 2 erhaltenen Verbindung (4,11 mmol) umgesetzt. Es wurde 1 g der Titelverbindung erhalten; Ausbeute: 99 %.
LC/MS (Methode D) (M+H)⁺ 200

### Verfahrensschritt 4:

(2R, 3S)-3-(2-Fluor-phenyl)-2-hydroxy-3-(4-isopropyl-benzoylamino)-propionsäure 342 mg 4-Isopropylobenzoesäure (2,1 mmol) und 416 mg des Verfahrensprodukts aus Schritt 3 (2,1 mmol) wurden in analoger Weise wie in Beispiel 4, Verfahrensschritt 4 umgesetzt. Es wurden 365 mg der Titelverbindung als weißer Feststoff erhalten;
Ausbeute: 50 %. LC/MS (Methode D) (M+H)⁺ 346

### Verfahrensschritt 5:

(2R, 3S)-2-Amino-5-[3-(2-fluor-phenyl)-2-hydroxy-3-(4-isopropyl-benzoylamino)-propionylamino]- benzimidazol-1-carbonsäure-tertiär-butylester

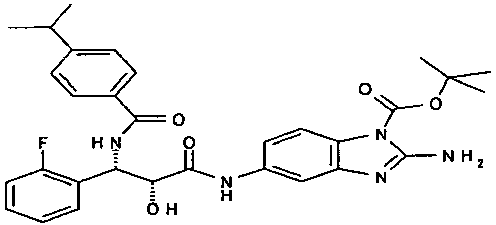

65 mg 2,5-Diamino-benzimidazol-1-carbonsäure-tertiär-butylester und 92 mg der aus Verfahrensschritt 4 erhaltenen Verbindung (0,266 mmol) wurden in 2 mL DMF gelöst.

Anschließend wurden 36 mg HOAt (0,265 mmol), 130 mg PyBrop (0,28 mmol) und 200 µL DIPEA (1,17 mmol) in 1 mL DMF zugefügt. Nach 3 h Rühren bei RT wurde die Mischung mit Ethylacetat verdünnt. Die organische Phase wurde mit einer Kochsalzlösung gewaschen, über Natriumsulfat getrocknet, gefiltert und unter verminderten Druck eingeengt. Das Rohprodukt wurde ohne weitere Reinigung im nächsten Verfahrensschritt eingesetzt. LC/MS (Methode D) (M+H)⁺ 576

### Verfahrensschritt 6:

### N-[(1S,2R)-2-(2-Amino-3H-benzimidazol-5-ylcarbamoyl)-1-(2-fluor-phenyl)-2-hydroxyethyl]-4- isopropyl-benzamid; Verbindung mit Trifluoressigsäure

Der Rückstand aus Verfahrensschritt 5 wurde in einer Mischung aus 2 mL DCM und 2 mL TFA gelöst. Nach Rühren für 2 h bei RT wurde das Lösungsmittel unter verminderten Druck verdampft und der Rückstand wurde mit einer präparativen HPLC gereinigt. Es wurden 65 mg der Titelverbindung als weißer Feststoff erhalten; Ausbeute: 44 %. LC/MS (Methode A) 475,20 (Rₜ= 1,38 min, 100%)
¹H NMR (400 MHz, DMSO-d₆) δ (ppm): 1,19 (d, 6H), 2,92 (hept, 1 H), 4,40 (s br, 1 H), 5,79 (dd, 1 H), 6,38 (s br, 1 H), 7,14-7,38 (m, 8H), 7,52 (dt, 1 H), 7,77 (d, 2H), 7,88 (s, 1 H), 8,40 (s, 1 H), 8,43 (d, 1 H), 10,00 (s, 1 H) 12,40 (s br, 1 H)

### Beispiel 7:

### N-[(1S,2R)-[2-(2-Amino-3H-benzimidazol-5-ylcarbamoyl)-2-hydroxy-1-phenyl-ethyl]-4-tertiär-butyl-benzamid; Verbindung mit Trifluoressigsäure

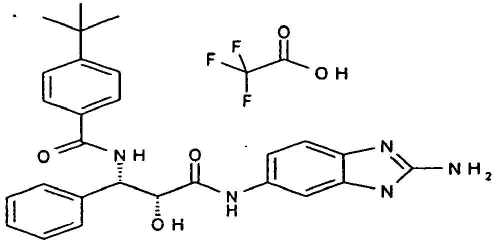

### Verfahrensschritt 1:

### (1S,2R)-2-Amino-6-(3-tert-butoxycarbonylamino-2-hydroxy-3-phenyl-propionylamino)-benzimidazol-1-carbonsäure-tertiär-butylester

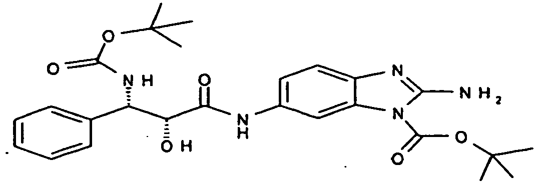

1,5 g (1S,2R)-3-tertiär-Butoxycarbonylamino-2-hydroxy-3-phenyl-propionsäure (5,3 mmol) und 1,32 g 2,5-Diamino-benzimidazol-1-carbonsäure-tertiär-butylester (5,3 mmol) wurden in 30 mL DMF gelöst. Zu dieser Lösung wurden 798 mg HOAt (5,86 mmol), 1,75 g PyBrop (5,86 mmol) und 2,8 mL DIPEA (16 mmol) in 15 mL DCM und 15 mL DMF zugefügt und bei RT für 5 h gerührt. Die organische Phase wurde mit wässriger Natriumhydrogencarbonat Lösung und Kochsalzlösung gewaschen, über Natriumsulfat getrocknet, gefiltert und unter verminderten Druck eingeengt. Nach Kieselgelchromatographie (Ethylacetat/Heptan 2:1) wurden 2 g der Titelverbindung als weißer Feststoff erhalten. Ausbeute: 74 %.
LC/MS (Methode D) (M+H-tBu)⁺ 456

### Verfahrensschritt 2:

### (1 S,2R)-3-Amino-N-(2-amino-3H-benzimidazol-5-yl)-2-hydroxy-3-phenyl-propionamid; Verbindung mit Trifluoressigsäure

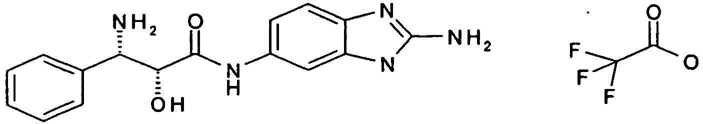

Das Verfahrensprodukt aus Schritt 1 wurde in 10 mL DCM und 10 mL TFA gelöst, 2 h bei RT gerührt und es wurden 2,1 g der Titelverbindung als rosa Feststoff erhalten; Ausbeute 99 %. LC/MS (Methode D) (M+H)⁺ 312

### Verfahrensschritt 3:

### N-[(1S,2R)-[2-(2-Amino-3H-benzimidazol-5-ylcarbamoyl)-2-hydroxy-1-phenyl-ethyl]-4-tertiär-butyl-benzamid; Verbindung mit Trifluoressigsäure

45 mg 4-tertiär-Butylbenzoesäure (0,25 mmol), 91 mg TOTU (0,28 mmol) und 150 µL N-Ethyl-morpholin (1,5 mmol) wurden in 2 mL DMF gelöst und für 30 min bei RT gerührt. 132 mg des Verfahrenprodukts aus Schritt 2 (0,25 mmol) wurden zugefügt und 2 h bei RT gerührt. Die Mischung wurde mit Ethylacetat verdünnt. Die organische Phase wurde mit wässriger Natriumhydrogencarbonat Lösung und Kochsalzlösung gewaschen, über Natriumsulfat getrocknet, gefiltert und unter verminderten Druck eingeengt. Der Rückstand wurde über HPLC gereinigt 36 mg der Titelverbindung wurde als weißer Feststoff erhalten. Ausbeute: 25 %.
LC/MS (Methode A) 472,31 (Rₜ= 1,39 min, 100%)
¹H NMR (400 MHz, DMSO-d₆) δ (ppm): 1,29 (s, 9H), 4,47 (s br, 1 H), 5,50 (dd, 1 H), 6,11 (s br, 1H), 7,20-7,35 (m, 5H), 7,40-7,48 (m, 5H), 7,76 (d, 2H), 7,85 (s, 1 H), 8,39 (s br, 1 H), 8,45 (d, 1H), 8,39 (d, 1 H), 9,99 (s, 1 H).

### Beispiel 8:

### N-[(1S,2R)-2-(4-Aminomethyl-phenylcarbamoyl)-2-hydroxy-1-phenyl-ethyl]-benzamid; Verbindung mit Trifluoressigsäure

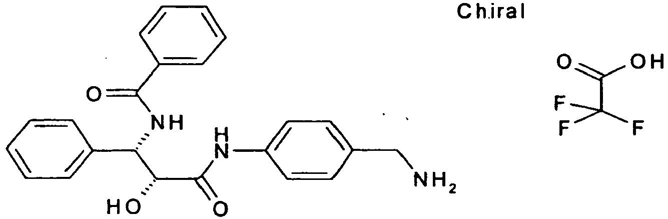

### Verfahrensschritt 1:

### [4-((2R,3S)-3-Benzoylamino-2-hydroxy-3-phenyl-propionylamino)-benzyl]-carbaminsäure tert-butyl ester

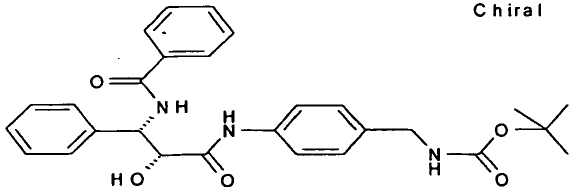

In Analogie zu Beispiel 1, Verfahrensschritt 1, wurden 33 mg (0,15 mmol) (4-Amino-benzyl)-carbaminsäure tert-butyl ester und 43 mg (0,15 mmol) (2R, 3S)-3-Benzoylamino-2-hydroxy-3-phenyl-propionsäure in 1 ml DCM und 1 ml DMF gelöst und nacheinander mit 78 µl DIPEA (0,45 mmol), 22,5 mg HOAt (0,165 mmol) und 76,9 mg (0,165 mmol) PyBrop versetzt. Ohne weitere Aufarbeitung wurde der erhaltene Ansatz filtriert und dann mit HPLC gereinigt. Die produkt-enthaltenden Fraktionen wurden lyophilisiert.

### Verfahrensschritt 2:

### N-[(1S,2R)-2-(4-Aminomethyl-phenylcarbamoyl)-2-hydroxy-1-phenyl-ethyl]-benzamid; Verbindung mit Trifluoressigsäure

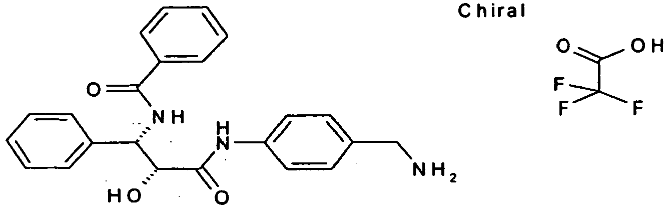

Das Verfahrensprodukt aus Schritt 1 wurde analog zu Beispiel 1; Verfahrensschritt 3, umgesetzt; danach wurde eingeengt, mit Wasser versetzt und lyophilisiert. Ausbeute: 31,5 mg (43 % über 2 Stufen).
LC/MS (Methode A) M-NH₂= 373,40 (Rₜ= 1,09 min, 85%)
¹H NMR (500 MHz, DMSO-d₆) δ (ppm): 3,97 (d, 2H), 4,47 (s br, 1 H), 5,50 (dd, 1 H), 6,11 (dd, 1 H), 7,23 (m, 1 H), 7,30-7,36 (m, 4H), 7,42-7,48 (m, 4H), 7,53 (m, 1 H), 7,62 (d, 2H), 7,84 (d, 2H), 8,05 (s br, 3H), 8,53 (d, 1 H), 9,99 (s, 1 H).

### Beispiel 9:

### N-[(1S,2R)-2-(2,4-Diamino-chinazolin-6-ylcarbamoyl)-2-hydroxy-1-phenyl-ethyl]-benzamid; Verbindung mit Trifluoressigsäure

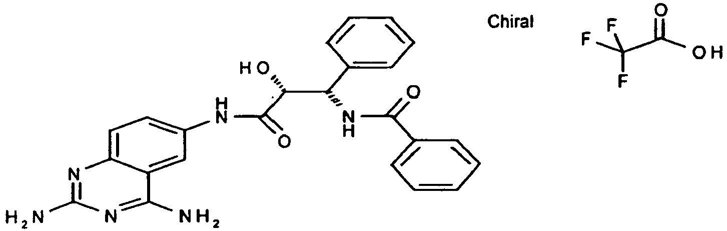

In Analogie zu Beispiel 1, Verfahrensschritt 1, wurden 76 mg (0,44 mmol) Chinazolin-2,4,6-triamin und 150 mg (0,52 mmol) (2R,3S)-3-Benzoylamino-2-hydroxy-3-phenyl-propionsäure in 3 ml DMF gelöst und nacheinander mit 153 µl DIPEA (0,86 mmol), 78,9 mg HOAt (0,57 mmol) und 251 mg (0,53 mmol) PyBrop versetzt. Ohne weitere Aufarbeitung wurde der Ansatz filtriert und mit HPLC gereinigt. Die produkt-enthaltenden Fraktionen wurden lyophilisiert.
Ausbeute: 114 mg (48 %).
LC/MS (Methode A) M +H = 443,32 (Rₜ= 1,10 min, 100%)
¹H NMR (500 MHz, DMSO-d₆) δ (ppm): 4,51 (m, 1 H), 5,54 (m, 1 H), 6,25 (d, 1 H), 7,22-7,51 (m, 9H), 7,75-7,83 (m, 4H), 8,36 (s br, 1 H), 8,53 (d, 1 H), 8,72 (s br, 1 H), 8,93 (s br, 1H), 10,15 (s br, 1H), 12,30 (s br, 1 H).

Die Verbindungen in der nachfolgenden Tabelle 1 wurden in analoger Weise wie in den vorstehenden Beispielen hergestellt.

Die Darstellung der hergestellten Verbindungen erfolgte gemäß den Regeln der Internationalen Union für Reine und Angewandte Chemie (IUPAC). Die absolute Stereochemie wurde nicht angegeben und die Verbindungen wurden jeweils als Salze der Trifluoressigsäure hergestellt.

**Tabelle 1:**

| Beispiel Nr. | Verbindung | Masse aus LC-MS | Rₜ aus LC-MS | LC-MS Meth ode |
|---|---|---|---|---|
| 10 | 4-Amino-N-[2-(2-amino-3H-benzimidazol-5-ylcarbamoyl)-2-hydroxy-1-phenyl-ethyl]-benzamid | 431,23 | 0,81 | A |
| 11 | Chinoxalin-2-carbonsäure [2-(2-amino-3H-benzimidazol-5-ylcarbamoyl)-2-hydroxy-1-phenyl-ethyl]-amid | 468,23 | 1,14 | A |
| 12 | N-[2-(2-Amino-3H-benzimidazol-5-ylcarbamoyl)-2-hydroxy-1-phenyl-ethyl]-4-methoxy-benzamid | 446,23 | 1,08 | A |
| 13 | Thiophen-3-carbonsäure [2-(2-amino-3H-benzimidazol-5-ylcarbamoyl)-2-hydroxy-1-phenyl-ethyl]-amid | 422,17 | 1 | A |
| 14 | 1-Methyl-1H-pyrrol-2-carbonsäure [(1S,2R)-2-(2-amino-3H-benzimidazol-5-ylcarbamoyl)-2-hydroxy-1-phenyl-ethyl]-amid | 419,23 | 1,02 | A |
| 15 | Chinolin-2-carbonsäure [2-(2-amino-3H-benzimidazol-5-ylcarbamoyl)-2-hydroxy-1-phenyl-ethyl]-amid | 467,23 | 1,23 | A |
| 16 | N-[2-(2-Amino-3H-benzimidazol-5-ylcarbamoyl)-2-hydroxy-1-phenyl-ethyl]-4-cyclohexyl-benzamid | 498,23 | 1,5 | A |
| 17 | N-[2-(2-Amino-3H-benzimidazol-5-ylcarbamoyl)-2-hydroxy-1-phenyl-ethyl]-4-isopropyl-benzamid | 458,2 | 1,33 | A |
| 18 | N-[2-(2-Amino-3H-benzimidazol-5-ylcarbamoyl)-2-hydroxy-1-phenyl-ethyl]-4-trifluormethoxy-benzamid | 500,11 | 1,33 | A |
| 19 | N-[2-(2-Amino-3H-benzimidazol-5-ylcarbamoyl)-2-hydroxy-1-phenyl-ethyl]-4-diethylamin-benzamid | 487,28 | 1 | A |
| 20 | N-[2-(2-Amino-3H-benzimidazol-5-ylcarbamoyl)-2-hydroxy-1-phenyl-ethyl]-4-phenoxy-benzamid | 508,24 | 1,4 | A |
| 21 | N-[2-(2-Amino-3H-benzimidazol-5-ylcarbamoyl)-2-hydroxy-1-phenyl-ethyl]-4-benzyl-benzamid | 506,27 | 1,41 | A |
| 22 | N-[2-(2-Amino-3H-benzimidazol-5-ylcarbamoyl)-2-hydroxy-1-phenyl-ethyl]-4-isopropoxy-benzamid | 474,27 | 1,47 | B |
| 23 | N-[2-(2-Amino-3H-benzimidazol-5-ylcarbamoyl)-2-hydroxy-1-phenyl-ethyl]-4-methylsulfanyl-benzamid | 462,22 | 1,37 | B |
| 24 | N-[2-(2-Amino-3H-benzimidazol-5-ylcarbamoyl)-2-hydroxy-1-phenyl-ethyl]-4-ethylsulfanyl-benzamid | 476,23 | 1,44 | B |
| 25 | N-[2-(2-Amino-3H-benzimidazol-5-ylcarbamoyl)-2-hydroxy-1-phenyl-ethyl]-4-[(4,6-dimethyl-pyrimidin-2-yl)-methyl-amin]-benzamid | 551,32 | 1,18 | B |
| 26 | N-[2-(2-Amino-3H-benzimidazol-5-ylcarbamoyl)-2-hydroxy-1-phenyl-ethyl]-4-pyridin-3-yl-benzamid | 493,26 | 0,95 | B |
| 27 | 4-(2-Acetylamino-3,3,3-trifluor-propyl)-N-[2-(2-amino-3H-benzimidazol-5-ylcarbamoyl)-2-hydroxy-1-phenyl-ethyl]-benzamid | 569,29 | 1,32 | B |
| 28 | 1-Methyl-1H-indol-2-carbonsäure [2-(2-amino-3H-benzimidazol-5-ylcarbamoyl)-2-hydroxy-1-phenyl-ethyl]-amid | 469,26 | 1,45 | B |
| 29 | 5-Methoxy-1H-indol-2-carbonsäure [2-(2-amino-3H-benzimidazol-5-ylcarbamoyl)-2-hydroxy-1-phenyl-ethyl]-amid | 485,26 | 1,36 | B |
| 30 | 5-Ethyl-1 H-indol-2-carbonsäure [2-(2-amino-3H-benzimidazol-5-ylcarbamoyl)-2-hydroxy-1-phenyl-ethyl]-amid | 483,28 | 1,58 | B |
| 31 | 6-Methoxy-1H-indol-2-carbonsäure [2-(2-amino-3H-benzimidazol-5-ylcarbamoyl)-2-hydroxy-1-phenyl-ethyl]-amid | 485,26 | 1,39 | B |
| 32 | 4-Methoxy-chinolin-2-carbonsäure [2-(2-amino-3H-benzimidazol-5-ylcarbamoyl)-2-hydroxy-1-phenyl-ethyl]-amid | 497,25 | 1,5 | B |
| 33 | 3-Ethoxy-chinoxalin-2-carbonsäure [2-(2-amino-3H-benzimidazol-5-ylcarbamoyl)-2-hydroxy-1-phenyl-ethyl]-amid | 512,27 | 1,45 | B |
| 34 | Naphthalen-2-carbonsäure [2-(2-amino-3H-benzimidazol-5-ylcarbamoyl)-2-hydroxy-1-phenyl-ethyl]-amid | 466,24 | 1,29 | A |
| 35 | N-[2-(2-Amino-3H-benzimidazol-5-ylcarbamoyl)-2-hydroxy-1-phenyl-ethyl]-4-pyrrol-1-yl-benzamid | 481,26 | 1,3 | A |
| 36 | N-[2-(2-Amino-3H-benzimidazol-5-ylcarbamoyl)-2-hydroxy-1-phenyl-ethyl]-4-trifluormethylsulfanyl-benzamid | 516,17 | 1,39 | A |
| 37 | Biphenyl-4-carbonsäure [2-(2-amino-3H-benzimidazol-5-ylcarbamoyl)-2-hydroxy-1-phenyl-ethyl]-amid | 492,28 | 1,53 | B |
| 38 | N-[2-(2-Amino-3H-benzimidazol-5-ylcarbamoyl)-2-hydroxy-1-phenyl-ethyl]-4-(2,2,2-trifluor-ethoxy)-benzamid | 514,25 | 1,44 | B |
| 39 | [2-(2-Amino-3H-benzimidazol-5-ylcarbamoyl)-2-hydroxy-1-phenyl-ethyl]-carbaminsäure benzylester | 446,25 | 1,27 | A |
| 40 | N-[2-(2-Amino-3H-benzimidazol-5-ylcarbamoyl)-2-hydroxy-1-phenyl-ethyl]-4-ethyl-benzamid | 444,23 | 1,4 | B |
| 41 | N-[2-(2-Amino-3H-benzimidazol-5-ylcarbamoyl)-2-hydroxy-1-phenyl-ethyl]-4-ethoxy-benzamid | 460,22 | 1,31 | B |
| 42 | 5-Butyl-pyridine-2-carbonsäure [2-(2-amino-3H-benzimidazol-5-ylcarbamoyl)-2-hydroxy-1-phenyl-ethyl]-amid | 473,24 | 1,62 | B |
| 43 | Benz[b]thiophen-2-carbonsäure [2-(2-amino-3H-benzimidazol-5-ylcarbamoyl)-2-hydroxy-1-phenyl-ethyl]-amid | 472,17 | 1,42 | B |
| 44 | 6-Methoxy-naphthalen-2-carbonsäure [2-(2-amino-3H-benzimidazol-5-ylcarbamoyl)-2-hydroxy-1-phenyl-ethyl]-amid | 496,22 | 1,45 | B |
| 45 | 4-(2,2,2-Trifluor-ethyl)-4H-thien[3,2-b]pyrrol-5-carbonsäure [2-(2-amino-3H-benzimidazol-5-ylcarbamoyl)-2-hydroxy-1-phenyl-ethyl]-amid | 543,17 | 1,54 | B |
| 46 | Biphenyl-3,4'-dicarbonsäure 4'-{[2-(2-amino-3H-benzimidazol-5-ylcarbamoyl)-2-hydroxy-1-phenyl-ethyl]-amid} 3-methylamid | 549,26 | 1,28 | B |
| 47 | N-[2-(1-Amino-isochinolin-7-ylcarbamoyl)-2-hydroxy-1-phenyl-ethyl]-4-diethylamin-benzamid | 497,24 | 1,09 | A |
| 48 | N-[2-(1-Amino-isochinolin-7-ylcarbamoyl)-2-hydroxy-1-phenyl-ethyl]-4-isopropyl-benzamid | 468,22 | 1,37 | A |
| 49 | N-[2-(1-Amino-isochinolin-7-ylcarbamoyl)-2-hydroxy-1-phenyl-ethyl]-6-pyrrolidin-1-yl-nicotinamid | 496,22 | 1,03 | A |
| 50 | N-[2-(1-Amino-isochinolin-7-ylcarbamoyl)-2-hydroxy-1-phenyl-ethyl]-4-morpholin-4-yl-benzamid | 511,22 | 1,16 | A |
| 51 | N-[2-(1-Amino-isochinolin-7-ylcarbamoyl)-2-hydroxy-1-phenyl-ethyl]-4-pyrrol-1-yl-benzamid | 491,2 | 1,37 | A |
| 52 | 1-Ethyl-2,3-dimethyl-1H-indol-5-carbonsäure [2-(2-amin-3H-benzimidazol-5-ylcarbamoyl)-2-hydroxy-1-phenyl-ethyl]-amid | 511,35 | 1,4 | A |
| 53 | N-[2-(2-Amin-3H-benzimidazol-5-ylcarbamoyl)-2-hydroxy-1-phenyl-ethyl]-4-morpholin-4-yl-benzamid | 501,33 | 1,2 | A |
| 54 | N-[2-(1-Amino-isochinolin-7-ylcarbamoyl)-2-hydroxy-1-phenyl-ethyl]-4-pyrrolidin-1-yl-benzamid | 495,23 | 1,34 | A |
| 55 | N-[2-(1-Amino-isochinolin-7-ylcarbamoyl)-2-hydroxy-1-phenyl-ethyl]-6-morpholin-4-yl-nicotinamid | 513,22 | 0,97 | A |
| 56 | 5,6-Dimethoxy-1H-indol-2-carbonsäure [2-(2-amin-3H-benzimidazol-5-ylcarbamoyl)-2-hydroxy-1-phenyl-ethyl]-amid | 515,34 | 1,16 | A |
| 57 | N-[2-(2-Amino-3H-benzimidazol-5-ylcarbamoyl)-2-hydroxy-1-phenyl-ethyl]-4-pyrrolidin-1-yl-benzamid | 485,11 | 1,37 | A |
| 58 | N-[2-(2-Amino-3H-benzimidazol-5-ylcarbamoyl)-2-hydroxy-1-phenyl-ethyl]-4-isobutyl-benzamid | 472,3 | 1,65 | B |
| 59 | N-[2-(2-Amino-3H-benzimidazol-5-ylcarbamoyl)-2-hydroxy-1-phenyl-ethyl]-4-butyl-benzamid | 472,3 | 1,67 | B |
| 60 | N-[2-(2-Amino-3H-benzimidazol-5-ylcarbamoyl)-2-hydroxy-1-phenyl-ethyl]-4-propyl-benzamid | 458,28 | 1,36 | A |
| 61 | 4H-Thieno[3,2-b]pyrrol-5-carbonsäure [2-(2-amin-3H-benzimidazol-5-ylcarbamoyl)-2-hydroxy-1-phenyl-ethyl]-amid | 461,2 | 1,3 | B |
| 62 | N-[2-(4-Carbamimidoyl-phenylcarbamoyl)-2-hydroxy-1-phenyl-ethyl]-4-diethylamin-benzamid | 473,24 | 0,98 | A |
| 63 | 5-Methoxy-1H-indol-2-carbonsäure[2-(1-amino-isochinolin-7-ylcarbamoyl)-2-hydroxy-1-phenyl-ethyl]-amid | 495,19 | 1,32 | A |
| 64 | 5-Ethyl-thiophene-2-carbonsäure [2-(1-amino-isochinolin-7-ylcarbamoyl)-2-hydroxy-1-phenyl-ethyl]-amid | 460,16 | 1,31 | A |
| 65 | 5-tert-Butyl-thiophen-2-carbonsäure [2-(1-amino-isochinolin-7-ylcarbamoyl)-2-hydroxy-1-phenyl-ethyl]-amid | 488,19 | 1,45 | A |
| 66 | N-[2-(1-Amino-isochinolin-6-ylcarbamoyl)-2-hydroxy-1-phenyl-ethyl]-benzamid | 426,17 | 1,15 | A |
| 67 | 6-Brom-benzofuran-2-carbonsäure [2-(2-amin-3H-benzimidazol-5-ylcarbamoyl)-2-hydroxy-1-phenyl-ethyl]-amid | 534,15 | 1,37 | A |
| 68 | 5-Ethyl-thiophen-2-carbonsäure [2-(2-amino-3H-benzimidazol-5-ylcarbamoyl)-2-hydroxy-1-phenyl-ethyl]-amid | 450,16 | 1,25 | A |
| 69 | Thieno[3,2-b]thiophen-2-carbonsäure [2-(2-amino-3H-benzimidazol-5-ylcarbamoyl)-2-hydroxy-1-phenyl-ethyl]-amid | 478,15 | 1,3 | A |
| 70 | 5-Methoxy-benzofuran-2-carbonsäure [2-(2-amin-3H-benzimidazol-5-ylcarbamoyl)-2-hydroxy-1-phenyl-ethyl]-amid | 486,2 | 1,26 | A |
| 71 | Benzo[b]thiophen-3-carbonsäure [2-(2-amino-3H-benzimidazol-5-ylcarbamoyl)-2-hydroxy-1-phenyl-ethyl]-amid | 472,17 | 1,26 | A |
| 72 | 4-Methyl-thiophen-2-carbonsäure [2-(2-amino-3H-benzimidazol-5-ylcarbamoyl)-2-hydroxy-1-phenyl-ethyl]-amid | 436,14 | 1,18 | A |
| 73 | N-[2-(2-Amin-benzothiazol-6-ylcarbamoyl)-2-hydroxy-1-phenyl-ethyl]-4-diethylamin-benzamid; Verbindung mit Trifluoressigsäure | 503,2 | 1,42 | A |
| 74 | 1 H-Indol-2-carbonsäure [2-(2-amino-3H-benzimidazol-5-ylcarbamoyl)-2-hydroxy-1-phenyl-ethyl]-amid | 455,22 | 1,21 | A |
| 75 | N-[2-(2-Amino-3H-benzimidazol-5-ylcarbamoyl)-2-hydroxy-1-phenyl-ethyl]-4-dimethylamin-benzamid; Verbindung mit Trifluoressigsäure | 459,25 | 1,06 | A |
| 76 | 5-tert-Butyl-thiophen-2-carbonsäure [2-(2-amino-3H-benzimidazol-5-ylcarbamoyl)-2-hydroxy-1-phenyl-ethyl]-amid | 478,2 | 1,38 | A |
| 77 | N-[2-(2-Amino-3H-benzimidazol-5-ylcarbamoyl)-2-hydroxy-1-phenyl-ethyl]-4-methoxy-3-methyl-benzamid | 460,19 | 1,2 | A |
| 78 | N-[2-(2-Amino-3H-benzimidazol-5-ylcarbamoyl)-2-hydroxy-1-phenyl-ethyl]-3-fluor-4-methyl-benzamid | 448,17 | 1,22 | A |
| 79 | 5-Isopropyl-thiophen-3-carbonsäure [2-(2-amin-3H-benzimidazol-5-ylcarbamoyl)-2-hydroxy-1-phenyl-ethyl]-amid | 464,15 | 1,31 | A |
| 80 | 5-Ethyl-thiophen-3-carbonsäure [2-(2-amino-3H-benzimidazol-5-ylcarbamoyl)-2- hydroxy-1-phenyl-ethyl]-amid | 450,12 | 1,24 | A |
| 81 | [2,2']Bithiophenyl-5-carbonsäure [2-(2-amino-3H-benzimidazol-5-ylcarbamoyl)-2-hydroxy-1-phenyl-ethyl]-amid | 504,14 | 1,35 | A |
| 82 | 5-Methoxy-1H-indol-2-carbonsäure [2-(1-amino-isochinolin-6-ylcarbamoyl)-2-hydroxy-1-phenyl-ethyl]-amid; Verbindung mit Trifluoressigsäure | 495,19 | 1,26 | A |
| 83 | N-[2-(1-Amino-isochinolin-6-ylcarbamoyl)-2-hydroxy-1-phenyl-ethyl]-4-methoxy-benzamid | 456,18 | 1,19 | A |
| 84 | N-[2-(1-Amino-isochinolin-6-ylcarbamoyl)-2-hydroxy-1-phenyl-ethyl]-4-isopropyl-benzamid | 468,22 | 1,38 | A |
| 85 | N-[2-(1-Amino-isochinolin-6-ylcarbamoyl)-2-hydroxy-1-phenyl-ethyl]-4-diethylamin-benzamid | 497,24 | 1,05 | A |
| 86 | N-[2-(1-Amino-isochinolin-6-ylcarbamoyl)-2-hydroxy-1-phenyl-ethyl]-4-ethoxy-benzamid | 470,2 | 1,32 | A |
| 87 | N-[2-(1-Amino-isochinolin-6-ylcarbamoyl)-2-hydroxy-1-phenyl-ethyl]-4-ethyl-benzamid | 454,2 | 1,37 | A |
| 88 | N-[2-(1-Amino-isochinolin-6-ylcarbamoyl)-2-hydroxy-1-phenyl-ethyl]-4-tert-butyl-benzamid | 482,23 | 1,43 | A |
| 89 | N-[2-(1-Amino-isochinolin-6-ylcarbamoyl)-2-hydroxy-1-phenyl-ethyl]-3-dimethylamin-benzamid | 469,21 | 0,97 | A |
| 90 | N-[2-(1-Amino-isochinolin-6-ylcarbamoyl)-2-hydroxy-1-phenyl-ethyl]-4-dimethylamin-benzamid | 469,21 | 1,13 | A |
| 91 | 5-Ethyl-thiophen-2-carbonsäure [2-(1-amino-isochinolin-6-ylcarbamoyl)-2-hydroxy-1-phenyl-ethyl]-amid | 460,16 | 1,32 | A |
| 92 | 5-Ethyl-thiophen-3-carbonsäure [2-(1-amino-isochinolin-6-ylcarbamoyl)-2-hydroxy-1-phenyl-ethyl]-amid | 460,16 | 1,31 | A |
| 93 | 5-tert-Butyl-thiophen-2-carbonsäure [2-(1-amino-isochinolin-6-ylcarbamoyl)-2-hydroxy-1-phenyl-ethyl]-amid | 488,19 | 1,43 | A |
| 94 | N-[2-(1-Amino-isochinolin-6-ylcarbamoyl)-2-hydroxy-1-phenyl-ethyl]-3-ethyl-benzamid | 454,2 | 1,36 | A |
| 95 | N-[2-(1-Amino-isochinolin-6-ylcarbamoyl)-2-hydroxy-1-phenyl-ethyl]-3-ethoxy-benzamid | 470,2 | 1,33 | A |
| 96 | N-[2-(2-Amino-1H-benzimidazol-5-ylcarbamoyl)-2-hydroxy-1-(3-trifluormethyl-phenyl)-ethyl]-4-isopropyl-benzamid | 526,19 | 1,53 | A |
| 97 | N-[2-(2-Amino-1H-benzimidazol-5-ylcarbamoyl)-2-hydroxy-1-(2-methoxy-phenyl)-ethyl]-4-isopropyl-benzamid | 488,35 | 1,4 | A |
| 98 | N-[2-(2-Amino-1H-benzimidazol-5-ylcarbamoyl)-2-hydroxy-1-(3-trifluormethyl-phenyl)-ethyl]-4-diethylamin-benzamid | 555,39 | 1,23 | A |
| 99 | N-[2-(2-Amino-1H-benzimidazol-5-ylcarbamoyl)-2-hydroxy-1-(2-methoxy-phenyl)-ethyl]-4-diethylamin-benzamid | 517,41 | 1,1 | A |
| 100 | N-[2-(2-Amino-1H-benzimidazol-5-ylcarbamoyl)-2-hydroxy-1-(4-methoxy-phenyl)-ethyl]-4-ethylamin-benzamid | 517,42 | 1,03 | A |
| 101 | N-[2-(1-Amino-isochinolin-6-ylcarbamoyl)-1-(3-ethoxy-phenyl)-2-hydroxy-ethyl]-4-diethylamin-benzamid | 541,27 | 1,28 | B |
| 102 | N-[2-(2-Amino-3H-benzimidazol-5-ylcarbamoyl)-2-hydroxy-1-(4-isopropyl-phenyl)-ethyl]-4-diethylamin-benzamid | 529,24 | 1,28 | A |
| 103 | N-[2-(2-Amino-3H-benzimidazol-5-ylcarbamoyl)-1-(3-ethoxy-phenyl)-2-hydroxy-ethyl]-4-isopropyl-benzamid | 502,16 | 1,46 | A |
| 104 | N-[2-(2-Amino-3H-benzimidazol-5-ylcarbamoyl)-1-(3-ethoxy-phenyl)-2-hydroxy-ethyl]-4-diethylamin-benzamid | 531,24 | 1,16 | A |
| 105 | N-[2-(1-Amino-isochinolin-6-ylcarbamoyl)-2-hydroxy-1-m-tolyl-ethyl]-4-diethylamin-benzamid | 511,26 | 1,15 | A |
| 106 | N-[2-(2-Amino-3H-benzimidazol-5-ylcarbamoyl)-2-hydroxy-1-(4-isopropyl-phenyl)-ethyl]-4-isopropyl-benzamid | 500,19 | 1,55 | A |
| 107 | N-[2-(2-Amino-benzothiazol-6-ylcarbamoyl)-2-hydroxy-1-(4-trifluormethyl-phenyl)-ethyl]-4-isopropyl-benzamid | 542,16 | 1,53 | A |
| 108 | N-[2-(2-Amino-benzothiazol-6-ylcarbamoyl)-2-hydroxy-1-m-tolyl-ethyl]-4-diethylamin-benzamid | 517,21 | 1,14 | A |
| 109 | N-[2-(2-Amino-benzothiazol-6-ylcarbamoyl)-2-hydroxy-1-m-tolyl-ethyl]-4-isopropyl-benzamid | 488,19 | 1,42 | A |
| 110 | N-[2-(2-Amino-benzothiazol-6-ylcarbamoyl)-2-hydroxy-1-(4-isopropyl-phenyl)-ethyl]-4-diethylamin-benzamid | 545,25 | 1,27 | A |
| 111 | N-[2-(2-Amino-benzothiazol-6-ylcarbamoyl)-1-(3-ethoxy-phenyl)-2-hydroxy-ethyl]-4-diethylamin-benzamid | 547,23 | 1,13 | A |
| 112 | N-[2-(2-Amino-benzothiazol-6-ylcarbamoyl)-2-hydroxy-1-(4-trifluormethyl-phenyl)-ethyl]- 4-diethylamin-benzamid | 571,19 | 1,27 | A |
| 113 | N-[2-(2-Amino-3H-benzimidazol-5-ylcarbamoyl)-1-(2-chlor-phenyl)-2-hydroxy-ethyl]-4-diethylamin-benzamid | 521,19 | 1,06 | A |
| 114 | N-[2-(2-Amino-3H-benzimidazol-5-ylcarbamoyl)-2-hydroxy-1-(4-trifluormethyl-phenyl)-ethyl]-4-isopropyl-benzamid | 526,14 | 1,5 | A |
| 115 | N-[2-(2-Amino-3H-benzimidazol-5-ylcarbamoyl)-2-hydroxy-1-(4-trifluormethyl-phenyl)-ethyl]-4-diethylamin-benzamid | 555,24 | 1,29 | A |
| 116 | N-[2-(2-Amino-benzothiazol-6-ylcarbamoyl)-1-(3-ethoxy-phenyl)-2-hydroxy-ethyl]-4-isopropyl-benzamid | 518,2 | 1,46 | A |
| 117 | N-[2-(2-Amino-benzothiazol-6-ylcarbamoyl)-1-(2-chlor-phenyl)-2-hydroxy-ethyl]-4-diethylamin-benzamid | M+1= 538/540 | 0,93 | D |
| 118 | N-[2-(1-Amino-isochinolin-6-ylcarbamoyl)-1-(3-brom-phenyl)-2-hydroxy-ethyl]-4-diethylamin-benzamid | 575,15 | 1,35 | B |
| 119 | N-[2-(1-Amino-isochinolin-6-ylcarbamoyl)-1-(3-brom-phenyl)-2-hydroxy-ethyl]-4-ethyl-benzamid | 532,11 | 1,63 | B |
| 120 | N-[2-(1-Amino-isochinolin-6-ylcarbamoyl)-2-hydroxy-1-(4-isopropyl-phenyl)-ethyl]-4-isopropyl-benzamid | 510,26 | 1,58 | A |
| 121 | N-[2-(1-Amino-isochinolin-6-ylcarbamoyl)-1-(2-chlor-phenyl)-2-hydroxy-ethyl]-4-isopropyl-benzamid | 502,18 | 1,44 | B |
| 122 | N-[2-(2-Amino-3H-benzimidazol-5-ylcarbamoyl)-2-hydroxy-1-m-tolyl-ethyl]-4-isopropyl-benzamid | 472,18 | 1,42 | A |
| 123 | N-[2-(2-Amino-3H-benzimidazol-5-ylcarbamoyl)-2-hydroxy-1-m-tolyl-ethyl]-4-diethylamin-benzamid | 501,23 | 1,09 | A |
| 124 | N-[2-(1-Amino-isochinolin-6-ylcarbamoyl)-1-(4-brom-phenyl)-2-hydroxy-ethyl]-4-diethylamin-benzamid | 575,15 | 1,37 | B |
| 125 | N-[2-(1-Amino-isochinolin-6-ylcarbamoyl)-1-(4-brom-phenyl)-2-hydroxy-ethyl]-4-isopropyl-benzamid | M + 1 = 547/54 8 | 1,24 | D |
| 126 | N-[2-(1-Amino-isochinolin-6-ylcarbamoyl)-1-(3-brom-phenyl)-2-hydroxy-ethyl]-4-isopropyl-benzamid | 546,13 | 1,66 | C |
| 127 | N-[2-(1-Amino-isochinolin-6-ylcarbamoyl)-2-hydroxy-1-m-tolyl-ethyl]-4-isopropyl-benzamid | 482,23 | 1,42 | A |
| 128 | N-[2-(1-Amino-isochinolin-6-ylcarbamoyl)-1-(3-ethoxy-phenyl)-2-hydroxy-ethyl]-4-isopropyl-benzamid | 512,24 | 1,43 | A |
| 129 | N-[2-(1-Amino-isochinolin-6-ylcarbamoyl)-2-hydroxy-1-(4-isopropyl-phenyl)-ethyl]-4-diethylamin-benzamid | 539,29 | 1,32 | A |
| 130 | N-[2-(1-Amino-isochinolin-6-ylcarbamoyl)-1-(2-chlor-phenyl)-2-hydroxy-ethyl]-4-diethylamin-benzamid | 531,2 | 1,16 | A |
| 131 | N-[2-(1-Amino-isochinolin-6-ylcarbamoyl)-2-hydroxy-1-(4-trifluormethyl-phenyl)-ethyl]-4-diethylamin-benzamid | 565,23 | 1,26 | A |
| 132 | N-[2-(1-Amino-isochinolin-6-ylcarbamoyl)-2-hydroxy-1-(4-trifluormethyl-phenyl)-ethyl]-4-isopropyl-benzamid | 536,2 | 1,83 | B |
| 133 | N-[2-(1-Amino-isochinolin-6-ylcarbamoyl)-1-(4-brom-phenyl)-2-hydroxy-ethyl]-4-ethyl-benzamid | 532,11 | 1,62 | C |
| 134 | N-[2-(2-Amino-3H-benzimidazol-5-ylcarbamoyl)-1-(3,5-dimethoxy-phenyl)-2-hydroxy-ethyl]-4-isopropyl-benzamid | 518,22 | 1,43 | A |
| 135 | N-[2-(2-Amino-3H-benzimidazol-5-ylcarbamoyl)-1-(3,5-dimethoxy-phenyl)-2-hydroxy-ethyl]-4-diethylamin-benzamid | 547,26 | 1,05 | A |
| 136 | N-[2-(1-Amino-isochinolin-6-ylcarbamoyl)-2-hydroxy-1-phenyl-ethyl]-4-propyl-benzamid | 468,22 | 1,36 | A |
| 137 | N-[2-(2-Amino-3H-benzimidazol-5-ylcarbamoyl)-1-(2-chloro-phenyl)-2-hydroxy-ethyl]-4-isopropyl-benzamid | 491,17 | 1,35 | A |
| 138 | N-[2-(1-Amino-isochinolin-6-ylcarbamoyl)-2-hydroxy-1-phenyl-ethyl]-4-cyclopropyl-benzamid | 466,2 | 1,45 | B |
| 139 | N-[2-(1-Amino-isochinolin-6-ylcarbamoyl)-1-(2-chlor-phenyl)-2-hydroxy-ethyl]-4-ethyl-benzamid | 488,16 | 1,53 | B |
| 140 | N-[2-(2-Amino-benzothiazol-6-ylcarbamoyl)-1-furan-2-yl-2-hydroxy-ethyl]-4-diethylamin-benzamid | 493,18 | 1,03 | B |
| 141 | N-[2-(2-Amino-3H-benzimidazol-5-ylcarbamoyl)-1-(2-fluor-phenyl)-2-hydroxy-ethyl]-4-diethylamin-benzamid | 504,23 | 1,04 | A |
| 142 | N-[2-(2-Amino-3H-benzimidazol-5-ylcarbamoyl)-1-(3,5-difluor-phenyl)-2-hydroxy-ethyl]-4-isopropyl-benzamid | 493,19 | 1,58 | B |
| 143 | N-[2-(2-Amino-3H-benzimidazol-5-ylcarbamoyl)-1-(3,5-difluor-phenyl)-2-hydroxy-ethyl]-4-diethylamin-benzamid | 522,22 | 1,21 | B |
| 144 | N-[2-(2-Amino-3H-benzimidazol-5-ylcarbamoyl)-1-(5-fluor-2-methansulfonyl-phenyl)-2-hydroxy-ethyl]-4-diethylamin-benzamid | 582,21 | 1,36 | A |
| 145 | N-[2-(2-Amino-3H-benzimidazol-5-ylcarbamoyl)-1-(5-fluor-2-methansulfonyl-phenyl)-2-hydroxy-ethyl]-4-diethylamin-benzamid | 553,18 | 1,54 | A |
| 146 | N-[2-(2-Amino-3H-benzimidazol-5-ylcarbamoyl)-1-(3-chloro-phenyl)-2-hydroxy-ethyl]-4-diethylamin-benzamid | 491,17 | 1,6 | A |
| 147 | N-[2-(2-Amino-3H-benzimidazol-5-ylcarbamoyl)-1-(3-chlor-phenyl)-2-hydroxy-ethyl]-4-diethylamin-benzamid | 520,2 | 1,43 | A |
| 148 | N-[2-(2-Amino-3H-benzimidazol-5-ylcarbamoyl)-2-hydroxy-1-(2-trifluormethoxy-phenyl)- ethyl]-4-isopropyl-benzamid | 541,19 | 1,6 | A |
| 149 | N-[2-(2-Amino-3H-benzimidazol-5-ylcarbamoyl)-2-hydroxy-1-(2-trifluormethoxy-phenyl)-ethyl]-4-diethylamin-benzamid | 570,22 | 1,47 | A |
| 150 | N-[2-(2-Amino-3H-benzimidazol-5-ylcarbamoyl)-2-hydroxy-1-o-tolyl-ethyl]-4-diethylamin-benzamid | M + 1 = 512 | 0,93 | D |
| 151 | N-[2-(2-Amino-3H-benzimidazol-5-ylcarbamoyl)-2-hydroxy-1-thiophen-2-yl-ethyl]-4-diethylamin-benzamid | 509,16 | 1,08 | A |
| 152 | [(1S,2R)-2-(2-Amino-3H-benzimidazol-5-ylcarbamoyl)-2-hydroxy-1-phenyl-ethyl] carbaminsäure 9H-fluoren-9-ylmethyl ester | 534,41 | 1,47 | A |
| 153 | [2-(2-Amino-3H-benzimidazol-5-ylcarbamoyl)-2-hydroxy-1-phenyl-ethyl]-carbaminsäure 2-chlor-benzyl ester | 480,19 | 1,26 | A |

### Pharmakologische Beispiele

### Faktor IXa Bestimmungsmethode

Die hergestellten Substanzen aus den Beispielen wurden mit dem Substrat PEFA 3107 (Firma Pentapharm/Loxo; über S. Black GmbH, Baumstrasse 41, 47198 Duisburg, Deutschland; Pr. Nr. 095-20) und Faktor IXa (Firma Calbiochem, Merck KGaA vertreibt Calbiochem in Deutschland, Life Science & Analytics, 64293 Darmstadt; Pr. Nr. 233290) auf Inhibition der enzymatischen Aktivität von FIXa geprüft. Dabei wurden zu 2 µl 10 mM Dimethylsulfoxid-Lösung der jeweiligen Testsubstanz, 28 µL Testpuffer (50 mM α,α,α-Tris-(hydroxymethyl)-methylamin (TRIS), 100 mM NaCl, 5 mM CaCl₂, 0,1 % Rinderserumalbumin, pH 7,4) und 10 µL Faktor IXa (277 nM Endkonzentration im Testansatz) gegeben und 15 Minuten bei RT in einer 96 half-well Mikrotiterplatte inkubiert. Die Enzymreaktion wurde durch Zugabe von 10 µL Substrat (1 mM Stocklösung in Wasser) gestartet. Der Zeitverlauf der Reaktion wurde bei 405 nm in einem Mikrotiterplattenreader (SpectraMax plus 384; Firma Molecular Devices) über 15 Minuten verfolgt.

Die IC₅₀ wurde aus den gemittelten Werten (Doppelbestimmung) einer Verdünnungsreihe der Testsubstanz mit Hilfe der Software Grafit 4 (Erithacus Software, UK) berechnet. Die Inhibitions-konstanten (Kᵢ) wurden gemäß der Cheng Prusoff Gleichung Ki = IC₅₀/(1+(S/Km) berechnet, wobei S = Konzentration des Testsubstrats im Test und Kₘ = Michaelis-Menten Konstante bedeutet.

Tabelle 2 zeigt die Ergebnisse.

**Tabelle 2:**

| Verbindung aus Beispiel | Faktor IXa-Enzym-Assay IC₅₀ [mikro M] | Verbindung aus Beispiel | Faktor IXa-Enzym-Assay IC₅₀ [mikro M] |
|---|---|---|---|
| 17 | 0,28 | 81 | 0,57 |
| 53 | 1,34 | 87 | 0,14 |
| 68 | 0,73 | 134 | 0,17 |

## Patentansprüche

1. Verbindung der Formel I 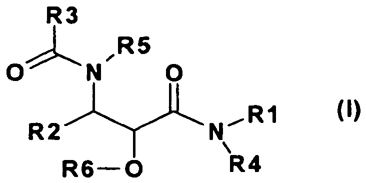 und/oder alle stereoisomeren Formen der Verbindung der Formel I und/oder Gemische dieser Formen in jedem Verhältnis, und/oder ein physiologisch verträgliches Salz der Verbindung der Formel I, wobei
R1 für
1) -(C₆-C₁₄)-Aryl-Z, wobei Aryl ausgewählt ist aus der Gruppe Phenyl und Naphthyl und worin Aryl unsubstituiert oder ein-, zwei- oder dreifach durch T substituiert ist und Z Amino, Amidino, Aminomethylen, Aminopyridinyl, Azetidinyl, Guanidino, Piperidinyl, Pyridinyl oder Pyrrolidinyl bedeutet, oder
2) ein vier- bis fünfzehngliedrigen Het-Z, wobei Het ausgewählt ist aus der Gruppe Acridinyl, Azepinyl, Azetidinyl, Benzimidazolinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, beta-Carbolinyl, Chinazolinyl, Chinolinyl, Chinolizinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, Dioxolenyl, 2H, 6H-1,5,2-Dithiazinyl, Furanyl, Furazanyl, Imidazolidinyl, Imidazolinyl, Imidazolyl, 1 H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Phenanthridinyl, Phenanthrenyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazinyl, Thiazolyl, Thienyl, Thienoimidazolyl, Thienooxazolyl, Thienopyridinyl, Thienopyrrolyl, Thienothiazolyl, Thienothiophenyl, Thiomorpholinyl, Thiopyranyl, Triazinyl, 1,2,3-Triazolyl, 1,2,4-Triazolyl, 1,2,5-Triazolyl, 1,3,4-Triazolyl oder Xanthenyl und worin Het unsubstituiert oder ein-, zwei- oder dreifach durch T substituiert ist und worin Z wie oben definiert ist, steht,
R2 für -(C₀-C₄)-Alkylen-(C₆-C₁₄)-Phenyl, worin Phenyl unsubstituiert oder ein-, zwei- oder dreifach durch T substituiert ist, steht,
R3 für
1) -(C₀-C₄)-Alkylen-(C₆-C₁₄)-Aryl, worin Aryl wie oben definiert ist und unsubstituiert oder ein-, zwei- oder dreifach durch T substituiert ist,
2) -(C₀-C₄)-Alkylen-Het, worin Het wie oben definiert ist und unsubstituiert oder ein-, zwei- oder dreifach durch T substituiert ist,
3) -(C₀-C₄)-Alkylen-(C₆-C₁₄)-Aryl-Q-(C₆-C₁₄)-Aryl, worin die beiden Aryle jeweils unabhängig voneinander wie oben definiert sind und jeweils unabhängig voneinander unsubstituiert oder ein-, zwei- oder dreifach durch T substituiert sind,
4) -(C₀-C₄)-Alkylen-(C₆-C₁₄)-Aryl-Q-(C₃-C₁₂)-Cycloalkyl, worin Aryl wie oben definiert ist und Cycloalkyl jeweils unabhängig voneinander unsubstituiert oder ein-, zwei- oder dreifach durch T substituiert sind,
5) -(C₀-C₄)-Alkylen-(C₆-C₁₄)-Aryl-Q-Het, worin Aryl und Het wie oben definiert sind und jeweils unabhängig voneinander unsubstituiert oder ein-, zwei- oder dreifach durch T substituiert sind,
6) -(C₀-C₄)-Alkylen-Het-Q-(C₆-C₁₄)-Aryl, worin Aryl und Het wie oben definiert sind und jeweils unabhängig voneinander unsubstituiert oder ein-, zwei- oder dreifach durch T substituiert sind, oder
7) -(C₀-C₄)-Alkylen-Het-Q-Het, worin die beiden Het-Reste wie oben definiert sind und jeweils unabhängig voneinander unsubstituiert oder ein-, zwei- oder dreifach durch T substituiert sind, steht,
Q für kovalente Bindung, -(C₁-C₄)-Alkylen, -NH-, -N((C₁-C₄)-Alkyl)- oder -O-steht,
T für
1) Halogen,
2) -(C₁-C₆)-Alkyl, worin Alkyl unsubstituiert oder unabhängig voneinander ein, zwei- oder dreifach durch -(C₁-C₃)-Fluoralkyl, -N-C(O)-OH oder -N-C(O)-(C₁-C₄)-Alkyl substituiert sind,
3) -(C₁-C₃)-Fluoralkyl,
4) -(C₃-C₆)-Cycloalkyl,
5) -OH,
6) -O-(C₁-C₄)-Alkyl,
7) -O-(C₁-C₃)-Fluoralkyl,
8) -NO₂,
9) -CN,
10) -N(R10)(R11), worin R10 und R11 unabhängig voneinander Wasserstoffatom, -(C₃-C₆)-Cycloalkyl, Halogen oder -(C₁-C₆)-Alkyl bedeuten,
11) -C(O)-NH-R10,
12) -NH-C(O)-R10,
13) -NH-SO₂-R10,
14) -SO₂-(C₁-C₄)-Alkyl,
15) -SO₂-NH-R10,
16) -SO₂-(C₁-C₃)-Fluoralkyl,
17) -S-(C₁-C₄)-Alkyl oder
18) -S-(C₁-C₃)-Fluoralkyl, steht,
R4 und R5 gleich oder verschieden sind und unabhängig voneinander Wasserstoffatom oder -(C₁-C₄)-Alkyl bedeuten, und
R6 für Wasserstoffatom, -C(O)-R12, -C(O)-O-R12, -C(O)-NH-R12 oder -(C₁-C₄)-Alkyl steht, wobei
R12 -(C₁-C₆)-Alkyl, -(C₃-C₆)-Cycloalkyl, -(C₆-C₁₄)-Aryl oder Het bedeutet.

2. Verbindung der Formel I gemäß den Anspruch 1, wobei
R1 für 4-Benzamidin, Aminomethylphenyl oder Het-Z steht, wobei Het ausgewählt ist aus der Gruppe Benzimidazolyl, Benzthiazolyl und Isochinolinyl, und worin Z für Amino steht,
R2 für Phenyl, worin Phenyl unsubstituiert oder ein- oder zweifach durch T substituiert ist, steht,
R3 für
1) Phenyl, worin Phenyl unsubstituiert oder ein- oder zweifach durch T substituiert ist,
2) worin Het-2, worin Het-2 ausgewählt ist aus der Gruppe Benzimidazolyl, Benzofuranyl, Benzothiophenyl, Chinolinyl, Chinoxalinyl, Furanyl, Indolyl, Isochinolinyl, Isoxazolyl, Morpholinyl, Piperidinyl, Pyridyl, Pyrimidinyl, Pyrrolidinyl, Pyrrolyl, Thienyl, Thienopyrrolyl oder Thienothiophenyl und Het-2 unsubstituiert oder ein- oder zweifach durch T substituiert ist,
3) -Phenyl-Q-Phenyl, worin die beiden Phenylreste jeweils unabhängig voneinander unsubstituiert oder ein- oder zweifach durch T substituiert sind,
4) Phenyl-Q-(C₃-C₆)-Cycloalkyl, worin Phenyl und Cycloalkyl jeweils unabhängig voneinander unsubstituiert oder ein- oder zweifach durch T substituiert sind,
5) Phenyl-Q-Het-2, worin Het-2 wie oben definiert ist und Phenyl und Het-2 jeweils unabhängig voneinander unsubstituiert oder ein- oder zweifach durch T substituiert sind,
6) Het-2-Q-Phenyl, worin Het-2 wie oben definiert ist und Phenyl und Het-2 jeweils unabhängig voneinander unsubstituiert oder ein- oder zweifach durch T substituiert sind, oder
7) Het-2-Q-Het-2, worin die beiden Het-2-Reste wie oben definiert sind und jeweils unabhängig voneinander unsubstituiert oder ein- oder zweifach durch T substituiert sind, steht,
Q für kovalente Bindung, -CH₂-, -N(CH₃)- oder -O- steht,
T für
1) F, Cl oder Br,
2) -(C₁-C₄)-Alkyl, worin Alkyl unsubstituiert oder unabhängig voneinander ein- oder zweifach durch -CF₃ oder -N-C(O)-CH₃ substituiert sind,
3) -CF₃,
4) -O-(C₁-C₄)-Alkyl,
5) -O-CF₃,
6) -NO₂,
7) -N(R10)(R11), worin R10 und R11 unabhängig voneinander Wasserstoffatom oder -(C₁-C₄)-Alkyl bedeuten,
8) -SO₂-CH₃,
9) -S-CF₃ oder
10) -S-(C₁-C₂)-Alkyl, steht,
R4, R5 und R6 jeweils Wasserstoffatom bedeuten.

3. Verbindung der Formel I gemäß den Ansprüchen 1 oder 2, **dadurch gekennzeichnet**, das es die Verbindung N-[(1S,2R)-2-(1-Amino-isochinolin-7-ylcarbamoyl)-2-hydroxy-1-phenyl-ethyl]-4-tert.-butyl-benzamid, N-[(1S,2R)-2-(1-Amino-isochinolin-6-ylcarbamoyl)-2-hydroxy-1-phenyl-ethyl]-4-diethylamino-benzamid, N-[(1S,2R)-2-(2-Amino-benzothiazol-6-ylcarbamoyl)-2-hydroxy-1-phenyl-ethyl]-4-isopropyl-benzamid, N-[(1S,2R)-2-(2-Amino-benzothiazol-6-ylcarbamoyl)-1-(2-chloro-phenyl)-2-hydroxy-ethyl]-4-isopropyl-benzamid, N-[(1S,2R)-2-(2-Amino-3H-benzimidazol-5-ylcarbamoyl)-1-(2-fluor-phenyl)-2-hydroxy-ethyl]-4-isopropyl-benzamid, N-[(1S,2R)-[2-(2-Amino-3H-benzimidazol-5-ylcarbamoyl)-2-hydroxy-1-phenyl-ethyl]-4-tertiär-butyl-benzamid, N-[(1S,2R)-2-(4-Aminomethyl-phenylcarbamoyl)-2-hydroxy-1-phenyl-ethyl]-benzamid, N-[(1S,2R)-2-(2,4-Diamino-chinazolin-6-ylcarbamoyl)-2-hydroxy-1-phenyl-ethyl]-benzamid, 4-Amino-N-[2-(2-amin-3H-benzimidazol-5-ylcarbamoyl)-2-hydroxy-1-phenyl-ethyl]-benzamid, Chinoxalin-2-carbonsäure [2-(2-amino-3H-benzimidazol-5-ylcarbamoyl)-2-hydroxy-1-phenyl-ethyl]-amid, N-[2-(2-Amino-3H-benzimidazol-5-ylcarbamoyl)-2-hydroxy-1-phenyl-ethyl]-4-methoxy-benzamid, Thiophen-3-carbonsäure [2-(2-amino-3H-benzimidazol-5-ylcarbamoyl)-2-hydroxy-1-phenyl-ethyl]-amid, 1-Methyl-1H-pyrrol-2-carbonsäure [(1S,2R)-2-(2-amino-3H-benzimidazol-5-ylcarbamoyl)-2-hydroxy-1-phenyl-ethyl]-amid, Chinolin-2-carbonsäure [2-(2-amino-3H-benzimidazol-5-ylcarbamoyl)-2-hydroxy-1-phenyl-ethyl]-amid, N-[2-(2-Amino-3H-benzimidazol-5-ylcarbamoyl)-2-hydroxy-1-phenyl-ethyl]-4-cyclohexyl-benzamid, N-[2-(2-Amino-3H-benzimidazol-5-ylcarbamoyl)-2-hydroxy-1-phenyl-ethyl]-4-isopropyl-benzamid, N-[2-(2-Amino-3H-benzimidazol-5-ylcarbamoyl)-2-hydroxy-1-phenyl-ethyl]-4-trifluormethoxy-benzamid, N-[2-(2-Amino-3H-benzimidazol-5-ylcarbamoyl)-2-hydroxy-1-phenyl-ethyl]-4-diethylamin-benzamid, N-[2-(2-Amino-3H-benzimidazol-5-ylcarbamoyl)-2-hydroxy-1-phenyl-ethyl]-phenoxy-benzamid, N-[2-(2-Amino-3H-benzimidazol-5-ylcarbamoyl)-2-hydroxy-1-phenyl-ethyl]-4-benzyl-benzamid, N-[2-(2-Amino-3H-benzimidazol-5-ylcarbamoyl)-2-hydroxy-1-phenyl-ethyl]-4-isopropoxy-benzamid, N-[2-(2-Amino-3H-benzimidazol-5-ylcarbamoyl)-2-hydroxy-1-phenyl-ethyl]-4-methylsulfanyl-benzamid, N-[2-(2-Amino-3H-benzimidazol-5-ylcarbamoyl)-2-hydroxy-1-phenyl-ethyl]-4-ethylsulfanyl-benzamid, N-[2-(2-Amino-3H-benzimidazol-5-ylcarbamoyl)-2-hydroxy-1-phenyl-ethyl]-4-[(4,6-dimethyl-pyrimidin-2-yl)-methyl-amin]-benzamid, N-[2-(2-Amino-3H-benzimidazol-5-ylcarbamoyl)-2-hydroxy-1-phenyl-ethyl]-4-pyridin-3-yl-benzamid, 4-(2-Acetylamino-3,3,3-trifluor-propyl)-N-[2-(2-amino-3H-benzimidazol-5-ylcarbamoyl)-2-hydroxy-1-phenyl-ethyl]-benzamid, 1-Methyl-1H-indol-2-carbonsäure [2-(2-amino-3H-benzimidazol-5-ylcarbamoyl)-2-hydroxy-1-phenyl-ethyl]-amid, 5-Methoxy-1H-indol-2-carbonsäure [2-(2-amino-3H-benzimidazol-5-ylcarbamoyl)-2- hydroxy-1-phenyl-ethyl]-amid, 5-Ethyl-1H-indol-2-carbonsäure [2-(2-amino-3H-benzimidazol-5-ylcarbamoyl)-2-hydroxy-1-phenyl-ethyl]-amid, 6-Methoxy-1H-indol-2-carbonsäure [2-(2-amino-3H-benzimidazol-5-ylcarbamoyl)-2-hydroxy-1-phenyl-ethyl]-amid, 4-Methoxy-chinolin-2-carbonsäure [2-(2-amino-3H-benzimidazol-5-ylcarbamoyl)-2-hydroxy-1-phenyl-ethyl]-amid, 3-Ethoxy-chinoxalin-2-carbonsäure [2-(2-amino-3H-benzimidazol-5-ylcarbamoyl)-2- hydroxy-1-phenyl-ethyl]-amid, Naphthalen-2-carbonsäure [2-(2-amino-3H-benzimidazol-5-ylcarbamoyl)-2-hydroxy-1-phenyl-ethyl]-amid, N-[2-(2-Amino-3H-benzimidazol-5-ylcarbamoyl)-2-hydroxy-1-phenyl-ethyl]-4-pyrrol-1-yl-benzamid, N-[2-(2-Amino-3H-benzimidazol-5-ylcarbamoyl)-2-hydroxy-1-phenyl-ethyl]-4-trifluormethylsulfanyl-benzamid, Biphenyl-4-carbonsäure [2-(2-amino-3H-benzimidazol-5-ylcarbamoyl)-2-hydroxy-1-phenyl-ethyl]-amid, N-[2-(2-Amino-3H-benzimidazol-5-ylcarbamoyl)-2-hydroxy-1-phenyl-ethyl]-4-(2,2,2-trifluor-ethoxy)-benzamid, [2-(2-Amino-3H-benzimidazol-5-ylcarbamoyl)-2-hydroxy-1-phenyl-ethyl]-carbaminsäure benzylester, N-[2-(2-Amino-3H-benzimidazol-5-ylcarbamoyl)-2-hydroxy-1-phenyl-ethyl]-4-ethyl-benzamid, N-[2-(2-Amino-3H-benzimidazol-5-ylcarbamoyl)-2-hydroxy-1-phenyl-ethyl]-4-ethoxy-benzamid, 5-Butyl-pyridine-2-carbonsäure [2-(2-amino-3H-benzimidazol-5-ylcarbamoyl)-2-hydroxy-1-phenyl-ethyl]-amid, Benz[b]thiophen-2-carbonsäure [2-(2-amino-3H-benzimidazol-5-ylcarbamoyl)-2-hydroxy-1-phenyl-ethyl]-amid, 6-Methoxy-naphthalen-2-carbonsäure [2-(2-amino-3H-benzimidazol-5-ylcarbamoyl)-2-hydroxy-1-phenyl-ethyl]-amid, 4-(2,2,2-Trifluor-ethyl)-4H-thien[3,2-b]pyrrol-5-carbonsäure [2-(2-amino-3H-benzimidazol-5-ylcarbamoyl)-2-hydroxy-1-phenyl-ethyl]-amid, Biphenyl-3,4'-dicarbonsäure 4'-{[2-(2-amino-3H-benzimidazol-5-ylcarbamoyl)-2-hydroxy-1-phenyl-ethyl]-amid} 3-methylamid, N-[2-(1-Amino-isochinolin-7-ylcarbamoyl)-2-hydroxy-1-phenyl-ethyl]-4-diethylamin-benzamid, N-[2-(1-Amino-isochinolin-7-ylcarbamoyl)-2-hydroxy-1-phenyl-ethyl]-4-isopropyl-benzamid, N-[2-(1-Amino-isochinolin-7-ylcarbamoyl)-2-hydroxy-1-phenyl-ethyl]-6-pyrrolidin-1-yl-nicotinamid, N-[2-(1-Amino-isochinolin-7-ylcarbamoyl)-2-hydroxy-1-phenyl-ethyl]-4-morpholin-4-yl-benzamid, N-[2-(1-Amino-isochinolin-7-ylcarbamoyl)-2-hydroxy-1-phenyl-ethyl]-4-pyrrol-1-yl-benzamid, 1-Ethyl-2,3-dimethyl-1 H-indol-5-carbonsäure [2-(2-amin-3H-benzimidazol-5-ylcarbamoyl)-2-hydroxy-1-phenyl-ethyl]-amid, N-[2-(2-Amin-3H-benzimidazol-5-ylcarbamoyl)-2-hydroxy-1-phenyl-ethyl]-4-morpholin-4-yl-benzamid, N-[2-(1-Amino-isochinolin-7-ylcarbamoyl)-2-hydroxy-1-phenyl-ethyl]-4-pyrrolidin-1-yl-benzamid, N-[2-(1-Amino-isochinolin-7-ylcarbamoyl)-2-hydroxy-1-phenyl-ethyl]-6-morpholin-4-yl-nicotinamid, 5,6-Dimethoxy-1H-indol-2-carbonsäure [2-(2-amin-3H-benzimidazol-5-ylcarbamoyl)-2-hydroxy-1-phenyl-ethyl]-amid, N-[2-(2-Amino-3H-benzimidazol-5-ylcarbamoyl)-2-hydroxy-1-phenyl-ethyl]-4-pyrrolidin-1-yl-benzamid, N-[2-(2-Amino-3H-benzimidazol-5-ylcarbamoyl)-2-hydroxy-1-phenyl-ethyl]-4-isobutyl-benzamid, N-[2-(2-Amino-3H-benzimidazol-5-ylcarbamoyl)-2-hydroxy-1-phenyl-ethyl]-4-butyl-benzamid, N-[2-(2-Amino-3H-benzimidazol-5-ylcarbamoyl)-2-hydroxy-1-phenyl-ethyl]-4-propyl-benzamid, 4H-Thieno[3,2-b]pyrrol-5-carbonsäure [2-(2-amin-3H-benzimidazol-5-ylcarbamoyl)-2-hydroxy-1-phenyl-ethyl]-amid, N-[2-(4-Carbamimidoyl-phenylcarbamoyl)-2-hydroxy-1-phenyl-ethyl]-4-diethylamin-benzamid, 5-Methoxy-1H-indol-2-carbonsäure [2-(1-amino-isochinolin-7-ylcarbamoyl)-2-hydroxy-1-phenyl-ethyl]-amid, 5-Ethyl-thiophene-2-carbonsäure [2-(1-amino-isochinolin-7-ylcarbamoyl)-2-hydroxy-1-phenyl-ethyl]-amid, 5-tert-Butyl-thiophen-2-carbonsäure [2-(1-amino-isochinolin-7-ylcarbamoyl)-2-hydroxy-1-phenyl-ethyl]-amid, N-[2-(1-Amino-isochinolin-6-ylcarbamoyl)-2-hydroxy-1-phenyl-ethyl]-benzamid, 6-Brom-benzofuran-2-carbonsäure [2-(2-amin-3H-benzimidazol-5-ylcarbamoyl)-2-hydroxyl-1-phenyl-ethyl]-amid, 5-Ethyl-thiophen-2-carbonsäure [2-(2-amino-3H-benzimidazol-5-ylcarbamoyl)-2-hydroxy-1-phenyl-ethyl]-amid, Thieno[3,2-b]thiophen-2-carbonsäure [2-(2-amino-3H-benzimidazol-5-ylcarbamoyl)-2-hydroxy-1-phenyl-ethyl]-amid, 5-Methoxy-benzofuran-2-carbonsäure [2-(2-amin-3H-benzimidazol-5-ylcarbamoyl)-2- hydroxy-1-phenyl-ethyl]-amid, Benzo[b]thiophen-3-carbonsäure [2-(2-amino-3H-benzimidazol-5-ylcarbamoyl)-2- hydroxy-1-phenylethyl]-amid, 4-Methyl-thiophen-2-carbonsäure [2-(2-amino-3H-benzimidazol-5-ylcarbamoyl)-2-hydroxy-1-phenyl-ethyl]-amid, N-[2-(2-Amin-benzothiazol-6-ylcarbamoyl)-2-hydroxy-1-phenyl-ethyl]-4-diethylamin-benzamid, 1H-Indol-2-carbonsäure [2-(2-amino-3H-benzimidazol-5-ylcarbamoyl)-2-hydroxy-1- phenylethyl]-amid, N-[2-(2-Amino-3H-benzimidazol-5-ylcarbamoyl)-2-hydroxy-1-phenyl-ethyl]-4-dimethylamin-benzamid, 5-tert-Butyl-thiophen-2-carbonsäure [2-(2-amino-3H-benzimidazol-5-ylcarbamoyl)-2-hydroxy-1-phenyl-ethyl]-amid, N-[2-(2-Amino-3H-benzimidazol-5-ylcarbamoyl)-2-hydroxy-1-phenyl-ethyl]-4-methoxy-3-methyl-benzamid, N-[2-(2-Amino-3H-benzimidazol-5-ylcarbamoyl)-2-hydroxy-1-phenyl-ethyl]-3-fluor-4-methyl-benzamid, 5-Isopropyl-thiophen-3-carbonsäure [2-(2-amin-3H-benzimidazol-5-ylcarbamoyl)-2-hydroxy-1-phenyl-ethyl]-amid, 5-Ethyl-thiophen-3-carbonsäure [2-(2-amino-3H-benzimidazol-5-ylcarbamoyl)-2- hydroxy-1-phenyl-ethyl]-amid, [2,2']Bithiophenyl-5-carbonsäure [2-(2-amino-3H-benzimidazol-5-ylcarbamoyl)-2-hydroxy-1-phenyl-ethyl]-amid, 5-Methoxy-1H-indol-2-carbonsäure [2-(1-amino-isochinolin-6-ylcarbamoyl)-2-hydroxy-1-phenyl-ethyl]-amid, N-[2-(1-Amino-isochinolin-6-ylcarbamoyl)-2-hydroxy-1-phenyl-ethyl]-4-methoxy-benzamid, N-[2-(1-Amino-isochinolin-6-ylcarbamoyl)-2-hydroxy-1-phenyl-ethyl]-4-isopropyl-benzamid, N-[2-(1-Amino-isochinolin-6-ylcarbamoyl)-2-hydroxy-1-phenyl-ethyl]-4-diethylamin-benzamid, N-[2-(1-Amino-isochinolin-6-ylcarbamoyl)-2-hydroxy-1-phenyl-ethyl]-4-ethoxy-benzamid, N-[2-(1-Amino-isochinolin-6-ylcarbamoyl)-2-hydroxy-1-phenyl-ethyl]-4-ethyl-benzamid, N-[2-(1-Amino-isochinolin-6-ylcarbamoyl)-2-hydroxy-1-phenyl-ethyl]-4-tert-butyl-benzamid, N-[2-(1-Amino-isochinolin-6-ylcarbamoyl)-2-hydroxy-1-phenyl-ethyl]-3-dimethylamin-benzamid, N-[2-(1-Amino-isochinolin-6-ylcarbamoyl)-2-hydroxy-1-phenyl-ethyl]-4-dimethylamin-benzamid, 5-Ethyl-thiophen-2-carbonsäure [2-(1-amino-isochinolin-6-ylcarbamoyl)-2-hydroxy-1-phenyl-ethyl]-amid, 5-Ethyl-thiophen-3-carbonsäure [2-(1-amino-isochinolin-6-ylcarbamoyl)-2-hydroxy-1-phenyl-ethyl]-amid, 5-tert-Butyl-thiophen-2-carbonsäure [2-(1-amino-isochinolin-6-ylcarbamoyl)-2-hydroxy-1-phenyl-ethyl]-amid, N-[2-(1-Amino-isochinolin-6-ylcarbamoyl)-2-hydroxy-1-phenyl-ethyl]-3-ethyl-benzamid, N-[2-(1-Amino-isochinolin-6-ylcarbamoyl)-2-hydroxy-1-phenylethyl]-3-ethoxy-benzamid, N-[2-(2-Amino-1H-benzimidazol-5-ylcarbamoyl)-2-hydroxy-1-(3-trifluormethyl-phenyl)-ethyl]-4-isopropyl-benzamid, N-[2-(2-Amino-1H-benzimidazol-5-ylcarbamoyl)-2-hydroxy-1-(2-methoxy-phenyl)-ethyl]-4-isopropyl-benzamid, N-[2-(2-Amino-1H-benzimidazol-5-ylcarbamoyl)-2-hydroxy-1-(3-trifluormethyl-phenyl)-ethyl]-4-diethylamin-benzamid, N-[2-(2-Amino-1H-benzimidazol-5-ylcarbamoyl)-2-hydroxy-1-(2-methoxy-phenyl)-ethyl]-4-diethylamin-benzamid, N-[2-(2-Amino-1H-benzimidazol-5-ylcarbamoyl)-2-hydroxy-1-(4-methoxy-phenyl)-ethyl]-4-diethylamin-benzamid, N-[2-(1-Amino-isochinolin-6-ylcarbamoyl)-1-(3-ethoxy-phenyl)-2-hydroxy-ethyl]-4-diethylamin-benzamid, N-[2-(2-Amino-3H-benzimidazol-5-ylcarbamoyl)-2-hydroxy-1-(4-isopropyl-phenyl)-ethyl]-4-diethylamin-benzamid, N-[2-(2-Amino-3H-benzimidazol-5-ylcarbamoyl)-1-(3-ethoxy-phenyl)-2-hydroxy-ethyl]-4-isopropyl-benzamid, N-[2-(2-Amino-3H-benzimidazol-5-ylcarbamoyl)-1-(3-ethoxy-phenyl)-2-hydroxy-ethyl]-4-diethylamin-benzamid, N-[2-(1-Amino-isochinolin-6-ylcarbamoyl)-2-hydroxy-1-m-tolyl-ethyl]-4-diethylamin-benzamid, N-[2-(2-Amino-3H-benzimidazol-5-ylcarbamoyl)-2-hydroxy-1-(4-isopropyl-phenyl)-ethyl]-4-isopropyl-benzamid, N-[2-(2-Amino-benzothiazol-6-ylcarbamoyl)-2-hydroxy-1-(4-trifluormethyl-phenyl)-ethyl]-4-isopropyl-benzamid, N-[2-(2-Amino-benzothiazol-6-ylcarbamoyl)-2-hydroxy-1-m-tolyl-ethyl]-4-diethylamin-benzamid, N-[2-(2-Amino-benzothiazol-6-ylcarbamoyl)-2-hydroxy-1-m-tolyl-ethyl]-4-isopropyl-benzamid, N-[2-(2-Amino-benzothiazol-6-ylcarbamoyl)-2-hydroxy-1-(4-isopropyl-phenyl)-ethyl]-4-diethylamin-benzamid, N-[2-(2-Amino-benzothiazol-6-ylcarbamoyl)-1-(3-ethoxy-phenyl)-2-hydroxy-ethyl]-4-diethylamin-benzamid, N-[2-(2-Amino-benzothiazol-6-ylcarbamoyl)-2-hydroxy-1-(4-trifluormethyl-phenyl)-ethyl]- 4-diethylamin-benzamid, N-[2-(2-Amino-3H-benzimidazol-5-ylcarbamoyl)-1-(2-chlor-phenyl)-2-hydroxy-ethyl]-4-diethylamin-benzamid, N-[2-(2-Amino-3H-benzimidazol-5-ylcarbamoyl)-2-hydroxy-1-(4-trifluormethyl-phenyl)-ethyl]-4-isopropyl-benzamid, N-[2-(2-Amino-3H-benzimidazol-5-ylcarbamoyl)-2-hydroxy-1-(4-trifluormethyl-phenyl)-ethyl]-4-diethylamin-benzamid, N-[2-(2-Amino-benzothiazol-6-ylcarbamoyl)-1-(3-ethoxy-phenyl)-2-hydroxy-ethyl]-4-isopropyl-benzamid, N-[2-(2-Amino-benzothiazol-6-ylcarbamoyl)-1-(2-chlor-phenyl)-2-hydroxy-ethyl]-4-diethylamin-benzamid, N-[2-(1-Amino-isochinolin-6-ylcarbamoyl)-1-(3-brom-phenyl)-2-hydroxy-ethyl]-4-diethylamin-benzamid, N-[2-(1-Amino-isochinolin-6-ylcarbamoyl)-1-(3-brom-phenyl)-2-hydroxy-ethyl]-4-ethyl-benzamid, N-[2-(1-Amino-isochinolin-6-ylcarbamoyl)-2-hydroxy-1-(4-isopropyl-phenyl)-ethyl]-4-isopropyl-benzamid, N-[2-(1-Amino-isochinolin-6-ylcarbamoyl)-1-(2-chlor-phenyl)-2-hydroxy-ethyl]-4-isopropyl-benzamid, N-[2-(2-Amino-3H-benzimidazol-5-ylcarbamoyl)-2-hydroxy-1-m-tolyl-ethyl]-4-isopropyl-benzamid, N-[2-(2-Amino-3H-benzimidazol-5-ylcarbamoyl)-2-hydroxy-1-m-tolyl-ethyl]-4-diethylamin-benzamid, N-[2-(1-Amino-isochinolin-6-ylcarbamoyl)-1-(4-brom-phenyl)-2-hydroxy-ethyl]-4-diethylamin-benzamid, N-[2-(1-Amino-isochinolin-6-ylcarbamoyl)-1-(4-brom-phenyl)-2-hydroxy-ethyl]-4-isopropyl-benzamid, N-[2-(1-Amino-isochinolin-6-ylcarbamoyl)-1-(3-brom-phenyl)-2-hydroxy-ethyl]-4-isopropyl-benzamid, N-[2-(1-Amino-isochinolin-6-ylcarbamoyl)-2-hydroxy-1-m-tolyl-ethyl]-4-isopropyl-benzamid, N-[2-(1-Amino-isochinolin-6-ylcarbamoyl)-1-(3-ethoxy-phenyl)-2-hydroxy-ethyl]-4-isopropyl-benzamid, N-[2-(1-Amino-isochinolin-6-ylcarbamoyl)-2-hydroxy-1-(4-isopropyl-phenyl)-ethyl]-4-diethylamin-benzamid, N-[2-(1-Amino-isochinolin-6-ylcarbamoyl)-1-(2-chlor-phenyl)-2-hydroxy-ethyl]-4-diethylamin-benzamid, N-[2-(1-Amino-isochinolin-6-ylcarbamoyl)-2-hydroxy-1-(4-trifluormethyl-phenyl)-ethyl]- 4-diethylamin-benzamid, N-[2-(1-Amino-isochinolin-6-ylcarbamoyl)-2-hydroxy-1-(4-trifluormethyl-phenyl)-ethyl]-4-isopropyl-benzamid, N-[2-(1-Amino-isochinolin-6-ylcarbamoyl)-1-(4-brom-phenyl)-2-hydroxy-ethyl]-4-ethyl-benzamid, N-[2-(2-Amino-3H-benzimidazol-5-ylcarbamoyl)-1-(3,5-dimethoxy-phenyl)-2-hydroxy-ethyl]-4-isopropyl-benzamid, N-[2-(2-Amino-3H-benzimidazol-5-ylcarbamoyl)-1-(3,5-dimethoxy-phenyl)-2-hydroxy-ethyl]-4-diethylamin-benzamid, N-[2-(1-Amino-isochinolin-6-ylcarbamoyl)-2-hydroxy-1-phenyl-ethyl]-4-propyl-benzamid, N-[2-(2-Amino-3H-benzimidazol-5-ylcarbamoyl)-1-(2-chloro-phenyl)-2-hydroxy-ethyl]-4-isopropyl-benzamid, N-[2-(1-Amino-isochinolin-6-ylcarbamoyl)-2-hydroxy-1-phenyl-ethyl]-4-cyclopropyl-benzamid, N-[2-(1-Amino-isochinolin-6-ylcarbamoyl)-1-(2-chlor-phenyl)-2-hydroxy-ethyl]-4-ethyl-benzamid, N-[2-(2-Amino-benzothiazol-6-ylcarbamoyl)-1-furan-2-yl-2-hydroxy-ethyl]-4-diethylamin-benzamid, N-[2-(2-Amino-3H-benzimidazol-5-ylcarbamoyl)-1-(2-fluor-phenyl)-2-hydroxy-ethyl]-4-diethylamin-benzamid, N-[2-(2-Amino-3H-benzimidazol-5-ylcarbamoyl)-1-(3,5-difluorphenyl)-2-hydroxy-ethyl]-4-isopropyl-benzamid, N-[2-(2-Amino-3H-benzimidazol-5-ylcarbamoyl)-1-(3,5-difluor-phenyl)-2-hydroxy-ethyl]-4-diethylamin-benzamid, N-[2-(2-Amino-3H-benzimidazol-5-ylcarbamoyl)-1-(5-fluor-2-methansulfonyl-phenyl)-2-hydroxy-ethyl]-4-diethylamin-benzamid, N-[2-(2-Amino-3H-benzimidazol-5-ylcarbamoyl)-1-(5-fluor-2-methansulfonyl-phenyl)-2-hydroxy-ethyl]-4-diethylamin-benzamid, N-[2-(2-Amino-3H-benzimidazol-5-ylcarbamoyl)-1-(3-chloro-phenyl)-2-hydroxy-ethyl]-4-diethylamin-benzamid, N-[2-(2-Amino-3H-benzimidazol-5-ylcarbamoyl)-1-(3-chlor-phenyl)-2-hydroxy-ethyl]-4-diethylamin-benzamid, N-[2-(2-Amino-3H-benzimidazol-5-ylcarbamoyl)-2-hydroxy-1-(2-trifluormethoxy-phenyl)-ethyl]-4-isopropyl-benzamid, N-[2-(2-Amino-3H-benzimidazol-5-ylcarbamoyl)-2-hydroxy-1-(2-trifluormethoxy-phenyl)-ethyl]-4-diethylamin-benzamid N-[2-(2-Amino-3H-benzimidazol-5-ylcarbamoyl)-2-hydroxy-1-o-tolyl-ethyl]-4-diethylamin-benzamid, N-[2-(2-Amino-3H-benzimidazol-5-ylcarbamoyl)-2-hydroxy-1-thiophen-2-yl-ethyl]-4-diethylamin-benzamid, [(1S,2R)-2-(2-Amino-3H-benzimidazol-5-ylcarbamoyl)-2-hydroxy-1-phenyl-ethyl] carbaminsäure 9H-fluoren-9-ylmethylester oder [2-(2-Amino-3H-benzimidazol-5-ylcarbamoyl)-2-hydroxy-1-phenyl-ethyl]-carbaminsäure 2-chlorbenzylester ist.

4. Verfahren zur Herstellung der Verbindung der Formel I gemäß einem oder mehreren der Ansprüche 1 bis 3, **dadurch gekennzeichnet, dass** man
a) eine Verbindung der Formel II 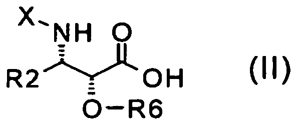 wobei X für eine Aminoschutzgruppe steht und die Reste R2 und R6 wie in Formel I definiert sind mit einer Verbindung NH(R1)(R4) zu einer Verbindung der Formel III umsetzt, 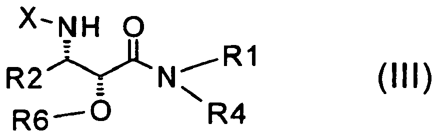 wobei X für eine Aminoschutzgruppe steht und die Reste R1, R2, R4 und R6 wie in Formel I definiert sind und die Verbindung der Formel III durch Abspaltung der Schutzgruppe in eine Verbindung der Formel IV umwandelt, 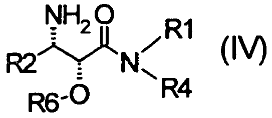 wobei die Reste R1, R2, R4 und R6 wie in Formel I definiert sind und mit der Verbindung der Formel V 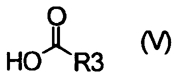 zur Verbindung der Formel umsetzt, oder
b) die nach Verfahren a) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
c) eine nach Verfahren a) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

5. Arzneimittel, **gekennzeichnet durch** einen wirksamen Gehalt an mindestens einer Verbindung der Formel I gemäß einem oder mehreren der Ansprüche 1 bis 3 zusammen mit einem pharmazeutisch geeigneten und physiologisch verträglichen Trägerstoff, Zusatzstoff und/oder anderen Wirk- und Hilfsstoffen.

6. Verwendung der Verbindung der Formel I gemäß einem oder mehreren der Ansprüche 1 bis 3 zur Herstellung eines Arzneimittels zur Prophylaxe, Sekundärprävention und Therapie all solcher Erkrankungen, die die mit Thrombosen, Embolien, Hyperkoagulabilität oder fibrotischen Veränderungen einhergehen.

7. Verwendung gemäß Anspruch 6, **dadurch gekennzeichnet, dass** es sich um Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder um die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkserkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung und/oder Behandlung von Narbenbildung.

## Claims

1. A compound of the formula I 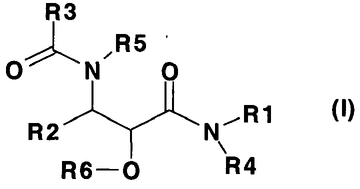 and/or all stereoisomeric forms of the compound of the formula I and/or mixtures of these forms in any ratio, and/or a physiologically tolerable salt of the compound of the formula I, where
R1 is
1) -(C6-C14)-aryl-Z, where aryl is selected from the group consisting of phenyl and naphthyl, and in which aryl is unsubstituted or mono-, di- or trisubstituted by T and Z is amino, amidino, aminomethylene, aminopyridinyl, azetidinyl, guanidino, piperidinyl, pyridinyl or pyrrolidinyl, or
2) a four- to fifteen-membered Het-Z, where Het is selected from the group consisting of acridinyl, azepinyl, azetidinyl, benzimidazolinyl, benzimidazolyl, benzofuranyl, benzothiofuranyl, benzothiophenyl, benzoxazolyl, benzothiazolyl, benzotriazolyl, benzotetrazolyl, benzisoxazolyl, benzisothiazolyl, carbazolyl, 4aH-carbazolyl, carbolinyl, beta-carbolinyl, quinazolinyl, quinolinyl, quinolizinyl, 4H-quinolizinyl, quinoxalinyl, quinuclidinyl, chromanyl, chromenyl, cinnolinyl, deca-hydroquinolinyl, dibenzofuranyl, dibenzothiophenyl, dihydrofuran[2,3-b]-tetrahydrofuranyl, dihydrofuranyl, dioxolyl, dioxanyl, dioxolenyl, 2H, 6H-1,5,2-dithiazinyl, furanyl, furazanyl, imidazolidinyl, imidazolinyl, imidazolyl, 1H-indazolyl, indolinyl, indolizinyl, indolyl, 3H-indolyl, isobenzofuranyl, isoquinolinyl, isochromanyl, isoindazolyl, isoindolinyl, isoindolyl, isothiazolidinyl, 2-isothiazolinyl, isothiazolyl, isoxazolyl, isoxazolidinyl, 2-isoxazolinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, oxothiolanyl, phenanthridinyl, phenanthrenyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridothiophenyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadazinyl, 1,2,3-thiadiazolyl, 1,2,4-thiadiazolyl, 1,2,5-thiadiazolyl, 1,3,4-thiadiazolyl, thianthrenyl, thiazinyl, thiazolyl, thienyl, thienoimidazolyl, thienooxazolyl, thienopyridinyl, thienopyrrolyl, thienothiazolyl, thienothiophenyl, thiomorpholinyl, thiopyranyl, triazinyl, 1,2,3-triazolyl, 1,2,4-triazolyl, 1,2,5-triazolyl, 1,3,4-triazolyl or xanthenyl and in which Het is unsubstituted or mono-, di- or trisubstituted by T and in which Z is as defined above,
R2 is -(C0-C4)-alkylene-(C6-C14)-phenyl, in which phenyl is unsubstituted or mono-, di- or trisubstituted by T,
R3 is
1) -(C0-C4)-alkylene-(C6-C14)-aryl, in which aryl is as defined above and is unsubstituted or mono-, di- or trisubstituted by T,
2) -(C0-C4)-alkylene-Het, in which Het is as defined above and is unsubstituted or mono-, di- or trisubstituted by T,
3) -(C0-C4)-alkylene-(C6-C14)-aryl-Q-(C6-C14)-aryl, in which the two aryls in each case independently of one another are as defined above and in each case independently of one another are unsubstituted or mono-, di- or trisubstituted by T,
4) -(C0-C4)-alkylene-(C6-C14)-aryl-Q-(C3-C12)-cycloalkyl, in which aryl is as defined above and cycloalkyl is unsubstituted or mono-, di- or trisubstituted by T,
5) -(C0-C4)-alkylene-(C6-C14)-aryl-Q-Het, in which aryl and Het are as defined above and in each case independently of one another are unsubstituted or mono-, di- or trisubstituted by T,
6) -(C0-C4)-alkylene-Het-Q-(C6-C14)-aryl, in which aryl and Het are as defined above and in each case independently of one another are unsubstituted or mono-, di- or trisubstituted by T, or
7) -(C0-C4)-alkylene-Het-Q-Het, in which the two Het radicals are as defined above and in each case independently of one another are unsubstituted or mono-, di- or trisubstituted by T,
Q is a covalent bond, -(C1-C4)-alkylene, -NH-, - N((C1-C4)-alkyl)- or -O-,
T is
1) halogen,
2) -(C1-C6)-alkyl, in which alkyl is unsubstituted or mono-, di- or trisubstituted by -(C1-C3)-fluoroalkyl, -N-C(O)-OH or -N-C(O)-(C1-C4)-alkyl,
3) -(C1-C3)-fluoroalkyl,
4) -(C3-C6)-cycloalkyl,
5) -OH,
6) -O-(C1-C4)-alkyl,
7) -O-(C1-C3)-fluoroalkyl,
8) -NO2,
9) -CN,
10) -N(R10)(R11), in which R10 and R11 independently of one another are a hydrogen atom, -(C3-C6)-cycloalkyl, halogen or -(C1-C6)-alkyl,
11) -C(O)-NH-R10,
12) -NH-C(O)-R10, 13) -NH-SO2-R10,
14) -SO2-(C1-C4)-alkyl,
15) -SO2-NH-R10,
16) -SO2-(C1-C3)-fluoroalkyl,
17) -S-(C1-C4)-alkyl or
18) -S-(C1-C3)-fluoroalkyl,
R4 and R5 are identical or different and independently of one another are a hydrogen atom or -(C1-C4)-alkyl, and
R6 is a NH-R12 hydrogen atom, -C(O)-R12, -C(O)-O-R12, -C(O)-or -(C1-C4)-alkyl, where
R12 is -(C1-C6)-alkyl, -(C3-C6)-cycloalkyl, -(C6-C14)- aryl or Het.

2. A compound of the formula I as claimed in claim 1, where
R1 is 4-benzamidine, aminomethylphenyl or Het-Z, where Het is selected from the group consisting of benzimidazolyl, benzothiazolyl and isoquinolinyl, and in which Z is amino,
R2 is phenyl, in which phenyl is unsubstituted or mono- or disubstituted by T,
R3 is
1) phenyl, in which phenyl is unsubstituted or mono- or disubstituted by T,
2) Het-2, in which Het-2 is selected from the group consisting of benzimidazolyl, benzofuranyl, benzothiophenyl, quinolinyl, quinoxalinyl, furanyl, indolyl, isoquinolinyl, isoxazolyl, morpholinyl, piperidinyl, pyridyl, pyrimidinyl, pyrrolidinyl, pyrrolyl, thienyl, thienopyrrolyl or thienothiophenyl and in which Het-2 is unsubstituted or mono- or disubstituted by T,
3) -phenyl-Q-phenyl, in which the two phenyl radicals in each case independently of one another are unsubstituted or mono- or disubstituted by T,
4) phenyl-Q-(C3-C6)-cycloalkyl, in which phenyl and cycloalkyl in each case independently of one another are unsubstituted or mono- or disubstituted by T,
5) phenyl-Q-Het-2, in which Het-2 is as defined above and phenyl and Het-2 in each case independently of one another are unsubstituted or mono- or disubstituted by T,
6) Het-2-Q-phenyl, in which Het-2 is as defined above and phenyl and Het-2 in each case independently of one another are unsubstituted or mono- or disubstituted by T, or
7) Het-2-Q-Het-2, in which the two Het-2 radicals are as defined above and in each case independently of one another are unsubstituted or mono- or disubstituted by T,
Q is a covalent bond, -CH2-, -N(CH3)- or -0-,
T is
1) F, Cl or Br,
2) -(C1-C4)-alkyl, in which alkyl is unsubstituted or mono- or disubstituted by -CF3 or -N-C(O)-CH3,
3) -CF3,
4) -O-(C1-C4)-alkyl,
5) -O-CF3,
6) -NO2,
7) -N(R10) (R11), in which R10 and R11 independently of one another are a hydrogen atom or -(C1-C4)-alkyl,
8) -SO2-CH3,
9) -S-CF3 or
10) -S-(C1-C2)-alkyl,
R4, R5 and R6 are in each case a hydrogen atom.

3. A compound of the formula I as claimed in claims 1 or 2, which is the compound N-[(1S,2R)-2-(1-aminoisoquinolin-7-ylcarbamoyl)-2-hydroxy-1-phenylethyl]-4-tert-butylbenzamide, N-[(1S,2R)-2-(1-aminoisoquinolin-6-ylcarbamoyl)-2-hydroxy-1-phenylethyl]-4-diethylaminobenzamide, N-[(1S,2R)-2-(2-aminobenzothiazol-6-ylcarbamoyl)-2-hydroxy-1-phenylethyl]-4-isopropylbenzamide, N-[(1S,2R)-2-(2-aminobenzothiazol-6-ylcarbamoyl)-1-(2-chlorophenyl)-2-hydroxyethyl]-4-isopropylbenzamide, N-[(1S,2R)-2-(2-amino-3H-benzimidazol-5-ylcarbamoyl)-1-(2-fluorophenyl)-2-hydroxyethyl]-4-isopropylbenzamide, N-[(1S,2R)-[2-(2-amino-3H-benzimidazol-5-ylcarbamoyl)-2-hydroxy-1-phenylethyl]-4-tertiary-butylbenzamide, N-[(1S,2R)-2-(4-aminomethylphenylcarbamoyl)-2-hydroxy-1-phenylethyl]benzamide, N-[(1S,2R)-2-(2,4-diaminoquinazolin-6-ylcarbamoyl)-2-hydroxy-1-phenylethyl]benzamide, 4-amino-N-[2-(2-amino-3H-benzimidazol-5-ylcarbamoyl)-2-hydroxy-1-phenylethyl]benzamide, quinoxaline-2-carboxylic acid [2-(2-amino-3H-benzimidazol-5-ylcarbamoyl)-2-hydroxy-1-phenylethyl] amide, N-[2-(2-amino-3H-benzimidazol-5-ylcarbamoyl)-2-hydroxy-1-phenylethyl]-4-methoxybenzamide, thiophene-3-carboxylic acid [2-(2-amino-3H-benzimidazol-5-ylcarbamoyl)-2-hydroxy-1-phenylethyl]amide, 1-methyl-1H-pyrrole-2-carboxylic acid [(1S,2R)-2-(2-amino-3H-benzimidazol-5-ylcarbamoyl)-2-hydroxy-1-phenylethyl]amide, quinoline-2-carboxylic acid [2-(2-amino-3H-benzimidazol-5-ylcarbamoyl)-2-hydroxy-1-phenylethyl]amide, N-[2-(2-amino-3H-benzimidazol-5-ylcarbamoyl)-2-hydroxy-1-phenylethyl]-4-cyclohexylbenzamide, N-[2-(2-amino-3H-benzimidazol-5-ylcarbamoyl)-2-hydroxy-1-phenylethyl]-4-isopropylbenzamide, N-[2-(2-amino-3H-benzimidazol-5-ylcarbamoyl)-2-hydroxy-1-phenylethyl]-4-trifluoromethoxy-benzamide, N-[2-(2-amino-3H-benzimidazol-5-ylcarbamoyl)-2-hydroxy-1-phenylethyl]-4-diethylaminobenzamide, N-[2-(2-amino-3H-benzimidazol-5-ylcarbamoyl)-2-hydroxy-1-phenylethyl]-4-phenoxybenzamide, N-[2-(2-amino-3H-benzimidazol-5-ylcarbamoyl)-2-hydroxy-1-phenylethyl]-4-benzyl-benzamide, N-[2-(2-amino-3H-benzimidazol-5-ylcarbamoyl)-2-hydroxy-1-phenylethyl]-4-isopropoxybenzamide, N-[2-(2-amino-3H-benzimidazol-5-ylcarbamoyl)-2-hydroxy-1-phenylethyl]-4-methylsulfanylbenzamide, N-[2-(2-amino-3H-benzimidazol-5-ylcarbamoyl)-2-hydroxy-1-phenylethyl]-4-ethylsulfanylbenzamide, N-[2-(2-amino-3H-benzimidazol-5-ylcarbamoyl)-2-hydroxy-1-phenylethyl]-4-[(4,6-dimethylpyrimidin-2-yl)methyl-amino]benzamide, N-[2-(2-amino-3H-benzimidazol-5-ylcarbamoyl)-2-hydroxy-1-phenylethyl]-4-pyridin-3-ylbenzamide, 4-(2-acetylamino-3,3,3-trifluoro-propyl)-N-[2-(2-amino-3H-benzimidazol-5-ylcarbamoyl)-2-hydroxy-1-phenylethyl]benzamide, 1-methyl-1H-indole-2-carboxylic acid [2-(2-amino-3H-benzimidazol-5-ylcarbamoyl)-2-hydroxy-1-phenylethyl]amide, 5-methoxy-1H-indole-2-carboxylic acid [2-(2-amino-3H-benzimidazol-5-ylcarbamoyl)-2- hydroxy-1-phenylethyl]amide, 5-ethyl-1H-indole-2-carboxylic acid [2-(2-amino-3H-benzimidazol-5-ylcarbamoyl)-2-hydroxy-1-phenylethyl]amide, 6-methoxy-1H-indole-2-carboxylic acid [2-(2-amino-3H-benzimidazol-5-ylcarbamoyl)-2- hydroxy-1-phenylethyl]amide, 4-methoxyquinoline-2-carboxylic acid [2-(2-amino-3H-benzimidazol-5-ylcarbamoyl)-2-hydroxy-1-phenylethyl]amide, 3-ethoxyquinoxaline-2-carboxylic acid [2-(2-amino-3H-benzimidazol-5-ylcarbamoyl)-2- hydroxy-1-phenylethyl]amide, naphthalene-2-carboxylic acid [2-(2-amino-3H-benzimidazol-5-ylcarbamoyl)-2-hydroxy-1-phenylethyl]amide, N-[2-(2-amino-3H-benzimidazol-5-ylcarbamoyl)-2-hydroxy-1-phenylethyl]-4-pyrrol-1-ylbenzamide, N-[2-(2-amino-3H-benzimidazol-5-ylcarbamoyl)-2-hydroxy-l-phenylethyl]-4-trifluoromethylsulfanylbenzamide, biphenyl-4-carboxylic acid [2-(2-amino-3H-benzimidazol-5-ylcarbamoyl)-2-hydroxy-1-phenylethyl]amide, N-[2-(2-amino-3H-benzimidazol-5-ylcarbamoyl)-2-hydroxy-1-phenylethyl]-4-(2,2,2-trifluoroethoxy)benzamide, benzyl [2-(2-amino-3H-benzimidazol-5-ylcarbamoyl)-2-hydroxy-1-phenylethyl]carbamate, N-[2-(2-amino-3H-benzimidazol-5-ylcarbamoyl)-2-hydroxy-1-phenylethyl]-4-ethylbenzamide, N-[2-(2-amino-3H-benzimidazol-5-ylcarbamoyl)-2-hydroxy-1-phenylethyl]-4-ethoxybenzamide, 5-butylpyridine-2-carboxylic acid [2-(2-amino-3H-benzimidazol-5-ylcarbamoyl)-2-hydroxy-l-phenylethyl]amide, benzo[b]thiophene-2-carboxylic acid [2-(2-amino-3H-benzimidazol-5-ylcarbamoyl)-2-hydroxy-1-phenylethyl]amide, 6-methoxynaphthalene-2-carboxylic acid [2-(2-amino-3H-benzimidazol-5-ylcarbamoyl)-2- hydroxy-1-phenylethyl]amide, 4-(2,2,2-trifluoroethyl)-4H-thieno[3,2-b]pyrrole-5-carboxylic acid [2-(2-amino-3H-benzimidazol-5-ylcarbamoyl)-2-hydroxy-1-phenylethyl]amide, biphenyl-3,4'-dicarboxylic acid 4'-{[2-(2-amino-3H-benzimidazol-5-ylcarbamoyl)-2-hydroxy-1-phenylethyl]amide} 3-methylamide, N-[2-(1-aminoisoquinolin-7-ylcarbamoyl)-2-hydroxy-1-phenylethyl]-4-diethylaminobenzamide, N-[2-(1-aminoisoquinolin-7-ylcarbamoyl)-2-hydroxy-1-phenylethyl]-4-isopropylbenzamide, N-[2-(1-aminoisoquinolin-7-ylcarbamoyl)-2-hydroxy-1-phenylethyl]-6-pyrrolidin-1-ylnicotinamide, N-[2-(1-aminoisoquinolin-7-ylcarbamoyl)-2-hydroxy-1-phenylethyl]-4-morpholin-4-ylbenzamide, N-[2-(1-aminoisoquinolin-7-ylcarbamoyl)-2-hydroxy-1-phenylethyl]-4-pyrrol-1-ylbenzamide, 1-ethyl-2,3-dimethyl-1H-indole-5-carboxylic acid [2-(2-amino-3H-benzimidazol-5-ylcarbamoyl)-2-hydroxy-1-phenylethyl]amide, N-[2-(2-amino-3H-benzimidazol-5-ylcarbamoyl)-2-hydroxy-l-phenylethyl]-4-morpholin-4-ylbenzamide, N-[2-(1-aminoisoquinolin-7-ylcarbamoyl)-2-hydroxy-l-phenylethyl]-4-pyrrolidin-1-ylbenzamide, N-[2-(1-aminoisoquinolin-7-ylcarbamoyl)-2-hydroxy-1-phenylethyl]-6-morpholin-4-yl-nicotinamide, 5,6-dimethoxy-1H-indole-2-carboxylic acid [2-(2-amino-3H-benzimidazol-5-ylcarbamoyl)-2-hydroxy-1-phenylethyl]amide, N-[2-(2-amino-3H-benzimidazol-5-ylcarbamoyl)-2-hydroxy-1-phenylethyl]-4-pyrrolidin-1-ylbenzamide, N-[2-(2-amino-3H-benzimidazol-5-ylcarbamoyl)-2-hydroxy-1-phenylethyl]-4-isobutylbenzamide, N-[2-(2-amino-3H-benzimidazol-5-ylcarbamoyl)-2-hydroxy-1-phenylethyl]-4-butylbenzamide, N-[2-(2-amino-3H-benzimidazol-5-ylcarbamoyl)-2-hydroxy-1-phenylethyl]-4-propylbenzamide, 4H-thieno[3,2-b]pyrrole-5-carboxylic acid [2-(2-amino-3H-benzimidazol-5-ylcarbamoyl)-2-hydroxy-1-phenylethyl]amide, N-[2-(4-carbamimidoyl-phenylcarbamoyl)-2-hydroxy-1-phenylethyl]-4-diethylaminobenzamide, 5-methoxy-1H-indole-2-carboxylic acid [2-(1-aminoisoquinolin-7-ylcarbamoyl)-2-hydroxy-l-phenylethyl]amide, 5-ethylthiophene-2-carboxylic acid [2-(1-aminoisoquinolin-7-ylcarbamoyl)-2-hydroxy-1-phenylethyl]amide, 5-tert-butyl-thiophene-2-carboxylic acid [2-(1-aminoisoquinolin-7-ylcarbamoyl)-2-hydroxy-1-phenylethyl]amide, N-[2-(1-aminoisoquinolin-6-ylcarbamoyl)-2-hydroxy-1-phenylethyl]benzamide, 6-bromobenzofuran-2-carboxylic acid [2-(2-amino-3H-benzimidazol-5-ylcarbamoyl)-2-hydroxy-l-phenylethyl]amide, 5-ethyl-thiophene-2-carboxylic acid [2-(2-amino-3H-benzimidazol-5-ylcarbamoyl)-2-hydroxy-1-phenylethyl]amide, thieno[3,2-b]thiophene-2-carboxylic acid [2-(2-amino-3H-benzimidazol-5-ylcarbamoyl)-2-hydroxy-l-phenylethyl]amide, 5-methoxybenzofuran-2-carboxylic acid [2-(2-amino-3H-benzimidazol-5-ylcarbamoyl)-2- hydroxy-1-phenylethyl]amide, benzo[b]thiophene-3-carboxylic acid [2-(2-amino-3H-benzimidazol-5-ylcarbamoyl)-2-hydroxy-1-phenylethyl]amide, 4-methylthiophene-2-carboxylic acid [2-(2-amino-3H-benzimidazol-5-ylcarbamoyl)-2-hydroxy-l-phenylethyl]amide, N-[2-(2-amino-benzothiazol-6-ylcarbamoyl)-2-hydroxy-1-phenylethyl]-4-diethylamino-benzamide, 1H-indole-2-carboxylic acid [2-(2-amino-3H-benzimidazol-5-ylcarbamoyl)-2-hydroxy-l-phenylethyl]amide, N-[2-(2-amino-3H-benzimidazol-5-ylcarbamoyl)-2-hydroxy-1-phenylethyl]-4-dimethylaminobenzamide, 5-tert-butylthiophene-2-carboxylic acid [2-(2-amino-3H-benzimidazol-5-ylcarbamoyl)-2-hydroxy-1-phenylethyl]amide, N-[2-(2-amino-3H-benzimidazol-5-ylcarbamoyl)-2-hydroxy-1-phenylethyl]-4-methoxy-3-methylbenzamide, N-[2-(2-amino-3H-benzimidazol-5-ylcarbamoyl)-2-hydroxy-1-phenylethyl]-3-fluoro-4-methylbenzamide, 5-isopropylthiophene-3-carboxylic acid [2-(2-amino-3H-benzimidazol-5-ylcarbamoyl)-2-hydroxy-l-phenylethyl]amide, 5-ethylthiophene-3-carboxylic acid [2-(2-amino-3H-benzimidazol-5-ylcarbamoyl)-2-hydroxy-1-phenylethyl]amide, [2,2_{'}]bithiophenyl-5-carboxylic acid [2-(2-amino-3H-benzimidazol-5-ylcarbamoyl)-2-hydroxy-1-phenylethyl]amide, 5-methoxy-1H-indole-2-carboxylic acid [2-(1-aminoisoquinolin-6-ylcarbamoyl)-2-hydroxy-1-phenylethyl]amide, N-[2-(1-aminoisoquinolin-6-ylcarbamoyl)-2-hydroxy-1-phenylethyl]-4-methoxybenzamide, N-[2-(1-aminoisoquinolin-6-ylcarbamoyl)-2-hydroxy-1-phenylethyl]-4-isopropylbenzamide, N-[2-(1-aminoisoquinolin-6-ylcarbamoyl)-2-hydroxy-1-phenylethyl]-4-diethylaminobenzamide, N-[2-(1-aminoisoquinolin-6-ylcarbamoyl)-2-hydroxy-1-phenylethyl]-4-ethoxybenzamide, N-[2-(1-aminoisoquinolin-6-ylcarbamoyl)-2-hydroxy-1-phenylethyl]-4-ethylbenzamide, N-[2-(1-aminoisoquinolin-6-ylcarbamoyl)-2-hydroxy-1-phenylethyl]-4-tert-butylbenzamide, N-[2-(1-aminoisoquinolin-6-ylcarbamoyl)-2-hydroxy-1-phenylethyl]-3-dimethylaminobenzamide, N-[2-(1-aminoisoquinolin-6-ylcarbamoyl)-2-hydroxy-1-phenylethyl]-4-dimethylaminobenzamide, 5-ethyl-thiophene-2-carboxylic acid [2-(1-aminoisoquinolin-6-ylcarbamoyl)-2-hydroxy-1-phenylethyl]amide, 5-ethylthiophene-3-carboxylic acid [2-(1-aminoisoquinolin-6-ylcarbamoyl)-2-hydroxy-1-phenylethyl]amide, 5-tert-butyl-thiophene-2-carboxylic acid [2-(1-aminoisoquinolin-6-ylcarbamoyl)-2-hydroxy-1-phenylethyl]amide, N-[2-(1-aminoisoquinolin-6-ylcarbamoyl)-2-hydroxy-1-phenylethyl]-3-ethylbenzamide, N-[2-(1-aminoisoquinolin-6-ylcarbamoyl)-2-hydroxy-1-phenylethyl]-3-ethoxybenzamide, N-[2-(2-amino-1H-benzimidazol-5-ylcarbamoyl)-2-hydroxy-1-(3-trifluoromethylphenyl)ethyl]-4-isopropylbenzamide, N-[2-(2-amino-1H-benzimidazol-5-ylcarbamoyl)-2-hydroxy-1-(2-methoxyphenyl)ethyl]-4-isopropylbenzamide, N-[2-(2-amino-1H-benzimidazol-5-ylcarbamoyl)-2-hydroxy-1-(3-trifluoromethylphenyl)ethyl]-4-diethylaminobenzamide, N-[2-(2-amino-1H-benzimidazol-5-ylcarbamoyl)-2-hydroxy-1-(2-methoxyphenyl)ethyl]-4-diethylaminobenzamide, N-[2-(2-amino-1H-benzimidazol-5-ylcarbamoyl)-2-hydroxy-1-(4-methoxyphenyl)ethyl]-4-diethylaminobenzamide, N-[2-(1-aminoisoquinolin-6-ylcarbamoyl)-1-(3-ethoxy-phenyl)-2-hydroxyethyl]-4-diethylaminobenzamide, N-[2-(2-amino-3H-benzimidazol-5-ylcarbamoyl)-2-hydroxy-1-(4-isopropylphenyl)ethyl]-4-diethylaminobenzamide, N-[2-(2-amino-3H-benzimidazol-5-ylcarbamoyl)-1-(3-ethoxyphenyl)-2-hydroxyethyl]-4-isopropylbenzamide, N-[2-(2-amino-3H-benzimidazol-5-ylcarbamoyl)-1-(3-ethoxyphenyl)-2-hydroxyethyl]-4-diethylaminobenzamide, N-[2-(1-aminoisoquinolin-6-ylcarbamoyl)-2-hydroxy-1-m-tolylethyl]-4-diethylaminobenzamide, N-[2-(2-amino-3H-benzimidazol-5-ylcarbamoyl)-2-hydroxy-1-(4-isopropylphenyl)ethyl]-4-isopropylbenzamide, N-[2-(2-aminobenzothiazol-6-ylcarbamoyl)-2-hydroxy-1-(4-trifluoromethylphenyl)ethyl]-4-isopropylbenzamide, N-[2-(2-aminobenzothiazol-6-ylcarbamoyl)-2-hydroxy-1-m-tolylethyl]-4-diethylamino-benzamide, N-[2-(2-aminobenzothiazol-6-ylcarbamoyl)-2-hydroxy-1-m-tolyl-ethyl]-4-isopropylbenzamide, N-[2-(2-aminobenzothiazol-6-ylcarbamoyl)-2-hydroxy-1-(4-isopropylphenyl)ethyl]-4-diethylaminobenzamide, N-[2-(2-aminobenzothiazol-6-ylcarbamoyl)-1-(3-ethoxyphenyl)-2-hydroxyethyl]-4-diethylaminobenzamide, N-[2-(2-aminobenzothiazol-6-ylcarbamoyl)-2-hydroxy-1-(4-trifluoromethylphenyl)ethyl]-4-diethylaminobenzamide, N-[2-(2-amino-3H-benzimidazol-5-ylcarbamoyl)-1-(2-chlorophenyl)-2-hydroxyethyl]-4-diethylaminobenzamide, N-[2-(2-amino-3H-benzimidazol-5-ylcarbamoyl)-2-hydroxy-1-(4-trifluoromethylphenyl)ethyl]-4-isopropylbenzamide, N-[2-(2-amino-3H-benzimidazol-5-ylcarbamoyl)-2-hydroxy-1-(4-trifluoromethylphenyl)-ethyl]-4-diethylaminobenzamide, N-[2-(2-aminobenzothiazol-6-ylcarbamoyl)-1-(3-ethoxyphenyl)-2-hydroxyethyl]-4-isopropylbenzamide, N-[2-(2-aminobenzothiazol-6-ylcarbamoyl)-1-(2-chlorophenyl)-2-hydroxyethyl]-4-diethylaminobenzamide, N-[2-(1-aminoisoquinolin-6-ylcarbamoyl)-1-(3-bromo-phenyl)-2-hydroxyethyl]-4-diethylaminobenzamide, N-[2-(1-aminoisoquinolin-6-ylcarbamoyl)-1-(3-bromophenyl)-2-hydroxyethyl]-4-ethylbenzamide, N-[2-(1-aminoisoquinolin-6-ylcarbamoyl)-2-hydroxy-1-(4-isopropylphenyl)ethyl]-4-isopropylbenzamide, N-[2-(1-aminoisoquinolin-6-ylcarbamoyl)-1-(2-chloro-phenyl)-2-hydroxyethyl]-4-isopropylbenzamide, N-[2-(2-amino-3H-benzimidazol-5-ylcarbamoyl)-2-hydroxy-1-m-tolylethyl]-4-isopropylbenzamide, N-[2-(2-amino-3H-benzimidazol-5-ylcarbamoyl)-2-hydroxy-1-m-tolylethyl]-4-diethylaminobenzamide, N-[2-(1-aminoisoquinolin-6-ylcarbamoyl)-1-(4-bromophenyl)-2-hydroxyethyl]-4-diethylaminobenzamide, N-[2-(1-aminoisoquinolin-6-ylcarbamoyl)-1-(4-bromophenyl)-2-hydroxyethyl]-4-isopropylbenzamide, N-[2-(1-aminoisoquinolin-6-ylcarbamoyl)-1-(3-bromophenyl)-2-hydroxyethyl]-4-isopropylbenzamide, N-[2-(1-aminoisoquinolin-6-ylcarbamoyl)-2-hydroxy-l-m-tolylethyl]-4-isopropylbenzamide, N-[2-(1-aminoisoquinolin-6-ylcarbamoyl)-1-(3-ethoxyphenyl)-2-hydroxyethyl]-4-isopropylbenzamide, N-[2-(1-aminoisoquinolin-6-ylcarbamoyl)-2-hydroxy-1-(4-isopropylphenyl)ethyl]-4-diethylaminobenzamide, N-[2-(1-aminoisoquinolin-6-ylcarbamoyl)-1-(2-chlorophenyl)-2-hydroxyethyl]-4-diethylaminobenzamide, N-[2-(1-aminoisoquinolin-6-ylcarbamoyl)-2-hydroxy-1-(4-trifluoromethylphenyl)-ethyl]-4-diethylaminobenzamide, N-[2-(1-aminoisoquinolin-6-ylcarbamoyl)-2-hydroxy-1-(4-trifluoromethylphenyl)ethyl]-4-isopropylbenzamide, N-[2-(1-aminoisoquinolin-6-ylcarbamoyl)-1-(4-bromophenyl)-2-hydroxyethyl]-4-ethylbenzamide, N-[2-(2-amino-3H-benzimidazol-5-ylcarbamoyl)-1-(3,5-dimethoxyphenyl)-2-hydroxyethyl]-4-isopropylbenzamide, N-[2-(2-amino-3H-benzimidazol-5-ylcarbamoyl)-1-(3,5-dimethoxyphenyl)-2-hydroxyethyl]-4-diethylaminobenzamide, N-[2-(1-aminoisoquinolin-6-ylcarbamoyl)-2-hydroxy-1-phenylethyl]-4-propylbenzamide, N-[2-(2-amino-3H-benzimidazol-5-ylcarbamoyl)-1-(2-chlorophenyl)-2-hydroxyethyl]-4-isopropylbenzamide, N-[2-(1-aminoisoquinolin-6-ylcarbamoyl)-2-hydroxy-1-phenylethyl]-4-cyclopropylbenzamide, N-[2-(1-aminoisoquinolin-6-ylcarbamoyl)-1-(2-chlorophenyl)-2-hydroxyethyl]-4-ethylbenzamide, N-[2-(2-aminobenzothiazol-6-ylcarbamoyl)-1-furan-2-yl-2-hydroxyethyl]-4-diethylaminobenzamide, N-[2-(2-amino-3H-benzimidazol-5-ylcarbamoyl)-1-(2-fluorophenyl)-2-hydroxyethyl]-4-diethylaminobenzamide, N-[2-(2-amino-3H-benzimidazol-5-ylcarbamoyl)-1-(3,5-difluorophenyl)-2-hydroxyethyl]-4-isopropylbenzamide, N-[2-(2-amino-3H-benzimidazol-5-ylcarbamoyl)-1-(3,5-difluorophenyl)-2-hydroxyethyl]-4-diethylaminobenzamide, N-[2-(2-amino-3H-benzimidazol-5-ylcarbamoyl)-1-(5-fluoro-2-methanesulfonylphenyl)-2-hydroxyethyl]-4-diethylaminobenzamide, N-[2-(2-amino-3H-benzimidazol-5-ylcarbamoyl)-1-(5-fluoro-2-methanesulfonylphenyl)-2-hydroxyethyl]-4-diethylaminobenzamide, N-[2-(2-amino-3H-benzimidazol-5-ylcarbamoyl)-1-(3-chlorophenyl)-2-hydroxyethyl]-4-diethylaminobenzamide, N-[2-(2-amino-3H-benzimidazol-5-ylcarbamoyl)-1-(3-chlorophenyl)-2-hydroxyethyl]-4-diethylaminobenzamide, N-[2-(2-amino-3H-benzimidazol-5-ylcarbamoyl)-2-hydroxy-1-(2-trifluoromethoxyphenyl)ethyl]-4-isopropylbenzamide, N-[2-(2-amino-3H-benzimidazol-5-ylcarbamoyl)-2-hydroxy-1-(2-trifluoromethoxyphenyl)ethyl]-4-diethylaminobenzamide, N-[2-(2-amino-3H-benzimidazol-5-ylcarbamoyl)-2-hydroxy-1-o-tolylethyl]-4-diethylaminobenzamide, N-[2-(2-amino-3H-benzimidazol-5-ylcarbamoyl)-2-hydroxy-1-thiophen-2-ylethyl]-4-diethylaminobenzamide, 9H-fluoren-9-ylmethyl [(1S,2R)-2-(2-amino-3H-benzimidazol-5-ylcarbamoyl)-2-hydroxy-1-phenylethyl]carbamate or 2-chlorobenzyl [2-(2-amino-3H-benzimidazol-5-ylcarbamoyl)-2-hydroxy-1-phenylethyl]carbamate.

4. A process for the preparation of the compound of the formula I as claimed in one or more of claims 1 to 3, which comprises
a) reacting a compound of the formula II 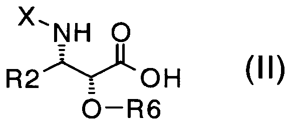 where X is an amino-protective group and the radicals R2 and R6 are as defined in formula I with a compound NH(R1)(R4) to give a compound of the formula III, 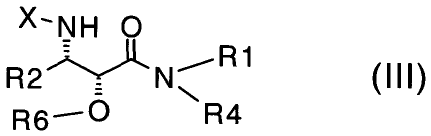 where X is an amino-protective group and the radicals R1, R2, R4 and R6 are as defined in formula I, and converting the compound of the formula III by removal of the protective group to a compound of the formula IV, 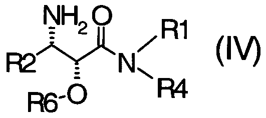 where the radicals R1, R2, R4 and R6 are as defined in formula I, and reacting with the compound of the formula V 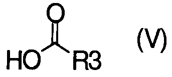 to give the compound of the formula I, or
b) either isolating the compound of the formula I prepared according to process a) in free form or releasing it from physiologically intolerable salts or, in the case of the presence of acidic or basic groups, converting it into physiologically tolerable salts, or
c) separating a compound of the formula I prepared according to process a), or a suitable precursor of the formula I which on account of its chemical structure occurs in enantiomeric or diastereomeric forms, into the pure enantiomers or diastereomers by salt formation with enantiomerically pure acids or bases, chromatography on chiral stationary phases or derivatization by means of chiral enantiomerically pure compounds such as amino acids, separation of the diastereomers thus obtained, and removal of the chiral auxiliary groups.

5. A medicament which comprises an efficacious amount of at least one compound of the formula I as claimed in one or more of claims 1 to 3 together with a pharmaceutically suitable and physiologically tolerable carrier, additive and/or other active substances and auxiliaries.

6. The use of the compound of the formula I as claimed in one or more of claims 1 to 3 for the production of a medicament for the prophylaxis, secondary prevention and therapy of all those diseases which accompany thromboses, embolisms, hypercoagulability or fibrotic changes.

7. The use as claimed in claim 6, wherein the disease is a myocardial infarct, angina pectoris and other forms of acute coronary syndrome, stroke, peripheral vascular diseases, deep vein thrombosis, pulmonary embolism, embolic or thrombotic events caused by cardiac arrhythmias, cardiovascular events such as restenosis after revascularization and angioplasty and similar events such as stent implantations and bypass operations, or the reduction of the risk of thrombosis after surgical interventions such as in knee and hip joint operations, or disseminated intravascular coagulation, sepsis and other intravascular events which accompany inflammation, atherosclerosis, diabetes and the metabolic syndrome and their sequelae, tumor growth and tumor metastasis, inflammatory and degenerative joint diseases such as rheumatoid arthritis and arthrosis, disorders of the hemostatic system such as fibrin deposits, fibrotic changes of the lungs such as chronic obstructive pulmonary disease, adult respiratory distress syndrome or fibrin deposits in the eye after eye operations or prevention and/or treatment of scar formation.

## Revendications

1. Composé de formule I 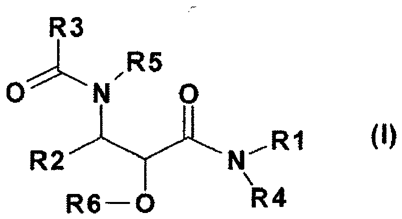 et/ou toutes les formes stéréoisomères du composé de formule I et/ou les mélanges de ces formes dans un rapport quelconque, et/ou un sel physiologiquement tolérable du composé de formule I, où
R1 est
1) - (C₆-C₁₄) -aryl-Z, dans lequel aryle est choisi dans le groupe constitué de phényle et naphtyle, et dans lequel aryle est non substitué ou mono-, di- ou trisubstitué par T et Z est amino, amidino, aminométhylène, aminopyridinyle, azétidinyle, guanidino, pipéridinyle, pyridinyle ou pyrrolidinyle, ou
2) Het-Z à quatre à quinze chaînons, dans lequel Z Het est choisi dans le groupe constitué d'acridinyle, azépinyle, azétidinyle, benzimidazolinyle, benzimidazolyle, benzofuranyle, benzothiofuranyle, benzothiophényle, benzoxazolyle, benzothiazolyle, benzotriazolyle, benzotétrazolyle, benzisoxazolyle, benzisothiazolyle, carbazolyle, 4aH-carbazolyle, carbolinyle, bêta-carbolinyle, quinazolinyle, quinoléinyle, quinolizinyle, 4H-quinolizinyle, quinoxalinyle, quinuclidinyle, chromanyle, chroményle, cinnolinyle, décahydroquinoléinyle, dibenzofuranyle, dibenzothiophényle, dihydrofurane[2,3-b]-tétrahydrofuranyle, dihydrofuranyle, dioxolyle, dioxanyle, dioxolényle, 2H,6H-1,5,2-dithiazinyle, furanyle, furazanyle, imidazolidinyle, imidazolinyle, imidazolyle, 1H-indazolyle, indolinyle, indolizinyle, indolyle, 3H-indolyle, isobenzofuranyle, isoquinoléinyle, isochromanyle, isoindazolyle, isoindolinyle, isoindolyle, isothiazolidinyle, 2-isothiazolinyle, isothiazolyle, isoxazolyle, isoxazolidinyle, 2-isoxazolinyle, morpholinyle, naphtyridinyle, octahydroisoquinoléinyle, oxadiazolyle, 1,2,3-oxadiazolyle, 1,2,4-oxadiazolyle, 1,2,5-oxadiazolyle, 1,3,4-oxadiazolyle, oxazolidinyle, oxazolyle, oxazolidinyle, oxothiolanyle, phénanthridinyle, phénanthrényle, phénanthrolinyle, phénazinyle, phénothiazinyle, phénoxathiinyle, phénoxazinyle, phtalazinyle, pipérazinyle, pipéridinyle, ptéridinyle, purinyle, pyranyle, pyrazinyle, pyrazolidinyle, pyrazolinyle, pyrazolyle, pyridazinyle, pyridooxazolyle, pyridoimidazolyle, pyridothiazolyle, pyridothiophényle, pyridyle, pyrimidinyle, pyrrolidinyle, pyrrolinyle, 2H-pyrrolyle, pyrrolyle, tétrahydrofuranyle, tétrahydroisoquinoléinyle, tétrahydroquinoléinyle, tétrahydropyridinyle, 6H-1,2,5-thiadazinyle, 1,2,3-thiadiazolyle, 1,2,4-thiadiazolyle, 1,2,5-thiadiazolyle, 1,3,4-thiadiazolyle, thianthrényle, thiazinyle, thiazolyle, thiényle, thiénoimidazolyle, thiénooxazolyle, thiénopyridinyle, thiénopyrrolyle, thiénothiazolyle, thiénothiophényle, thiomorpholinyle, thiopyranyle, triazinyle, 1,2,3-triazolyle, 1,2,4-triazolyle, 1,2,5-triazolyle, 1,3,4-triazolyle ou xanthényle et dans lequel Het est non substitué ou mono-, di- ou trisubstitué par T et dans lequel Z est tel que défini ci-dessus,
R2 est -(C₀-C₄)-alkylène-(C₆-C₁₄)-phényle, dans lequel phényle est non substitué ou mono-, di- ou trisubstitué par T,
R3 est
1) -(C₀-C₄)-alkylène-(C₆-C₁₄)-aryle, dans lequel aryle est tel que défini ci-dessus et non substitué ou mono-, di- ou trisubstitué par T,
2) -(C₀-C₄)-alkylène-Het, dans lequel Het est tel que défini ci-dessus et non substitué ou mono-, di- ou trisubstitué par T,
3) - (C₀-C₄)-alkylène-(C₆-C₁₄) -aryl-Q-(C₆-C₁₄)-aryle, dans lequel les deux aryles dans chaque cas indépendamment l'un de l'autre sont tels que définis ci-dessus et dans chaque cas indépendamment l'un de l'autre sont non substitués ou mono-, di- ou trisubstitués par T,
4) -(C₀-C₄)-alkylène-(C₆-C₁₄)-aryl-Q-(C₃-C₁₂)-cycloalkyle, dans lequel aryle, tel que défini ci-dessus, et cycloalkyle dans chaque cas indépendamment l'un de l'autre sont non substitués ou mono-, di- ou trisubstitués par T,
5) -(C₀-C₄)-alkylène-(C₆-C₁₄)-aryl-Q-Het, dans lequel aryle et Het sont tels que définis ci-dessus et dans chaque cas indépendamment l'un de l'autre sont non substitués ou mono-, di- ou trisubstitués par T,
6) - (C₀-C₄)-alkylène-Het-Q-(C₆-C₁₄)-aryle, dans lequel aryle et Het sont tels que définis ci-dessus et dans chaque cas indépendamment l'un de l'autre sont non substitués ou mono-, di- ou trisubstitués par T, ou
7) -(C₀-C₄)-alkylène-Het-Q-Het, dans lequel les deux radicaux Het sont tels que définis ci-dessus et dans chaque cas indépendamment l'un de l'autre sont non substitués ou mono-, di- ou trisubstitués par T,
Q est une liaison covalente, -(C₁-C₄)-alkylène, -NH-, -N((C₁-C₄)-alkyl)- ou -O-,
T est
1)halogène,
2) - (C₁-C₆)-alkyle, dans lequel alkyle est non substitué ou indépendamment l'un de l'autre mono- di-ou trisubstitué par -(C₁-C₃)-fluoroalkyle, -N-C(O)-OH ou -N-C(O)-(C₁-C₄)-alkyle,
3) -(C₁-C₃)-fluoroalkyle,
4) -(C₃-C₆)-cycloalkyle,
5) -OH,
6) -O-(C₁-C₄)-alkyle,
7) -O-(C₁-C₃-fluoroalkyle,
8) -NO₂,
9) -CN,
10) -N(R10)(R11), dans lequel R10 et R11 indépendamment l'un de l'autre sont un atome d'hydrogène, -(C₃-C₆)-cycloalkyle, halogène ou -(C₁-C₆)-alkyle,
11) -C(O)-NH-R10,
12) -NH-C(O)-R10,
13) -NH-SO₂-R10,
14) -SO₂-(C₁-C₄)-alkyle,
15) -SO₂-NH-R10,
16) -SO₂-(C₁-C₃)-fluoroalkyle,
17) -S-(C₁-C₄)-alkyle ou
18) -S-(C₁-C₃-fluoroalkyle,
R4 et R5 sont identiques ou différents et indépendamment l'un de l'autre sont un atome d'hydrogène ou -(C₁-C₄)-alkyle, et
R6 est un atome d'hydrogène, -C(O)-R12, -C(O)-O-R12, -C(O)-NH-R12 ou -(C₁-C₄)-alkyle, où
R12 est -(C₁-C₆)-alkyle, -(C₃-C₆)-cycloalkyle, - (C₆-C₁₄)-aryle ou Het.

2. Composé de formule I selon la revendication 1, **caractérisé en ce que**
R1 est 4-benzamidine, aminométhylphényle ou Het-Z, dans lequel Het est choisi dans le groupe constitué de benzimidazolyle, benzothiazolyle et isoquinoléinyle, et dans lequel Z est amino,
R2 est phényle, dans lequel phényle est non substitué ou mono- ou disubstitué par T, ou
R3 est
1) phényle, dans lequel phényle est non substitué ou mono- ou disubstitué par T,
2) Het-2, dans lequel Het-2 est choisi dans le groupe constitué de benzimidazolyle, benzofuranyle, benzothiophényle, quinoléinyle, quinoxalinyle, furanyle, indolyle, isoquinoléinyle, isoxazolyle, morpholinyle, pipéridinyle, pyridyle, pyrimidinyle, pyrrolidinyle, pyrrolyle, thiényle, thiénopyrrolyle ou thiénothiophényle et dans lequel Het-2 est non substitué ou mono- ou disubstitué par T,
3) -phényl-Q-phényle, dans lequel les deux radicaux phényle dans chaque cas indépendamment l'un de l'autre sont non substitués ou mono- ou disubstitués par T,
4) phényl-Q-(C₃-C₆)-cycloalkyle, dans lequel phényle et cycloalkyle dans chaque cas indépendamment l'un de l'autre sont non substitués ou mono- ou disubstitués par T,
5) phényl-Q-Het-2, dans lequel Het-2 est tel que défini ci-dessus et phényle et Het-2 dans chaque cas indépendamment l'un de l'autre sont non substitués ou mono- ou disubstitués par T,
6) Het-2-Q-phényle, dans lequel Het-2 est tel que défini ci-dessus et phényle et Het-2 dans chaque cas indépendamment l'un de l'autre sont non substitués ou mono- ou disubstitués par T, ou
7) Het-2-Q-Het-2, dans lequel les deux radicaux Het-2 sont tels que définis ci-dessus et dans chaque cas indépendamment l'un de l'autre sont non est substitués ou mono- ou disubstitués par T,
Q est une liaison covalente, -CH₂-, -N(CH₃)- ou -O-,
T est
1) F, Cl ou Br,
2) -(C₁-C₄-alkyle, dans lequel alkyle est non substitué ou indépendamment l'un de l'autre mono- ou disubstitué par -CF₃ ou -N-C(O)-CH)₃,
3) -CF₃,
4) -O-(C₁-C₄)-alkyle,
5) -O-CF₃,
6) -NO₂,
7) -N(R10)(R11), dans lequel R10 et R11 indépendamment l'un de l'autre sont un atome d'hydrogène ou -(C₁-C4)-alkyle,
8) -SO₂-CH₃,
9) -S-CF₃ ou
10) -S-(C₁-C₂-alkyle,
R4, R5 et R6 sont dans chaque cas un atome d'hydrogène.

3. Composé de formule I selon les revendications 1 ou 2, **caractérisé en ce qu'**il s'agit du composé N-[(1S,2R)-2-(1-aminoisoquinoléin-7-ylcarbamoyl)-2-hydroxy-1-phényléthyl]-4-tert-butylbenzamide, N-[(1S,2R)-2-(1-aminoisoquinoléin-6-ylcarbamoyl)-2-hydroxy-1-phényléthyl]-4-diéthylaminobenzamide, N-[(1S,2R)-2-(2-aminobenzothiazol-6-ylcarbamoyl)-2-hydroxy-1-phényléthyl]-4-isopropylbenzamide, N-[(1S,2R)-2-(2-aminobenzothiazol-6-ylcarbamoyl)-1-(2-chlorophényl)-2-hydroxyéthyl]-4-isopropylbenzamide, N-[(1S,2R)-2-(2-amino-3H-benzimidazol-5-ylcarbamoyl)-1-(2-fluorophényl)-2-hydroxyéthyl]-4-isopropylbenzamide, N-[(1S,2R)-[2-(2-amino-3H-benzimidazol-5-ylcarbamoyl)-2-hydroxy-1-phényléthyl]-4-tert-butylbenzamide, N-[(1S,2R)-2-(4-aminométhylphénylcarbamoyl)-2-hydroxy-1-phényléthyl]benzamide, N-[(1S,2R)-2-(2,4-diaminoquinazolin-6-ylcarbamoyl)-2-hydroxy-1-phényléthyl]benzamide, 4-amino-N-[2-(2-amino-3H-benzimidazol-5-ylcarbamoyl)-2-hydroxy-1-phényléthyl]benzamide, [2-(2-amino-3H-benzimidazol-5-ylcarbamoyl)-2-hydroxy-1-phényléthyl]amide d'acide quinoxaline-2-carboxylique, N-[2-(2-amino-3H-benzimidazol-5-ylcarbamoyl)-2-hydroxy-1-phényléthyl]-4-méthoxybenzamide, [2-(2-amino-3H-benzimidazol-5-ylcarbamoyl)-2-hydroxy-1-phényléthyl]amide d'acide thiophène-3-carboxylique, [(1S,2R)-2-(2-amino-3H-benzimidazol-5-ylcarbamoyl)-2-hydroxy-1-phényléthyl]amide d'acide 1-méthyl-1H-pyrrole-2-carboxylique, [2-(2-amino-3H-benzimidazol-5-ylcarbamoyl)-2-hydroxy-1-phényléthyl]amide d'acide quinoléine-2-carboxylique, N-[2-(2-amino-3H-benzimidazol-5-ylcarbamoyl)-2-hydroxy-1-phényléthyl]-4-cyclohexylbenzamide, N-[2-(2-amino-3H-benzimidazol-5-ylcarbamoyl)-2-hydroxy-1-phényléthyl]-4-isopropylbenzamide, N-[2-(2-amino-3H-benzimidazol-5-ylcarbamoyl)-2-hydroxy-1-phényléthyl]-4-trifluorométhoxybenzamide, N-[2-(2-amino-3H-benzimidazol-5-ylcarbamoyl)-2-hydroxy-1-phényléthyl]-4-diéthylaminobenzamide, N-[2-(2-amino-3H-benzimidazol-5-ylcarbamoyl)-2-hydroxy-1-phényléthyl]-4-phénoxybenzamide, N-[2-(2-amino-3H-benzimidazol-5-ylcarbamoyl)-2-hydroxy-1-phényléthyl]-4-benzylbenzamide, N-[2-(2-amino-3H-benzimidazol-5-ylcarbamoyl)-2-hydroxy-1-phényléthyl]-4-isopropoxybenzamide, N-[2-(2-amino-3H-benzimidazol-5-ylcarbamoyl)-2-hydroxy-1-phényléthyl]-4-méthylsulfanylbenzamide, N-[2-(2-amino-3H-benzimidazol-5-ylcarbamoyl)-2-hydroxy-1-phényléthyl]-4-éthylsulfanylbenzamide, N-[2-(2-amino-3H-benzimidazol-5-ylcarbamoyl)-2-hydroxy-1-phényléthyl]-4-[(4,6-diméthylpyrimidin-2-yl)méthylamino]benzamide, N-[2-(2-amino-3H-benzimidazol-5-ylcarbamoyl)-2-hydroxy-1-phényléthyl]-4-pyridin-3-ylbenzamide, 4-(2-acétylamino-3,3,3-trifluoropropyl)-N-[2-(2-amino-3H-benzimidazol-5-ylcarbamoyl)-2-hydroxy-1-phényléthyl]benzamide, [2-(2-amino-3H-benzimidazol-5-ylcarbamoyl)-2-hydroxy-1-phényléthyl]amide d'acide 1-méthyl-1H-indole-2-carboxylique, [2-(2-amino-3H-benzimidazol-5-ylcarbamoyl)-2-hydroxy-1-phényléthyl]amide d'acide 5-méthoxy-1H-indole-2-carboxylique, [2-(2-amino-3H-benzimidazol-5-ylcarbamoyl)-2-hydroxy-1-phényléthyl]amide d'acide 5-éthyl-1H-indole-2-carboxylique, [2-(2-amino-3H-benzimidazol-5-ylcarbamoyl)-2-hydroxy-1-phényléthyl]amide d'acide 6-méthoxy-1H-indole-2-carboxylique, [2-(2-amino-3H-benzimidazol-5-ylcarbamoyl)-2-hydroxy-1-phényléthyl]amide d'acide 4-méthoxyquinoléine-2-carboxylique, [2-(2-amino-3H-benzimidazol-5-ylcarbamoyl)-2-hydroxy-1-phényléthyl]amide d'acide 3-éthoxyquinoxaline-2-carboxylique, [2-(2-amino-3H-benzimidazol-5-ylcarbamoyl)-2-hydroxy-1-phényléthyl]amide d'acide naphtalène-2-carboxylique, N-[2-(2-amino-3H-benzimidazol-5-ylcarbamoyl)-2-hydroxy-1-phényléthyl]-4-pyrrol-1-ylbenzamide, N-[2-(2-amino-3H-benzimidazol-5-ylcarbamoyl)-2-hydroxy-1-phényléthyl]-4-trifluorométhylsulfanylbenzamide, [2-(2-amino-3H-benzimidazol-5-ylcarbamoyl)-2-hydroxy-1-phényléthyl]amide d'acide biphényl-4-carboxylique, N-[2-(2-amino-3H-benzimidazol-5-ylcarbamoyl)-2-hydroxy-1-phényléthyl]-4-(2,2,2-trifluoroéthoxy)benzamide, [2-(2-amino-3H-benzimidazol-5-ylcarbamoyl)-2-hydroxy-1-phényléthyl]carbamate de benzyle, N-[2-(2-amino-3H-benzimidazol-5-ylcarbamoyl)-2-hydroxy-1-phényléthyl]-4-éthylbenzamide, N-[2-(2-amino-3H-benzimidazol-5-ylcarbamoyl)-2-hydroxy-1-phényléthyl]-4-éthoxybenzamide, [2-(2-amino-3H-benzimidazol-5-ylcarbamoyl)-2-hydroxy-1-phényléthyl] amide d'acide 5-butylpyridine-2-carboxylique, [2-(2-amino-3H-benzimidazol-5-ylcarbamoyl)-2-hydroxy-1-phényléthyl]amide d'acide benzo[b]thiophène-2-carboxylique, [2-(2-amino-3H-benzimidazol-5-ylcarbamoyl)-2-hydroxy-1-phényléthyl]amide d'acide 6-méthoxynaphtalène-2-carboxylique, [2-(2-amino-3H-benzimidazol-5-ylcarbamoyl)-2-hydroxy-1-phényléthyl]amide d'acide 4-(2,2,2-trifluoroéthyl)-4H-thiéno[3,2-b]pyrrole-5-carboxylique, 4'-{[2-(2-amino-3H-benzimidazol-5-ylcarbamoyl)-2-hydroxy-1-phényléthyl]amide}-3-méthylamide d'acide biphényl-3,4'-dicarboxylique, N-[2-(1-aminoisoquinoléin-7-ylcarbamoyl)-2-hydroxy-1-phényléthyl]-4-diéthylaminobenzamide, N-[2-(1-aminoisoquinoléin-7-ylcarbamoyl)-2-hydroxy-1-phényléthyl]-4-isopropylbenzamide, N-[2-(1-aminoisoquinoléin-7-ylcarbamoyl)-2-hydroxy-1-phényléthyl]-6-pyrrolidin-1-ylnicotinamide, N-[2-(1-aminoisoquinoléin-7-ylcarbamoyl)-2-hydroxy-1-phényléthyl]-4-morpholin-4-ylbenzamide, N-[2-(1-aminoisoquinoléin-7-ylcarbamoyl)-2-hydroxy-1-phényléthyl]-4-pyrrol-l-ylbenzamide, [2-(2-amino-3H-benzimidazol-5-ylcarbamoyl)-2-hydroxy-1-phényléthyl]amide d'acide 1-éthyl-2,3-diméthyl-1H-indole-5-carboxylique, N-[2-(2-amino-3H-benzimidazol-5-ylcarbamoyl)-2-hydroxy-1-phényléthyl]-4-morpholin-4-ylbenzamide, N-[2-(1-aminoisoquinoléin-7-ylcarbamoyl)-2-hydroxy-1-phényléthyl]-4-pyrrolidin-1-ylbenzamide, N-[2-(1-aminoisoquinoléin-7-ylcarbamoyl)-2-hydroxy-1-phényléthyl]-6-morpholin-4-ylnicotinamide, [2-(2-amino-3H-benzimidazol-5-ylcarbamoyl)-2-hydroxy-1-phényléthyl]amide d'acide 5,6-diméthoxy-1H-indole-2-carboxylique, N-[2-(2-amino-3H-benzimidazol-5-ylcarbamoyl)-2-hydroxy-1-phényléthyl]-4-pyrrolidin-1-ylbenzamide, N-[2-(2-amino-3H-benzimidazol-5-ylcarbamoyl)-2-hydroxy-1-phényléthyl]-4-isobutylbenzamide, N-[2-(2-amino-3H-benzimidazol-5-ylcarbamoyl)-2-hydroxy-1-phényléthyl]-4-butylbenzamide, N-[2-(2-amino-3H-benzimidazol-5-ylcarbamoyl)-2-hydroxy-1-phényléthyl]-4-propylbenzamide, [2-(2-amino-3H-benzimidazol-5-ylcarbamoyl)-2-hydroxy-1-phényléthyl]amide d'acide 4H-thiéno[3,2-b]pyrrole-5-carboxylique, N-[2-(4-carbamimidoylphénylcarbamoyl)-2-hydroxy-1-phényléthyl]-4-diéthylaminobenzamide, [2-(1-aminoisoquinoléin-7-ylcarbamoyl)-2-hydroxy-1-phényléthyl]amide d'acide 5-méthoxy-1H-indole-2-carboxylique, [2-(1-aminoisoquinoléin-7-ylcarbamoyl)-2-hydroxy-1-phényléthyl]amide d'acide 5-éthylthiophène-2-carboxylique, [2-(1-aminoisoquinoléin-7-ylcarbamoyl)-2-hydroxy-1-phényléthyl]amide d'acide 5-tert-butylthiophène-2-carboxylique, N-[2-(1-aminoisoquinoléin-6-ylcarbamoyl)-2-hydroxy-1-phényléthyl]benzamide, [2-(2-amino-3H-benzimidazol-5-ylcarbamoyl)-2-hydroxy-1-phényléthyl]amide d'acide 6-bromobenzofurane-2-carboxylique, [2-(2-amino-3H-benzimidazol-5-ylcarbamoyl)-2-hydroxy-1-phényléthyl]amide d'acide 5-éthylthiophène-2-carboxylique, [2-(2-amino-3H-benzimidazol-5-ylcarbamoyl)-2-hydroxy-1-phényléthyl]amide d'acide thiéno[3,2-b]thiophène-2-carboxylique, [2-(2-amino-3H-benzimidazol-5-ylcarbamoyl)-2-hydroxy-1-phényléthyl]amide d'acide 5-méthoxybenzofurane-2-carboxylique, [2-(2-amino-3H-benzimidazol-5-ylcarbamoyl)-2-hydroxy-1-phényléthyl]amide d'acide benzo[b]thiophène-3-carboxylique, [2-(2-amino-3H-benzimidazol-5-ylcarbamoyl)-2-hydroxy-1-phényléthyl]amide d'acide 4-méthylthiophène-2-carboxylique, N-[2-(2-aminobenzothiazol-6-ylcarbamoyl)-2-hydroxy-1-phényléthyl]-4-diéthylaminobenzamide, [2-(2-amino-3H-benzimidazol-5-ylcarbamoyl)-2-hydroxy-1-phényléthyl]amide d'acide 1H-indole-2-carboxylique, N-[2-(2-amino-3H-benzimidazol-5-ylcarbamoyl)-2-hydroxy-1-phényléthyl]-4-diméthylaminobenzamide, [2-(2-amino-3H-benzimidazol-5-ylcarbamoyl)-2-hydroxy-1-phényléthyl]amide d'acide 5-tert-butylthiophène-2-carboxylique, N-[2-(2-amino-3H-benzimidazol-5-ylcarbamoyl)-2-hydroxy-1-phényléthyl]-4-méthoxy-3-méthylbenzamide, N-[2-(2-amino-3H-benzimidazol-5-ylcarbamoyl)-2-hydroxy-1-phényléthyl]-3-fluoro-4-méthylbenzamide, [2-(2-amino-3H-benzimidazol-5-ylcarbamoyl)-2-hydroxy-1-phényléthyl]amide d'acide 5-isopropylthiophène-3-carboxylique, [2-(2-amino-3H-benzimidazol-5-ylcarbamoyl)-2-hydroxy-1-phényléthyl]amide d'acide 5-éthylthiophène-3-carboxylique, [2-(2-amino-3H-benzimidazol-5-ylcarbamoyl)-2-hydroxy-1-phényléthyl]amide d'acide [2,2']bithiophényl-5-carboxylique, [2-(1-aminoisoquinoléin-6-ylcarbamoyl)-2-hydroxy-1-phényléthyl]amide d'acide 5-méthoxy-1H-indole-2-carboxylique, N-[2-(1-aminoisoquinoléin-6-ylcarbamoyl)-2-hydroxy-1-phényléthyl]-4-méthoxybenzamide, N-[2-(1-aminoisoquinoléin-6-ylcarbamoyl)-2-hydroxy-1-phényléthyl]-4-isopropylbenzamide, N-[2-(1-aminoisoquinoléin-6-ylcarbamoyl)-2-hydroxy-1-phényléthyl]-4-diéthylaminobenzamide, N-[2-(1-aminoisoquinoléin-6-ylcarbamoyl)-2-hydroxy-1-phényléthyl]-4-éthoxybenzamide, N-[2-(1-aminoisoquinoléin-6-ylcarbamoyl)-2-hydroxy-1-phényléthyl]-4-éthylbenzamide, N-[2-(1-aminoisoquinoléin-6-ylcarbamoyl)-2-hydroxy-1-phényléthyl]-4-tert-butylbenzamide, N-[2-(1-aminoisoquinoléin-6-ylcarbamoyl)-2-hydroxy-1-phényléthyl]-3-diméthylaminobenzamide, N-[2-(1-aminoisoquinoléin-6-ylcarbamoyl)-2-hydroxy-1-phényléthyl]-4-diméthylaminobenzamide, [2-(1-aminoisoquinoléin-6-ylcarbamoyl)-2-hydroxy-1-phényléthyl]amide d'acide 5-éthylthiophène-2-carboxylique, [2-(1-aminoisoquinoléin-6-ylcarbamoyl)-2-hydroxy-1-phényléthyl]amide d'acide 5-éthylthiophène-3-carboxylique, [2-(1-aminoisoquinoléin-6-ylcarbamoyl)-2-hydroxy-1-phényléthyl]amide d'acide 5-tert-butylthiophène-2-carboxylique, N-[2-(1-aminoisoquinoléin-6-ylcarbamoyl)-2-hydroxy-1-phényléthyl]-3-éthylbenzamide, N-[2-(1-aminoisoquinoléin-6-ylcarbamoyl)-2-hydroxy-1-phényléthyl]-3-éthoxybenzamide, N-[2-(2-amino-1H-benzimidazol-5-ylcarbamoyl)-2-hydroxy-1-(3-trifluorométhylphényl)éthyl]-4-isopropylbenzamide, N-[2-(2-amino-1H-benzimidazol-5-ylcarbamoyl)-2-hydroxy-1-(2-méthoxyphényl)éthyl]-4-isopropylbenzamide, N-[2-(2-amino-1H-benzimidazol-5-ylcarbamoyl)-2-hydroxy-1-(3-trifluorométhylphényl)éthyl]-4-diéthylaminobenzamide, N-[2-(2-amino-1H-benzimidazol-5-ylcarbamoyl)-2-hydroxy-1-(2-méthoxyphényl)éthyl]-4-diéthylaminobenzamide, N-[2-(2-amino-1H-benzimidazol-5-ylcarbamoyl)-2-hydroxy-1-(4-méthoxyphényl)éthyl]-4-diéthylaminobenzamide, N-[2-(1-aminoisoquinoléin-6-ylcarbamoyl)-1-(3-éthoxyphényl)-2-hydroxyéthyl]-4-diéthylaminobenzamide, N-[2-(2-amino-3H-benzimidazol-5-ylcarbamoyl)-2-hydroxy-1-(4-isopropylphényl)éthyl]-4-diéthylaminobenzamide, N-[2-(2-amino-3H-benzimidazol-5-ylcarbamoyl)-1-(3-éthoxyphényl)-2-hydroxyéthyl]-4-isopropylbenzamide, N-[2-(2-amino-3H-benzimidazol-5-ylcarbamoyl)-1-(3-éthoxyphényl)-2-hydroxyéthyl]-4-diéthylaminobenzamide, N-[2-(1-aminoisoquinoléin-6-ylcarbamoyl)-2-hydroxy-1-m-tolyléthyl]-4-diéthylaminobenzamide, N-[2-(2-amino-3H-benzimidazol-5-ylcarbamoyl)-2-hydroxy-1-(4-isopropylphényl)éthyl]-4-isopropylbenzamide, N-[2-(2-aminobenzothiazol-6-ylcarbamoyl)-2-hydroxy-1-(4-trifluorométhylphényl)éthyl]-4-isopropylbenzamide, N-[2-(2-aminobenzothiazol-6-ylcarbamoyl)-2-hydroxy-l-m-tolyléthyl]-4-diéthylaminobenzamide, N-[2-(2-aminobenzothiazol-6-ylcarbamoyl)-2-hydroxy-l-m-tolyléthyl]-4-isopropylbenzamide, N-[2-(2-aminobenzothiazol-6-ylcarbamoyl)-2-hydroxy-1-(4-isopropylphényl)éthyl]-4-diéthylaminobenzamide, N-[2-(2-aminobenzothiazol-6-ylcarbamoyl)-1-(3-éthoxyphényl)-2-hydroxyéthyl]-4-diéthylaminobenzamide, N-[2-(2-aminobenzothiazol-6-ylcarbamoyl)-2-hydroxy-1-(4-trifluorométhylphényl)éthyl]-4-diéthylaminobenzamide, N-[2-(2-amino-3H-benzimidazol-5-ylcarbamoyl)-1-(2-chlorophényl)-2-hydroxyéthyl]-4-diéthylaminobenzamide, N-[2-(2-amino-3H-benzimidazol-5-ylcarbamoyl)-2-hydroxy-1-(4-trifluorométhylphényl)éthyl]-4-isopropylbenzamide, N-[2-(2-amino-3H-benzimidazol-5-ylcarbamoyl)-2-hydroxy-1-(4-trifluorométhylphényl)éthyl]-4-diéthylaminobenzamide, N-[2-(2-aminobenzothiazol-6-ylcarbamoyl)-1-(3-éthoxyphényl)-2-hydroxyéthyl]-4-isopropylbenzamide, N-[2-(2-aminobenzothiazol-6-ylcarbamoyl)-1-(2-chlorophényl)-2-hydroxyéthyl]-4-diéthylaminobenzamide, N-[2-(1-aminoisoquinoléin-6-ylcarbamoyl)-1-(3-bromophényl)-2-hydroxyéthyl]-4-diéthylaminobenzamide, N-[2-(1-aminoisoquinoléin-6-ylcarbamoyl)-1-(3-bromophényl)-2-hydroxyéthyl]-4-éthylbenzamide, N-[2-(1-aminoisoquinoléin-6-ylcarbamoyl)-2-hydroxy-1-(4-isopropylphényl)éthyl]-4-isopropylbenzamide, N-[2-(1-aminoisoquinoléin-6-ylcarbamoyl)-1-(2-chlorophényl)-2-hydroxyéthyl]-4-isopropylbenzamide, N-[2-(2-amino-3H-benzimidazol-5-ylcarbamoyl)-2-hydroxy-1-m-tolyléthyl]-4-isopropylbenzamide, N-[2-(2-amino-3H-benzimidazol-5-ylcarbamoyl)-2-hydroxy-1-m-tolyléthyl]-4-diéthylaminobenzamide, N-[2-(1-aminoisoquinoléin-6-ylcarbamoyl)-1-(4-bromophényl)-2-hydroxyéthyl]-4-diéthylaminobenzamide, N-[2-(1-aminoisoquinoléin-6-ylcarbamoyl)-1-(4-bromophényl)-2-hydroxyéthyl]-4-isopropylbenzamide, N-[2-(1-aminoisoquinoléin-6-ylcarbamoyl)-1-(3-bromophényl)-2-hydroxyéthyl]-4-isopropylbenzamide, N-[2-(1-aminoisoquinoléin-6-ylcarbamoyl)-2-hydroxy-1-m-tolyléthyl]-4-isopropylbenzamide, N-[2-(1-aminoisoquinoléin-6-ylcarbamoyl)-1-(3-éthoxyphényl)-2-hydroxyéthyl]-4-isopropylbenzamide, N-[2-(1-aminoisoquinoléin-6-ylcarbamoyl)-2-hydroxy-1-(4-isopropylphényl)éthyl]-4-diéthylaminobenzamide, N-[2-(1-aminoisoquinoléin-6-ylcarbamoyl)-1-(2-chlorophényl)-2-hydroxyéthyl]-4-diéthylaminobenzamide, N-[2-(1-aminoisoquinoléin-6-ylcarbamoyl)-2-hydroxy-1-(4-trifluorométhylphényl)éthyl]-4-diéthylaminobenzamide, N-[2-(1-aminoisoquinoléin-6-ylcarbamoyl)-2-hydroxy-1-(4-trifluorométhylphényl)éthyl]-4-isopropylbenzamide, N-[2-(1-aminoisoquinoléin-6-ylcarbamoyl)-1-(4-bromophényl)-2-hydroxyéthyl]-4-éthylbenzamide, N-[2-(2-amino-3H-benzimidazol-5-ylcarbamoyl)-1-(3,5-diméthoxyphényl)-2-hydroxyéthyl]-4-isopropylbenzamide, N-[2-(2-amino-3H-benzimidazol-5-ylcarbamoyl)-1-(3,5-diméthoxyphényl)-2-hydroxyéthyl]-4-diéthylaminobenzamide, N-[2-(1-aminoisoquinoléin-6-ylcarbamoyl)-2-hydroxy-1-phényléthyl]-4-propylbenzamide, N-[2-(2-amino-3H-benzimidazol-5-ylcarbamoyl)-1-(2-chlorophényl)-2-hydroxyéthyl]-4-isopropylbenzamide, N-[2-(1-aminoisoquinoléin-6-ylcarbamoyl)-2-hydroxy-1-phényléthyl]-4-cyclopropylbenzamide, N-[2-(1-aminoisoquinoléin-6-ylcarbamoyl)-1-(2-chlorophényl)-2-hydroxyéthyl]-4-éthylbenzamide, N-[2-(2-aminobenzothiazol-6-ylcarbamoyl)-1-furan-2-yl-2-hydroxyéthyl]-4-diéthylaminobenzamide, N-[2-(2-amino-3H-benzimidazol-5-ylcarbamoyl)-1-(2-fluorophényl)-2-hydroxyéthyl]-4-diéthylaminobenzamide, N-[2-(2-amino-3H-benzimidazol-5-ylcarbamoyl)-1-(3,5-difluorophényl)-2-hydroxyéthyl]-4-isopropylbenzamide, N-[2-(2-amino-3H-benzimidazol-5-ylcarbamoyl)-1-(3,5-difluorophényl)-2-hydroxyéthyl]-4-diéthylaminobenzamide, N-[2-(2-amino-3H-benzimidazol-5-ylcarbamoyl)-1-(5-fluoro-2-méthanesulfonylphényl)-2-hydroxyéthyl]-4-diéthylaminobenzamide, N-[2-(2-amino-3H-benzimidazol-5-ylcarbamoyl)-1-(5-fluoro-2-méthanesulfonylphényl)-2-hydroxyéthyl]-4-diéthylaminobenzamide, N-[2-(2-amino-3H-benzimidazol-5-ylcarbamoyl)-1-(3-chlorophényl)-2-hydroxyéthyl]-4-diéthylaminobenzamide, N-[2-(2-amino-3H-benzimidazol-5-ylcarbamoyl)-1-(3-chlorophényl)-2-hydroxyéthyl]-4-diéthylaminobenzamide, N-[2-(2-amino-3H-benzimidazol-5-ylcarbamoyl)-2-hydroxy-1-(2-trifluorométhoxyphényl)éthyl]-4-isopropylbenzamide, N-[2-(2-amino-3H-benzimidazol-5-ylcarbamoyl)-2-hydroxy-1-(2-trifluorométhoxyphényl)éthyl]-4-diéthylaminobenzamide, N-[2-(2-amino-3H-benzimidazol-5-ylcarbamoyl)-2-hydroxy-1-o-tolyléthyl]-4-diéthylaminobenzamide, N-[2-(2-amino-3H-benzimidazol-5-ylcarbamoyl)-2-hydroxy-1-thiophén-2-yléthyl]-4-diéthylaminobenzamide, [(1S,2R)-2-(2-amino-3H-benzimidazol-5-ylcarbamoyl)-2-hydroxy-1-phényléthyl]carbamate de 9H-fluorén-9-ylméthyle ou [2-(2-amino-3H-benzimidazol-5-ylcarbamoyl)-2-hydroxy-1-phényléthyl]carbamate de 2-chlorobenzyle.

4. Procédé de préparation du composé de formule I selon une ou plusieurs des revendications 1 à 3, **caractérisé en ce qu'**il comprend
a) la réaction d'un composé de formule II 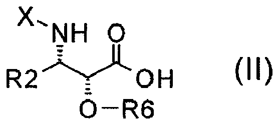 dans laquelle X est un groupement protecteur d'amino et les radicaux R2 et R6 sont tels que définis dans la formule I, avec un composé NH(R1)(R4) pour donner un composé de formule III, 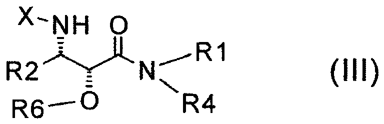 dans laquelle X est un groupement protecteur d'amino et les radicaux R1, R2, R4 et R6 sont tels que définis dans la formule I, et la transformation du composé de formule III en un composé de formule IV par l'élimination du groupement protecteur, 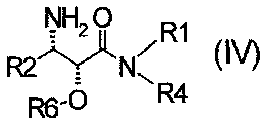 dans laquelle les radicaux R1, R2, R4 et R6 sont tels que définis dans la formule I, et la réaction avec le composé de formule V 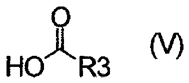 pour donner le composé de formule I, ou
b) l'isolement du composé de formule I préparé selon le procédé a) sous forme libre ou sa libération à partir de sels physiologiquement intolérables ou, en cas de présence de groupements acides ou basiques, sa transformation en sels physiologiquement tolérables, ou
c) la séparation d'un composé de formule I préparé selon le procédé a), ou un précurseur convenable de formule I, qui en raison de sa structure chimique existe sous des formes énantiomères ou diastéréoisomères, en les énantiomères ou les diastéréoisomères purs par formation de sels avec des acides ou des bases énantiomériquement purs, chromatographie sur des phases stationnaires chirales ou dérivatisation à l'aide de composés chiraux énantiomériquement purs tels que des acides aminés, la séparation des diastéréoisomères ainsi obtenus, et l'élimination des groupements auxiliaires chiraux.

5. Médicament, **caractérisé en ce qu'**il comprend une quantité efficace d'au moins un composé de formule I selon une ou plusieurs des revendications 1 à 3, conjointement avec un support ou additif pharmaceutiquement convenable et physiologiquement tolérable et/ou d'autres substances actives et auxiliaires.

6. Utilisation du composé de formule I selon une ou plusieurs des revendications 1 à 3, pour la production d'un médicament destiné à la prophylaxie, la prévention secondaire et la thérapie de toutes les maladies associées aux thromboses, aux embolismes, à l'hypercoagulabilité ou aux changements fibrotiques.

7. Utilisation selon la revendication 6, **caractérisée en ce que** la maladie est un infarctus du myocarde, l'angine de poitrine et les autres formes de syndrome coronaire aigu, l'accident vasculaire cérébral, les maladies vasculaires périphériques, la thrombose veineuse profonde, l'embolisme pulmonaire, des événements emboliques ou thrombotiques provoqués par des arythmies cardiaques, des événements cardiovasculaires tels que la resténose suivant une revascularisation et l'angioplastie et des interventions analogues telles que les implantations de stent et les opérations de pontage, ou la réduction du risque de thrombose suivant des interventions chirurgicales, telles que les chirurgies des articulations du genou et de la hanche, ou la coagulation intravasculaire disséminée, l'état septique et d'autres événements intravasculaires associés aux inflammations, l'athérosclérose, le diabète et le syndrome métabolique et leurs séquelles, la croissance des tumeurs et la métastase des tumeurs, les maladies inflammatoires et dégénératives des articulations telles que la polyarthrite rhumatoïde et l'arthrose, les troubles du système hémostatique tels que les dépôts de fibrine, des changements fibrotiques des poumons tels que la maladie pulmonaire obstructive chronique, le syndrome de détresse respiratoire chez l'adulte ou les dépôts de fibrine dans l'oeil après des chirurgies de l'oeil ou la prévention et/ou le traitement de la formation de cicatrices.
